# EUROPEAN PATENT SPECIFICATION

(11) **EP 1 051 413 B1**
(45) Date of publication and mention of the grant of the patent: **04.06.2003**
(21) Application number: 99907388.5
(22) Date of filing: 21.01.1999
(51) Int. Cl.: C07D 401/06, A61K 31/47, C07D 401/12, C07D 401/14, C07D 405/14

(54) **QUINOLINE DERIVATIVES AS ANTIBACTERIALS**
CHINOLINDERIVATE ALS ANTIBAKTERIELLES ARZNEIMITTEL
DERIVES DE QUINOLINE UTILISES COMME ANTIBACTERIENS

(30) Priority: 26.01.1998 GB 9801630; 29.09.1998 GB 9821072
(43) Date of publication of application: 15.11.2000
(73) Proprietor: SmithKline Beecham plc, Brentford, Middlesex TW8 9GS (GB)
(72) Inventor: COATES,William,John GlaxoSmithklineServicesUnLmdt, Harlow, Essex CM19 5AW (GB); GWYNN, Michael, Norman Research & Development, Collegeville, PA 19426-0989 (US); HATTON,Ian,Keith GlaxoSmithKline ServicesUnLtd, Harlow, Essex CM19 5AW (GB); MASTERS,Philip,John GlaxoSmithKline Services Unltd, Harlow Essex CM 19 5AW (GB); PEARSON,Neil,David GlaxoSmithKline Services UnLtd, Harlow Essex CM19 5AW (GB); RAHMAN,Shahzad,Sharooq GlaxoSmithKlineServicesUnLd, Harlow, Essex CM19 5AW (GB); SLOCOMBE,Brian GlaxoSmithKline Services Unldt, Harlow, Essex CM19 5AW (GB); WARRACK,Julie,Dorothy GlaxoSmithKlineServicesUnLtd, Harlow, Essex CM19 5AW (GB)
(74) Representative: Valentine, Jill Barbara
(86) International application number: EP9900333
(87) International publication number: WO99037635

(56) References cited:
- EP-A- 0 030 044
- DE-A- 2 315 148
- GB-A- 1 496 371
- US-A- 3 362 875

## Description

This invention relates to novel medicaments, being novel antibacterial compositions based upon a novel use of known quinoline compounds, as well as novel quinoline compounds.

DE 2315148A, EP 030044, NL7908030, EP0053964, EP0031753, EP0042781 and BE706646 disclose quinoline compounds having cardiovascular, hypnotic, anticonvulsant, and antimalarial effect.

EP0579263, EP0742207, JP2169569, EP0296560, WO9103243, EP0449186 disclose piperidine compounds as acetylcholinesterase inhibitors and sigma receptor antagonists

WO9802438 and WO9703069 disclose certain bicyclic heteroaromatic compounds having protein tyrosine kinase and cell proliferation inhibitor activity.

GB1496371 and US3362875 disclose certain 4-aminoquinoline derivatives having antibacterial activity.

The present inventors have discovered that certain quinoline compounds have an unexpected antibacterial effect, which has lead to development of novel formulations incorporating such quinolines.

Accordingly this invention provides the use of a quinoline of formula (I) in the free base form or a pharmaceutically acid addition or quaternary ammonium salt or N-oxide thereof for the preparation of a medicament for the treatment of bacterial infections in mammals, particularly in man. 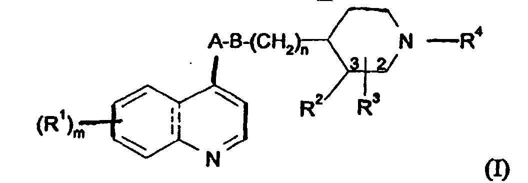 wherein:
m is 1 or 2 ;
each R¹ is independently hydroxy; (C₁₋₆) alkoxy optionally substituted by (C₁₋₆)alkoxy, amino, piperidyl, guanidino or amidino optionally N-substituted by one or two (C₁₋₆)alkyl, acyl or (C₁₋₆)alkylsulphonyl groups, NH₂CO, hydroxy, thiol, (C₁₋ ₆)alkylthio, heterocyclylthio, heterocyclyloxy, arylthio, aryloxy, acylthio, acyloxy or (C₁₋ ₆)alkylsulphonyloxy; (C₁₋₆)alkoxy-substituted (C₁₋₆)alkyl; halogen; (C₁₋₆)alkyl; (C₁-₆)alkylthio; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups;
   either R² is hydrogen; and
R³ is in the 2- or 3-position and is hydrogen or (C ₁₋₆)alkyl or (C₂₋₆)alkenyl optionally substituted with 1 to 3 groups selected from:
   thiol; halogen; (C₁₋₆)alkylthio; trifluoromethyl; azido; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁ ₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁-₆)alkylcarbonyl or (C₂₋₆)alkenylcarbonyl; amino optionally mono- or disubstituted by (C₁₋₆)alkoxycarbonyl, (C₁₋₆)alkylcarbonyl, (C₂₋₆)alkenyloxycarbonyl, (C₂-₆)alkenylcarbonyl, (C₁₋₆)alkyl, (C₂₋₆)alkenyl, (C₁₋₆)alkylsulphonyl, (C₂-₆)alkenylsulphonyl or aminocarbonyl wherein the amino group is optionally substituted by (C₁₋₆)alkyl or (C₂₋₆)alkenyl; aminocarbonyl wherein the amino group is optionally mono- or disubstituted by (C₁₋₆)alkyl, hydroxy(C₁₋₆)alkyl, aminocarbonyl(C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl]; oxo; (C₁₋₆)alkylsulphonyl; (C₂₋₆)alkenylsulphonyl; or aminosulphonyl wherein the amino group is optionally substituted by (C₁₋₆)alkyl or (C₂-₆)alkenyl; or
R³ is in the 3-position and R² and R³ together are a divalent residue =CR^{5¹}R^{6¹} where R^{5¹} and R^{6¹} are independently selected from H, (C₁₋₆)alkyl, (C₂₋₆)alkenyl, aryl(C₁₋₆)alkyl and aryl(C₂₋₆)alkenyl, any alkyl or alkenyl moiety being optionally substituted by 1 to 3 groups selected from those listed above for substituents on R³;
R⁴ is a group -CH₂-R⁵ in which R⁵ is selected from:
   (C₃₋₁₂)alkyl; hydroxy(C₃₋₁₂)alkyl; (C₁₋₁₂)alkoxy(C₃₋₁₂)alkyl; (C₁₋₁₂)alkanoyloxy(C₃₋₁₂)alkyl; (C₃₋₆)cycloalkyl(C₃-₁₂)alkyl; hydroxy-, (C₁-₁₂)alkoxy- or (C₁₋₁₂)alkanoyloxy-(C₃₋₆)cycloalkyl(C₃₋₁₂)alkyl; cyano(C₃₋₁₂)alkyl; (C₂₋₁₂)alkenyl; (C₂₋₁₂)alkyny; tetrahydrofuryl; mono- or di-(C₁₋₁₂)alkylamino(C₃₋₁₂)alkyl; acylamino(C₃₋₁₂)alkyl; (C₁₋₁₂)alkyl- or acyl-aminocarbonyl(C₃₋₁₂)alkyl; mono- or di-(C₁₋₁₂)alkylamino(hydroxy) (C₃₋₁₂)alkyl; optionally substituted phenyl(C₁₋₂)alkyl, phenoxy(C₁₋₂)alkyl or phenyl(hydroxy)(C₁₋₂)alkyl; optionally substituted diphenyl(C₁₋₂)alkyl; optionally substituted phenyl(C₂₋₃)alkenyl; optionally substituted benzoyl or benzoylmethyl; optionally substituted heteroaryl(C₁₋₂)alkyl; and optionally substituted heteroaroyl or heteroaroylmethyl;
or R⁴ is 3-benzoylpropyl or 3-(4-fluorobenzoyl)propyl
n is 0, 1 or 2;
A is NR¹¹, O, S(O)ₓ or CR⁶R⁷ and B is NR¹¹, O, S(O)ₓ or CR⁸R⁹ where x is 0, 1 or 2 and wherein:
   each of R⁶, R⁷, R⁸ and R⁹ is independently selected from: H; thiol; (C₁₋₆)alkylthio; halo; trifluoromethyl; azido; (C₁₋₆)alkyl; (C₂₋₆)alkenyl; (C₁-₆)alkoxycarbonyl; (C₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₁₋₆)alkenyl;
   or R⁶ and R⁸ together represent a bond and R⁷ and R⁹ are as above defined;
   or R⁶ and R⁸ together represent -O- and R⁷ and R⁹ are both hydrogen;
   or R⁶ and R⁷ or R⁸ and R⁹ together represent oxo;
   and each R¹¹ is independently H, trifluoromethyl, (C₁₋₆)alkyl, (C₁₋₆)alkenyl, (C₁₋₆)alkoxycarbonyl, (C₁₋₆)alkylcarbonyl, aminocarbonyl wherein the amino group is optionally substituted by (C₁₋₆)alkoxycarbonyl, (C₁₋₆)alkylcarbonyl, (C₁₋₆)alkenyloxycarbonyl, (C₂₋₆)alkenylcarbonyl, (C₁₋₆)alkyl or (C₁₋₆)alkenyl and optionally further substituted by (C₁₋₆)alkyl or (C₁₋₆)alkenyl;
   provided that A and B cannot both be selected from NR¹¹, O and S(O)ₓ and when one of A and B is CO the other is not CO, O or S(O)ₓ.

The invention also provides the use of a compound of formula (I) in the free base form or a pharmaceutically acceptable acid addition or quaternary ammonium salt or N-oxide thereof in the manufacture of a medicament for use in the treatment of bacterial infections in mammals.

The invention also provides a pharmaceutical composition for use in the treatment of bacterial infections in mammals comprising a compound of formula (I) in the free base form or a pharmaceutically acceptable acid addition or quaternary ammonium salt or N-oxide thereof, and a pharmaceutically acceptable carrier.

In a preferred aspect, when A is CH₂ or CHOH and B is CH₂ or A is CH₂ and B is CHOH and n is 1 the substitutents at the 3- and 4-position of the piperidine ring are cis.

When R¹ is substituted alkoxy it is preferably (C₂₋₆)alkoxy substituted by optionally N-substituted amino, guanidino or amidino, more preferably by amino, or (C₁₋₆)alkoxy substituted by piperidyl. Suitable examples of R¹ alkoxy include methoxy, n-propyloxy, i-butyloxy, aminoethyloxy, aminopropyloxy, aminopentyloxy, guanidinopropyloxy, piperidin-4-ylmethyloxy, or 2-aminocarbonylprop-2-oxy. Preferably R¹ is in the 6-position on the quinoline nucleus. Preferably R¹ is methoxy, amino(C₃₋₅)alkyloxy, nitro or fluoro.

Preferably m is 1,
R³ is preferably (C₁₋₆) alkyl, (C₁- ₆)alkenyl, optionally substituted 1-hydroxy-(C₁₋₆) alkyl, more preferably 1,2-dihydroxy (C₂₋₆)alkyl wherein the 2-hydroxy group is optionally substituted. Preferred examples of R³ include hydroxymethyl, 1-hydroxyethylor 1,2-dihydroxyethyl wherein the 2-hydroxy group is optionally substituted with alkylcarbonyl or aminocarbonyl where the amino group is optionally substituted. Other suitable examples of R³ include 2-hydroxyethyl, 2- or 3-hydroxypropyl, ethyl or vinyl.
R³ is preferably in the 3-position.

When R² and R³ together form a group, this is preferably =CHCH₃.

Preferably A is NH, NCH₃, O, CH₂, CHOH, CH(NH₂), C(Me)(OH) or CH(Me).

Preferably B is CH₂, CHOH, CO or S.

Alternatively and preferably, A is CR⁶R⁷ and B CR⁸R⁹ and R⁶ and R⁸ together represent -O- and R⁷ and R⁹ are both hydrogen.

Preferably n is 0 or 1.

More preferably:
when A is NH, B is CO and n is 1 or 0;
when A is O, B is CH₂ and n is 1 or 0;
when A is CH₂ or CH₂OH, B is CH₂, and n is 1 or 0;
when A is NCH₃, CH(NH₂), C(Me)(OH) or CH(Me), B is CH₂ and n is 1
when A is CR⁶R⁷ and B CR⁸R⁹ and R⁶ and R⁸ together represent -O- and R⁷ and R⁹ are both hydrogen and n is 1.

Suitable groups R⁴ include n-pentyl, n-hexyl, n-heptyl, n-octyl, n-nonyl, n-decyl, n-dodecyl, methoxybutyl, phenylethyl, phenylpropyl or 3-phenyl-prop-2-en-yl optionally substituted on the phenyl ring, 3-benzoylpropyl, 3-(4-fluorobenzoyl)propyl and phenoxyethyl.

Preferably R⁴ is (C₅₋₁₀)alkyl, unsubstituted phenyl(C₂₋₃)alkyl or unsubstituted phenyl(C₃₋₄)alkenyl, more preferably hexyl, heptyl, 5-methylhexyl, 6-methylheptyl, 3-phenyl-prop-2-en-yl or 3-phenylpropyl.

Most preferably R⁵ is unbranched at the α and, where appropnate, β positions.

Halo or halogen includes fluoro, chloro, bromo and iodo.

The term 'heterocyclic' as used herein is an aromatic or non-aromatic, single or fused, ring suitably containing up to four hetero-atoms in each ring selected from oxygen, nitrogen and sulphur, which rings may be unsubstituted or substituted by, up to three groups selected from amino, halogen, (C₁₋₆)alkyl, (C₁₋₆)alkoxy, halo(C₁₋₆)alkyl, hydroxy, carboxy, carboxy salts, (C₁₋₆)alkoxycarbonyl, (C₁₋₆)alkoxycarbonyl(C₁₋₆)alkyl, aryl, and oxo groups. Each heterocyclic ring has from 4 to 7, preferably 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

Where an amino group forms part of a single or fused non-aromatic heterocyclic ring as defined above suitable optional substituents in such substituted amino groups are (C₁₋₆)alkyl optionally substituted by hydroxy, (C₁₋₆)alkoxy, thiol, (C₁₋₆)alkylthio, halo or trifluoromethyl, and amino-protecting groups such as acyl or (C₁₋₆)alkylsulphonyl groups.

The term 'heteroaryl' is an aromatic heterocyclic group referred to above. Examples of heteroaryl groups include pyridyl, triazolyl, tetrazolyl, indolyl, thienyl, isoimidazolyl, thiazolyl, furanyl,quinolinyl, imidazolidinyl and benzothienyl.

When used herein the term 'aryl', is phenyl or naphthyl, each optionally substituted with up to five, preferably up to three, groups selected from halogen, mercapto, (C₁₋₆)alkyl, phenyl, (C₁₋₆)alkoxy, hydroxy(C₁₋₆)alkyl, mercapto (C_{1- 6})alkyl, halo(C₁₋₆)alkyl, hydroxy, amino, nitro, carboxy, (C₁-₆)alkylcarbonyloxy, (C₁₋₆)alkoxycarbonyl, formyl, or (C₁₋₆)alkylcarbonyl groups.

The term 'acyl' is an (C₁₋₆)alkoxycarbonyl, formyl or (C₁₋₆) alkylcarbonyl group.

Compounds of formula (I) wherein:
R³ is hydroxy(C₁₋₆)alkyl or 1,2-dihydroxy(C₂₋₆)alkyl optionally substituted on the hydroxy group(s) as claimed, hereinafter 'compounds of formula (IA)', are novel and as such form part of the invention.

Compounds of formula (I) wherein:
at least one R¹ is (C₂₋₆) alkoxy substituted by optionally N-substituted amino, guanidino or amidino or C₁₋₆alkoxy substituted by piperidyl, A is CH₂, CHOH, CH(NH₃), C(Me)(OH) or CH(Me) and B is CH₂, CHOH or CO,hereinafter 'compounds of formula (IB)', also form part of the invention.

The invention also provides a pharmaceutical composition comprising a compound of formula (IA) or (IB), in the free base from or a pharmaceutically acceptable acid addition or quaternary ammonium salt or N-oxide thereof, and a pharmaceutically acceptable carrier.

Some of the compounds of this invention may be crystallised or recrystallised from solvents such as organic solvents. In such cases solvates may be formed. This invention includes within its scope stoichiometric solvates including hydrates as well as compounds containing variable amounts of water that may be produced by processes such as lyophilisation.

Since the compounds of fomula (I) are intended for use in pharmaceutical compositions it will readily be understood that they are each provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 59% of a compound of the formula (I) or salt thereof.

Pharmaceutically acceptable derivatives of the above-mentioned compounds of formula (I) are the free base form or their acid addition or quaternary ammonium salts, for example their salts with mineral acids e.g. hydrochloric, hydrobromic or sulphuric acids, or organic acids, e.g. acetic, fumaric or tartaric acids. Compounds of formula (I) may also be prepared as the N-oxide.

Certain of the above-mentioned compounds of formula (I) may exist in the form of optical isomers, e.g. diastereoisomers and mixtures of isomers in all ratios, e.g. racemic mixtures. The invention includes all such forms, in particular the pure isomeric forms. For examples the invention includes compound in which an A-B group CH(OH)-CH₂ is in either isomeric configuration.

Compounds of formula (I) may be prepared by the processes described and exemplified in the above-mentioned patent publications.

In a further aspect of the invention there is provided a process for preparing compounds of formula (IA),(IB) and compounds of the examples, or a pharmaceutically acceptable derivative thereof, which process comprises:
(a) reacting a compound of formula (IV) with a compound of formula (V): 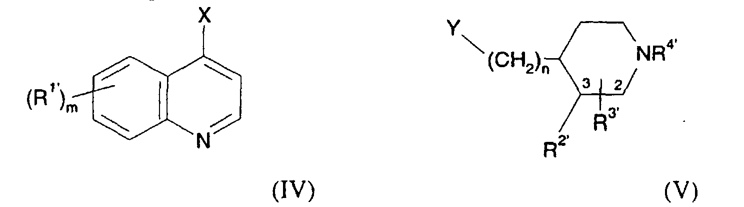 wherein m, n, R¹, R², R³ and R⁴ are as defined in formula (I), and X and Y may be the following combinations:
   (i) X is M and Y is CH₂CO₂R^{x}
   (ii) X is CO₂R^{y} and Y is CH₂CO₂R^{x}
   (iii) one of X and Y is CH=SPh₂ and the other is CHO
   (iv) X is CH₃ and Y is CHO
   (v) X is CH₃ and Y is CO₂R^{x}
   (vi) X is CH₂CO₂R^{y} and Y is CO₂R^{X}
   (vii) X is CH=PRZ^{z}₃ and Y is CHO
   (viii) X is CHO and Y is CH=PR^{z}₃
   (ix) X is halogen and Y is CH=CH₂
   (x) one of X and Y is COW and the other is NHR^{11'}
   (xi) one of X and Y is (CH₂)ₚ-V and the other is (CH₂)_{q}NHR^{11'}, (CH₂)_{q}OH, (CH₂)_{q}SH or (CH₂)_{q}SCOR^{X} where p+q=1
   (xii) one of X and Y is CHO and the other is NHR^{11'}
   (xiii) one of X and Y is OH and the other is -CH=N₂
   in which V and W are leaving groups, R^{x} and R^{y} are (C₁₋₆)alkyl and R^{z} is aryl or (C₁₋₆)alkyl;
(b) rearranging a compound of formula (II): 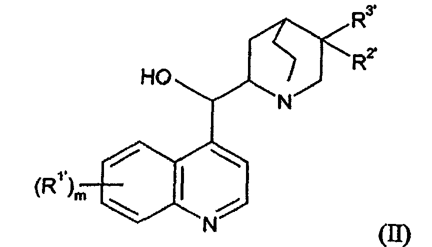 to give a compound of formula (III) which is a compound of formula (I) where R³ is in the 3-position, n is 1, A-B is COCH₂ or disubstituted epoxide and R² is H, and thereafter optionally reducing to a compound of formula (VII) which is a compound of formula (I) where n is 1, A-B is CHOHCH₂ or CH₂CHOH and R² is H;
(c) photooxygenating a compound of formula (VI): 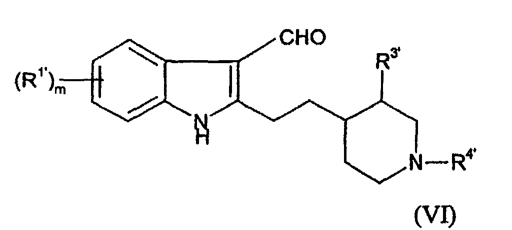 or
(d) reacting a compound of formula (IV) with a compound of formula (Vb): 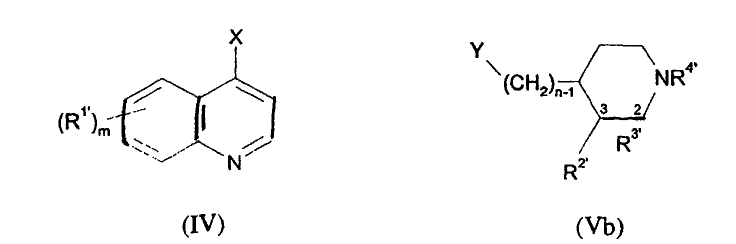
wherein m, n, R¹, R², R³ and R⁴ are as defined in formula (I), X is CH₂NHR^{11'} and Y is CHO or COW or X is CH₂OH and Y is -CH=N₂;
in which R^{11'}, R^{1'}, R^{2'}_{,} R^{3'}and R^{4'} are R¹¹, R¹, R², R³ and R⁴ or groups convertible thereto, and thereafter optionally or as necessary converting R^{11'}, R^{1'}, R^{2'}_{,} R^{3'} and R^{4'} to R^{11'}, R¹, R²_{,} R³ and R⁴_{,} converting A-B to other A-B, interconverting R¹¹, R¹, R², R³ and/or R⁴ and forming a pharmaceutically acceptable derivative thereof.

Process variants (a)(i), (a)(ii), (b) in certain aspects and (c) initially produce compounds of formula (I) where A-B is COCH₂. The product of variants (b) and (c) have n=1.

Process variants (a)(iii) and (b) in other aspects initially produce compounds of formula (I) wherein A-B is CH₂CHOH or CHOHCH₂.

Process variant (a)(iv) initially produces compounds of formula (I) wherein A-B is CH₂CHOH.

Process variants (a)(v) and (a)(vi), initially produce compounds of formula (I) wherein A-B is CH₂CO.

Process variants (a)(vii), (a)(viii) and (a)(ix) initially produce compounds where A-B is CH=CH.

Process variant (a)(x) initially produces compounds of formula (I) wherein A-B is CONHR¹¹ or NHR¹¹CO.

Process variant (a)(xi) initially produces compounds of formula (I) wherein one of A and B is CH₂ and the other is NHR¹¹, O or S.

Process variant (a)(xii), initially produce compounds of formula (I) wherein A-B is CH₂NHR¹¹ or NHR¹¹CH₂.

Process variant (a)(xiii) initially produces compounds of formul a(I) wherein A-B is OCH₂ or CH₂O.

Process variant (d) initially produces compounds of formula (I) wherein AI is CH₂ and B is NHR¹¹ or O.

In process variant (a)(i) M is preferably an alkali metal, more preferably Li. The reaction is conducted in an aprotic solvent preferably THF, ether or benzene at -78 to 25°C. An analogous route is described in iethe et al (1972) Helv. Chimica.Acta.. 55, 1044.

In process variant (a)(ii) the process is two step: firstly a condensation using a base, preferably sodium hydride or alkoxide, sodamide, alkyl lithium or lithium dialkylamide, preferably in an aprotic solvent e.g. ether, THF or benzene; secondly, hydrolysis using an inorganic acid, preferably HCl in aqueous organic solvent at 0-100°C. Analogous routes are described in DE330945, EP31753, EP53964 and H. Sargent, J. Am. Chem. Soc. **68**, 2688-2692 (1946).

In process variant (a)(iii) if a base is used it is preferably NaH, KH, an alkyl lithium e.g. BuLi, a metal alkoxide e.g. NaOEt, sodamide or lithium dialkylamide e.g.diisopropylamide. An analogous method is described in US 3989691 and in Taylor et al. (1972) JACS 94, 6218)

In process variant (a)(iv) the reaction is carried out in the presence of a base, preferably organometallic or metal hydride e.g. NaH, lithium diisopropylamide or NaOEt, preferably in an aprotic solvent, preferably THF, ether or benzene at -78 to 25°C (analogous process in Gutswiller et al. (1978) JACS 100, 576).

In process variant (a)(v) the reaction is carried out in the presence of a base, preferably organometallic or metal hydride e.g. NaH, lithium diisopropylamide or NaOEt, preferably in an aprotic solvent, preferably THF, ether or benzene at -78 to 25°C. An analogous method is described in US 3772302.

In process variant (a)(vi) a similar Claisen methodology to that described for (a)(ii) is used, analogous to that described in Soszko et. al., Pr.Kom.Mat. Przyr.Poznan.Tow.Przyj.Nauk., (1962), 10, 15.

In process variants (a)(vii) and (viii) if a base is used it is preferably NaH, KH, an alkyl lithium e.g. BuLi, a metal alkoxide e.g. NaOEt, sodamide or lithium dialkylamide e.g.di- isopropylamide. An analogous method is described in US 3989691 and M.Gates et. al. (1970) J. Amer.Chem.Soc., 92, 205, as well as Taylor et al. (1972) JACS 94, 6218.

In process variant (a)(ix) the reaction is carried out using palladium catalysis. The palladium catalyst is preferably palladium acetate in the presence of trialkyl or triaryl phosphine and a trialkylamine e.g. triphenyl phosphine and tributylamine. An analogous method is described in S. Adam et. al. (1994) Tetrahedron, 50, 3327.

In process variant (a)(x), or (d) where Y is COW,the reaction is a standard amide formation reaction:
1. Activation of a carboxylic acid (e.g., to an acid chloride, mixed anhydride, active ester, O-acyl-isourea or other species), and treatment with an amine (Ogliaruso, M. A.; Wolfe, J. F. in *The Chemistry of Functional Groups (Ed. Patai, S.) Suppl. B: The Chemistry of Acid Derivatives, Pt. l* (John Wiley and Sons, 1979), pp 442-8; Beckwith, A. L. J. in *The Chemistry of Functional Groups (Ed. Patai S.) Suppl. B: The Chemistry of Amides (Ed. Zabricky, J.)* (John Wiley and Sons, 1970), p 73 ff. The acid and amide are preferably reacted in the presence of an activating agent such as 1-(dimethylaminopropyl)-3-ethylcarbodiimide hydrochloride (EDC) or 1-hydroxybenzotriazole (HOBT).
2. Aminolysis of esters (Suzuki, K.; Nagasawa, T. in *Encyclopedia of Reagents for Organic Synthesis (Ed. Paquette. L. A.)* (John Wiley and Sons, 1995), p 5188 and refs. cited therein.)
3. The specific methods of:
   a. *in situ* conversion of an acid into the amine component by a modified Curtius reaction procedure (Shioiri, T.; Murata, M.; Hamada, Y., *Chem. Pharm. Bull.* **1987**, *35*, 2698)
   b. *in situ* conversion of the acid component into the acid chloride under neutral conditions (Villeneuve, G. B.; Chan, T. H., *Tet. Lett.* **1997**, *38*, 6489).

In process variant (d) a final reduction step provides the required amine.

In process variant (a)(xi) where one of X and Y contains NHR¹¹ the leaving group V is halogen and the reaction is a standard amine formation reaction such as direct alkylation described in (Malpass, J. R., in *Comprehensive Organic Chemistry,* Vol. 2 (Ed. Sutherland, I. O.), p 4 ff.) or aromatic nucleophilic displacement reactions (see references cited in *Comprehensive Organic Chemistry,* Vol. 6, p 946-947 (reaction index); Smith, D. M. in *Comprehensive Organic Chemistry,* Vol. 4 (Ed. Sammes, P. G.) p 20 ff.). This is analogous to the methods described in GB 1177849.

In process variant (a)(xi) where one of X and Y contains OH or SH, this is preferably converted to an OM or SM group where M is an alkali metal by treatment of an alcohol, thiol or thioacetate with a base. The base is preferably inorganic such as NaH, lithium diisopropylamide or sodium, or, for SH, metal alkoxide such as sodium methoxide. The X/Y group containing, the thioacetate SCOR^{x} is prepared by treatment of an alcohol or alkyl halide with thioacetic acid or a salt thereof under Mitsunobu conditions. The leaving group V is a halogen. The reaction may be carried out as described in Chapman et.al., J. Chem Soc., (1956),1563, Gilligan et. al., J. Med. Chem., (1992), **35**, 4344, Aloup et. al., J. Med. Chem. (1987), **30**, 24, Gilman et al., J.A.C.S. (1949), **71**, 3667 and Clinton et al., J.A.C.S. (1948), **70**, 491, Barluenga et al., J. Org. Chem. (1987) **52**, 5190. Alternatively where X is OH and Y is CH₂V. V is a hydroxy group activated under Mitsunobu conditions (Fletcher et.al. J Chem Soc. (1995), 623).

In process variants (a)(xii) and (d) where Y is CHO the reaction is a standard reductive alkylation using, e.g., sodium triacetoxyborohydride (Gribble, G. W. in *Encyclopedia of Reagents for Organic Synthesis (Ed. Paquette, L. A.)* (John Wiley and Sons, 1995), p 4649).

In process variant (a)(xiii), or (d) where X is CH₂OH and Y is -CH=N₂, the reaction is as described in den Hertzog et. al., reel.Trav. Chim. Pays-Bas. (1950),**69**, 700.

In process variant (b) the rearrangement may be effected by treatment with an acid, preferably an organic acid such as acetic acid and the reaction temperature is 80-120°C. Alternatively, in accordance with the procedure of P. Singh et al. *Indian J. Chem.* 25B, 1034-1037 (1986), the compound of formula (II) is quaternised by treatment with an alkylating agent and treated with base to give, depending upon the stereochemistry of the OH and the nature of the quaternery salt and base (see EP0035821), either the ketone or epoxide of formula (III). The use of, for example, NaOH in benzene/toluene or KOH in t-butanol gives the epoxide. Epoxides can be opened to the alcohol of formula (VII) by reduction with suitable reducing agents such as lithium aluminium hydride or hydrogenation over palladium on carbon (see EP0035821). The use of hydrogenation leads to concomitant reduction of any R³ alkylene to alkyl.

In process variant (c) the reaction is preferably carries out in an alcohol, preferably methanol under irradiation conditions which are known to generate singlet oxygen as described in M. Ihara et.al. (1988), J.Chem Soc Perkin Trans. 1, 1277.

Reduction of A or B CO to CHOH can be readily accomplished using reducing agents well known to those skilled in the art, e.g. sodium borohydride in aqueous ethanol or lithium aluminiun hydride in ethereal solution.. This is analogous to methods described in EP 53964, US 384556 and J. Gutzwiller et. al. (1978) J.Amer.Chem.Soc., 100, 576.

The carbonyl group A or B may be reduced to CH₂ by treatment with a reducing agent such as hydrazine in ethylene glycol at 130-160°C in the presence of potassium hydroxide.

Reaction of a carbonyl group A or B with an organometallic reagent yields a group where R⁶ or R⁸ is OH and R⁷ or R⁹ is alkyl.

A hydroxy group A or B may be oxidised to a carbonyl group by oxidants well known to those skilled in the art , for example, manganese dioxide, pyridinium chlorochromate or pyridinium dichromate.

An A-B group COCH₂ may be converted to COCH-halogen, by treating the ketone or a derivative with a halogenating agent, reduced to CHOHCHCI and then converted to the epoxide which may in turn be reduced to CH₂CHOH.

Methods for conversion of CH=CH by reduction to CH₂CH₂ are well known to those skilled in the art, for example using hydrogenation over palladium on carbon as catalyst. Methods for conversion of CH=CH to give the A-B group as CHOHCH₂ or CH₂CHOH are well known to those skilled in the art for example by epoxidation and subsequenct reduction by metal hydrides, hydration, hydroboration or oxymercuration.

A hydroxyalkyl group A-B CH₂CHOH or CHOHCH₂ may be dehydrated to give the group CH=CH by treatment with an acid anhydride such as acetic anhydride.

An amide group CONHR^{11'} or NHR^{11'}CO may be reduced to the amine using a reducing agent such as lithium aluminium hydride.

A ketone group may be converted to an amide CONH via the oxime by a Beckmann rearrangement (Ogliaruso, M.A.; Wolfe, J. F., *ibid.* pp 450-451; Beckwith, A. L. J., *ibid*. pp 131 ff.)

A hydroxy group A or B may be converted to azido by activation and displacement e.g. under Mitsunobu conditions using hydrazoic acid or by treatment with diphenylphosphorylazide and base, and the azido group in turn may be reduced to amino by hydrogenation.

A sulphur group A or B may be converted to the sulphoxide S(O)ₓ by oxidation with peracids or a wide range of oxidants known to those skilled in the art (see Advanced Organic Chemistry *(Ed. March, J.)* (John Wiley and Sons, 1985), p 1089 and refs. cited therein).

R¹', R²', R³' and R⁴' are preferably R¹, R²_{,} R³ and R⁴. R¹'is preferably methoxy. R²' is preferably hydrogen. R³' is preferably vinyl. R⁴' is preferably H.

Conversions of R¹', R²', R³'and R⁴' and interconversions of R¹, R², R³ and R⁴ are conventional. In compounds which contain an optionally substituted hydroxy group, suitable conventional hydroxy protecting groups which may be removed without disrupting the remainder of the molecule include acyl and alkylsilyl groups.

For example R^{1'} methoxy is convertible to R^{1'} hydroxy by treatment with lithium and diphenylphosphine (general method described in Ireland et. al. (1973) J.Amer.Chem.Soc.,7829) or HBr. Alkylation of the hydroxy group with a suitable alkyl derivative bearing a leaving group such as halide and a protected amino, piperidyl, amidino or guanidino group or group convertible thereto, yields, after conversion/deprotection, R¹ (C₁₋₆) alkoxy substituted by optionally N-substituted amino, piperidyl, guanidino or amidino.

R^{3'} alkenyl is convertible to hydroxyalkyl by hydroboration using a suitable reagent such as 9-borabicyclo[3.3.1]nonane, epoxidation and reduction or oxymercuration.

R³ 1,2-dihydroxy can be prepared from R³' alkenyl using osmium tetroxide or other reagents well known to those skilled in the art (see Advanced Organic Chemistry *(Ed. March, J.*) (John Wiley and Sons, 1985), p 732-737 and refs. cited therein) or epoxidation followed by hydrolysis (see Advanced Organic Chemistry *(Ed. March, J.)* (John Wiley and Sons, 1985), p 332.333 and refs. cited therein).

R³ vinyl can be chain extended by standard homologation e.g by conversion to hydroxyethyl followed by oxidation to the aldehyde which is then subjected to a Wittig reaction.

Compounds of formula (I) where R² and R³ are a divalent residue =CR^{5¹}R^{6¹} can be prepared by treatment of a compound of formula (I) where R³ is alken-1-yl with a strong base in an aprotic solvent. Suitable bases include Ph₂PLi/PhLi (as described in Ireland et. al., J. Amer. Chem .Soc. (1973), 7829), t-BuLi, and suitable solvents include THF and ether.

Substituents on R³ alkyl or alkenyl may be interconverted by conventional methods, for example hydroxy may be derivatised by esterification, acylation or etherification. Hydroxy groups may be converted to halogen, thiol, alkylthio, azido, alkylcarbonyl, amino, aminocarbonyl, oxo, alkylsulphonyl, alkenylsulphonyl or aminosuiphonyl by conversion to a leaving group and substitution by the required group, hydrolysis or oxidation as appropriate or reaction with an activated acid, isocyanate or alkoxyisocyanate. Primary and secondary hydroxy groups can be oxidised to an aldehyde or ketone respectively and alkyated with a suitable agent such as an organometallic reagent to give a secondary or tertiary alcohol as appropriate.

In a further aspect the invention provides a process for the preparation of a compound of formula (IA) or a pharmaceutically acceptable derivative thereof which comprises converting a compound of formula (I) in which R³ is alkenyl in to a compound of formula (IA) and forming a pharmaceutically acceptable derivative thereof.

NH is converted to NR⁴ by conventional means such as alkylation with an alkyl halide in the presence of base, acylation/reduction or reductive alkylation with an aldehyde.

It will be appreciated that under certain circumstances interconvertions may interfere, for example, A or B hydroxy groups and the piperidine NH will require protection e.g. as a carboxy- or silyl-ester group for hydroxy and as an acyl derivative for piperidine nitrogen, during conversion of R^{1'}, R^{2'}, R^{3'} or R^{4'}.

Examples containing a *trans*-3,4-substituted piperidine ring may be prepared from the trans-3-vinyl-4-substituted piperidine prepared from the corresponding 3-vinyl-4-*cis*-isomer by the method of G. Engler *et al.* Helv. Chim. Acta **68**, 789-800 (1985); also described in Patent Application EP 0031753 (Pharmindustrie).

The method involves heating a 3-vinyl-4-alkyl-piperidine derivative of formula (VIII): 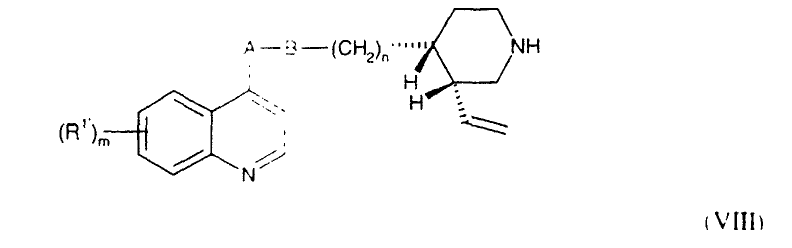 (prepared as an intermediate in the process of the invention) in dilute acid, preferably hydrochloric acid at pH 3.5, with 0.3-1.0 mol equivalents of formaldehyde. The main product of the reaction is the *trans*-isomer, which may be separated from the small quantity of *cis* isomer present, by conventional silica gel chromatography. It is convenient to convert the mixture of cis- and *trans*-piperidines (R⁴'= H) to the tertiary amines of formula (I) by alkylation with an alkyl halide (preferably an iodide) in DMF in the presence of anhydrous potassium carbonate, prior to silica gel chromatography.

Compounds of formula (II) include quinine and derivatives thereof.

Compounds of formulae (IV), (V) and (Vb) are known compounds or prepared analogously, see for example the references cited above for reaction variant (a).

For compounds of formula (V) where Y is NHR^{11'} suitable amines may be prepared from the corresponding acid or alcohol (Y is CO₂H or CH₂OH). In a first instance, an N-protected piperidine containing an acid bearing substituent, can undergo a Curtius rearrangement and the intermediate isocyanate can be converted to a carbamate by reaction with an alcohol. Conversion to the amine may be achieved by standard methods well known to those skilled in the art used for amine protecting group removal. For example, the t-butoxycarbonyl-protected 3-vinyl-4-piperidine acetic acid can undergo a Curtius rearrangement e.g. on treatment with diphenylphosphoryl azide and heating, and the intermediate isocyanate reacts in the presence of 2-trimethylsilylethanol to give the trimethylsilylethylcarbamate (T.L.Capson & C.D.Poulter, Tetrahedron Letters,1984, 25, 3515). This undergoes cleavage on treatment with tetrabutylammonium fluoride to give the 4-piperidinemethylamine. Alternatively, an acid group (CH₂)ₙ₋₁CO₂H may be converted to (CH₂)ₙNHR₁₁ by reaction with an activating agent such as isobutyl chloroformate followed by an amine R^{11'}NH₂ and the resulting amide reduced with a reducing agent such as LiAlH₄.

In a second instance, an N-protected piperidine containing an alcohol bearing substituent undergoes a Mitsunobu reaction (for example as reviewed in Mitsunobu, Synthesis, (1981), 1), for example with succinimide in the presence of diethyl azodicarboxylate and triphenylphoaphinr to give the phthalimidoethylpiperidine. Removal of the phthaloyl group, for example by treatment with methylhydrazine, gives the amine of formula (V).

Compounds of formula (VI) are known compounds or may be prepared analogously, see for example Ihara et al JCS Perkin **1** 1988. 1277-1281.

Conversions of R^{1'}, R²' and R^{3'} may be carried out on the intermediates of formulae (II), (III),(IV), (V), (Vb) and (VI) prior to their reaction to produce compounds of formula (I) in the same way as described above for conversions after their reaction.

For example, a compound of formula (II) where R^{3'} is an alken-1-yl group can be isomerised to a compound where R^{2'} and R^{3'} together are a divalent residue = CR^{5¹} R^{6¹} with acids such as HBr by methods analogous to that described in Diaz-Arauzo et. al. J. Natural Products, (1990), **53**, 112, and then treated with subsequent conversion to compounds of formula (III) as described inRenfrew and Butler, J. Amer. Chem.Soc., (1940), **62**, 3304.

Diaz-Arauzo et. al. J. Natural Products, (1990), **53**, 112, describe preparation of compounds where R3' in compounds of formula (II) is 1,2-dihydroxyethyl from R³' is vinyl using osmium tetroxide.

Where a *trans*-substituted compound of formula (I) is required, a *trans*-substituted piperidine moiety of formula (V) may be prepared from the corresponding *cis* isomer of formula (V) having an R³' vinyl group in the 3-position by heating in formaldehyde with a substituent that can subsequently be converted to the required group (CH₂)ₙY, for example CH₂CO₂R (where R is an alkyl group eg methyl or ethyl).

The pharmaceutical compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable salt or *in vivo* hydrolysable ester thereof is administered in the above-mentioned dosage range.

The compound of formula (I) may be the sole therapeutic agent in the compositions of the invention or a combination with other antibiotics or with a β-lactamase inhibitor may be employed.

Compounds of formula (I) are active against a wide range of organisms including both Gram-negative and Gram-positive organisms.

The following examples illustrate the preparation of certain compounds of formula (I) and the activity of certain compounds of formula (I) against various bacterial organisms.

Compounds of formula (IA),(IB) and compounds of the examples also form part of the invention.

### Example 1. [3R,4R]-3-Ethyl-1-hexyl-4-[3-oxo-3-(6-methoxyquinolin-4-yl)propyl]piperidine.

### (a) [3R,4R] 3-Ethyl-4-[3-oxo-3-(6-methoxyquinolin-4-yl)propyl]piperidine.

Hydroquinidine hydrochloride (20g) was dissolved in glacial acetic acid (18ml) and water (150ml) was added. The reaction mixture was refluxed and stirred for 48 hours. The mixture was poured into ice (50g) and sodium hydroxide was added until the solution reached pH12. The mixture was extracted with toluene (3x50ml) then the organic layer was washed with brine, dried over anhydrous magnesium sulphate and evaporated to give an oil. The product was chromatographed on Kieselgel 60 eluting with chloroform/acetone/diethylamine (10:8:1) and gave the title compound (9.56g). δ_{H} (CDCl₃) 8.82 (d), 8.80 (d), 7.80 (d), 7.50 (d), 7.38 (dd), 3.90 (s), 3.05-2.85 (m), 2.50-2.65 (m), 1.62-1.80 (m), 1.20-1.55 (m), 0.89 (t);
mass spectrum El, M⁺, 326;C₂₀H₂₆N₂O₂ requires M, 326.

### (b) Title compound

The product from Example 1a (2g) was dissolved in toluene (7ml) and potassium carbonate (1.68g) and 1-bromohexane (1.03ml) were added under nitrogen. The reaction mixture was stirred and refluxed for 7 hours. Water (50ml) was added and the aqueous phase extracted with toluene (2x75ml). The organic layer was washed with brine, dried over anhydrous magnesium sulphate and evaporated to give an oil. The product was chromatographed on Kieselgel 60 eluting with chloroform/acetone/diethylamine (20:4:1) and gave the title compound (1.9g).
δ_{H} (CDCl₃) 8.84 (d), 8.03 (d), 7.80 (d), 7.60 (d), 7.40 (dd), 3.92 (s), 3.02 (ABq) 2.55 (m), 2.40-2.10(m), 1.80-1.40 (m), 1.30 (m), 0.90 (2t) ;
mass spectrum El M⁺, 410, C₂₆H₃₈N₂O₂ requires M,410.

### Example 2. [3R,4R]-3-Ethyl-1-hexyl-4-[3-(R,S)-hydroxy-3-(6-methoxyquinolin-4-yl)propyl]piperidine.

The product from Example 1b (4. 1g) was dissolved in isopropanol (20ml) cooled to -10°C and a suspension of sodium borohydride (443mg) in isopropan-1-ol (45ml) was added in such a way that the temperature did not exceed -5°C. The reaction mixture was stirred for 2hr at -10°C and then water (50ml) was added and the mixture extracted with chloroform (3x100ml). The organic layers were washed with water, brine and then dried over anhydrous magnesium sulphate and finally evaporated to give an oil. The product was chromatographed on Kieselgel 60 eluting with chloroform/diethylamine (20:1) and gave the title compound (3.6g).
δH (d₆DMSO) 8.73 (d), 7.96 (d), 7.57 (d), 7.38 (m), 5.30 (m), 3.91 (s), 2-1.4 (br m), 1.4-1.2 (m), 1-0.7 (m).
The individual diastereomers were separated by preparative HPLC using a Chiralpak AD column.

### Example 3. [3R,4R]-3-Ethyl-1-heptyl-4-[3-oxo-3-(6-methoxyquinolin-4-yl)propyl]piperidine.

The product from Example 1a (6.53g) was alkylated using method of Example 1b with 1-bromoheptane (4.3g) to give an oil. The product was chromatographed on Kieselgel 60 eluting with chloroform/acetone/diethylamine (20:4:1) and gave the title compound (6.12g).
δ (CD₃OD) 6.80 (d), 7.97 (d), 7.65 (dd), 7.45 (dd), 4.92 (s), 3.94 (s), 3.31 (m), ca 3.11 (br m), 2.5-2.1 (br m), 1.8-1.4 (br m), 1.3-1.2 (m), 1.0-0.8 (m).

### Example 4. [3R,4R]-3-Ethyl-1-heptyl-4-[3-(R,S)-hydroxy-3-(6-methoxyquinolin-4-yl)propyl]piperidine.

The product from Example 3 (6.1g) was reduced by the method of Example 2 to give an oil. The product was chromatographed on Kieselgel 60 eluting with chloroform/diethylamine (20:1) and gave the title compound (5.25g).
δ_{H} (d₆DMSO) 8.70 (d), 7.95 (d), 7.58 (m), 7.38 (m), 3.88 (s), 3.81 (s), 3.40-2.6 (br m), 1.9-1.4 (br m), 1.23 (m), 0.9-0.7 (m)
The individual diastereomers were separated by preparative HPLC using a Chiralpak AD column.

### Example 5. [3R,4R] 3-Ethyl-1-octyl-4-[3-oxo-3-(6-methoxyquinoIin-4-yl)propyl]piperidine.

The product from Example 1a (2g) was alkylated with 1-bromo octane (1.26ml) using the method of Example 1b to give an oil. The product was chromatographed on Kieselgel 60 eluting with chloroform/diethylamine (20:1) and gave the title compound (2.15g).
δ_{H} (CDCl₃) 8.86 (d) 8.05 (d), 7.83 (d), 7.60 (d), 7.41 (dd), 3.94 (s), 3.02 (ABq), 2.60 (m), 2.36-2.00 (m), 1.80-1.20 (m), 0.91 (t), 0.89 (t);
mass spectrum EI *M*⁺, 438. C₂₈H₄₂N₂O₂ requires M, 438.

### Example 6. [3R,4R]-3-Ethyl-1-octyl-4-[3-(R,S)-hydroxy-3-(6-methoxyquinolin-4-yl)propyl]piperidine.

The product from Example 5 (1.15g) was reduced by the method of Example 2 to give an oil. The product was chromatographed on Kieselgel 60 eluting with chloroform/diethylamine (20:1) and gave the title compound (0.8g).
δ_{H} 50/50 mixture of the two isomers (CDCl₃) 8.54 (d) 7.92 (d), 7.44 (2d), 7.29 (dd), 7.14, 7.19 (2d), 5.24 (m), 4.6 (m), 3.88 (s), 2.43 (m), 2.3-1.6 (m), 1.6-1.1 (m), 0.90-0.75 (m);
mass spectrum El M⁺, 440. C₂₈H₄₄N₂O₂ requires M, 440.

### Example 7. [3R,4R]-3-Ethyl-1-decyl-4-[3-oxo-3-(6-methoxyquinolin-4-yl)propyl]piperidine.

The product from Example 1a (2g) was alkylated with 1-bromo decane (1.51ml) using the method of Example 1b to give an oil. The product was chromatographed on Kieselgel 60 eluting with chloroform/diethylamine (20:1) and gave the title compound (1.61g).
δ_{H} (CDCl₃) 8.86 (d), 8.05 (d), 7.82 (d), 7.59 (d), 7.41 (dd), 3.96 (s), 3.05 (ABq), 2.57 (m), 2.36-2.00 (m), 1.80-1.15(m), 0.93 (t), 0.89 (t);
mass spectrum El *M*+. 466. C₃₀H₄₆N₂O₂ requires M, 466.

### Example 8. [3R,4R]-3-Ethyl-1-decyl-4-[3-( R.S)-hydroxy-3-(6-methoxyquinolin-4-yl)propyl]piperidine.

The product from Example 7 (1. 1g ) was reduced using the method of Example 2 give an oil. The product was chromatographed on Kieseigel 60 eluting with chloroform/diethylamine (20.5:5) and gave the title compound (1.13g).
δ_{H}(CDCl₃) 50/50 mixture of the two isomers : 8.65 (d), 8.00 (d), 7.50, 7.48 (2d), 7.34 (dd), 7.24, 7.20 (2d), 5.30 (m), 3.91 (s), 3.55 (m), 2.60-1.70 (m), 1.70-1.10 (m) 0.92-0.78 (m);
mass spectrum EI *M*⁺, 468. C₃₀H₄₈N₂O₂ requires M, 468.

### Example 9. [3R,4R]-3-Ethyl-l-dodecyl-4-[3-oxo-3-(6-methoxyquinolin-4-yl)propyl]piperidine.

The product from Example 1a (2g) was alkylated with 1-bromo dodecane using the method of Example 1b to give an oil. The product was chromatographed on Kieselgel 60 eluting with chloroform/diethylamine (20:1) and gave the title compound (1.42g).
δ_{H}(CDCl₃) 8.86 (d), 8.04 (d), 7.82 (d), 7.59 (d), 7.41 (dd), 3.95 (s), 3.02 (ABq), 2.58 (m), 2.35-2.00 (m), 1.80-1.20 (m), 0.91(t), 0.89 (t);
mass spectrum EI *M*⁺, 494. C₃₂H₅₀N₂O₂ requires M, 494.

### Example 10. [3R,4R] 3-Ethyl-1-dodecyl-4-[3-(R,S)-hydroxy-3-(6-methoxyquinolin-4-yl)propyl]piperidine.

The product from Example 9 (1.17g) was reduced using the method of Example 2 to give an oil. The product was chromatographed on Kieselgel 60 eluting with chloroform/diethylamine (24:1) and gave the title compound (1.13g).
δ_{H} (CDCl₃) 50/50 mixture of the two isomers: 8.62 (d), 8.00 (d), 7.50, 7.45 (2d), 7.32 (dd), 7.23, 7.21, (2d), 5.30 (m), 3.91 (s), 3.67 (m), 2.50 (m), 2.35-1.70 (m) 1.70-1.10 (m); 0.95-0.80 (m);
mass spectrum EI *M*⁺, 496. C₃₂H₅₂N₂O₂ requires M, 496.

### Example 11. [3R,4R]-3-Ethyl-1-cinnamyl-4-[3-(R,S)-hydroxy-3-(6-methoxyquinolin-4-yl)propyl]piperidine.

### (a) [3R,4R]-3-Ethyl-4-[3-(R,S)-hydroxy-3-(6-methoxyquinolin-4-yl)propyl]piperidine.

The product from Example 1a (500mg) was reduced using the method of Example 2 to give a pale yellow oil. Column chromatrography on silica gel gave the title compound (315mg) as a white foam.
δ_{H} (CDCl₃) 8.69 (d, *J*4.5Hz, 1H), 8.0 (d, *J*9.2Hz, 1H), 7.48 (m, 1H), 7.34 (dd, *J* 9.2, 2.7 Hz. 1H), 7.22 (m. 1H), 5.3 (m, 1H), 3.91 (s, 3H); El *M*+, 328 (found: M⁺ 328.2149.C₂₀H₂₈N₂O₂ requires 328.2151).

### (b) Title compound.

The product from Example 11a (205mg) was alkylated with cinnamyl bromide using the method of Example 1b to give a brown oil. Column chromatography on silica gel gave the title compound (73mg) as a pale brown foam. δ_{H} (CDC1₃) 6.5 (d, *J* 15.8Hz, 1H), 6.26 (dt, *J* 22.5, 7Hz, 1H), 3.09 (ddd, *J* 27.8, 13.5, 6 Hz, 1H); El, *M*⁺444 (found: M⁺ 444.2775.C₂₉H₃₆N₂O₂ requires 444.2777).

### Example 12. [3R,4R]-3-Ethyl-1.heptyl.4-[3-(6-methoxyquinolin-4-yl)propyl]piperidine.

### (a) [3R,4R]-3-Ethyl-4-[3-(6-methoxyquinolin-4-yl)propyl]piperidine.

The product from Example 1a (7.5g) was dissolved in ethylene glycol (20ml) and hydrazine hydrate (1.6ml) was added. The reaction mixture was heated to 145° under a stream of argon for 2 hours. A further 1ml of hydrazine hydrate was added and heating continued for 2 hours. Solid potassium hydroxide (3g) was added over 10 min. and the mixture reheated to 145° for a further 2 hour during which time nitrogen was evolved. The mixture was allowed to cool to room temperature and water (30ml) was added. The mixture was extracted with toluene (4 x 50ml) and the combined extracts dried over anhydrous magnesium sulphate, filtered and evaporated to give a brown oil. Column chromatography on silica gel gave the title compound (5.2g) as an oil.
δ_{H} (CDCl₃) 8.66 (d, *J* 4.4Hz). 8.01 (d, *J* 9.2Hz, 1H), 7.37 (dd, *J* 9.2, 2.8Hz, 1H), 7.22 (dd, *J*7.8, 2.7 Hz. 1H), 3.95 (s, 3H); EI *M*⁺312 (found: M⁺ 312.2207.C₂₀H₂₈N₂O requires 312.2202).

### (b) Title compound

The product from Example 12a (312mg) was dissolved in dry dimethylformamide (5ml) and n-heptyl bromide (0.172ml) and potassium carbonate (76mg) were added. The reaction mixture was heated to 80° with stirring for 2 hours, allowed to cool, diluted with water (10ml) and extracted with ethyl acetate (3 x 20ml). The combined organic extracts were dried over anhydrous magnesium sulphate, filtered and evaporated. Column chromatography on silica-gel gave the title compound (283mg) as a white foam.
δ_{H}(CDCl₃) 8.68 (d. *J* 4.4Hz, 1H), 8.02 (d, *J* 9.2Hz, 1H), 7.37 (dd, *J* 9.2, 2.8 Hz, 1H), 7.21 (dd, *J* 9.0,4.5 Hz, 1H), 3.95 (s, 3H), 3.01 (m, 1H): El *M*⁺410 (found: M⁺ 410.3299. C₂₇H₄₂N₂O requires 410.3297).

### Example 13. [3R,4R]-3-Ethenyl-1-heptyl-4-[3-(R,S)-hydroxy-3-(6-methoxyquinolin-4-yl)propyl]piperidine

### (a) [3R,4R]-3-Ethenyl-4-[3-oxo-3-(6-methoxyquinolin-4-yl)propyl]piperidine.

Quinidine (16.4g) was dissolved in glacial acetic acid (15ml) and water was added (125ml). The reaction mixture was stirred and refluxed for 48 hours; On cooling the pH of the mixture was adjusted to 12 by the addition of sodium hydroxide followed by extraction with toluene (3 x 150ml). The organic layers were combined, dried with anhydrous magnesium sulphate filtered and evaporated to give a brown oil.

### (b) [3R,4R]-3-Ethenyl-4-[3-(R,S)-hydroxy-3-(6-methoxyquinolin-4-yl)propyl]piperidine.

The crude product 13a (16g) was reduced by the method of Example 2 to give a pale yellow oil. Column chromatography on silica gel gave the title compound (12.95g) as a white foam.
δ_{H} (CDCl₃) 8.67 (d, *J*4.5Hz, 1H), 8.0 (d, *J* 9.2Hz, 1H), 7.46 (t, *J*4Hz, 1H), 7.35 (dd, *J* 9.2, 2.7Hz, 1H), 7.22 (dd, *J* 9.3,2.7Hz, 1H), 6.01 (m, 1H), 5.27 (m, 1H), 5.02 (m, 2H), 3.91 (s, 3H); EI *M*⁺ 326 (found: M⁺ 326.1994. C₂₀H₂₆N₂O₂ requires 326.1994).

### (c) Title compound

The product from Example 13b (12.7g) was alkylated as for Example 3. Column chromatography on silica gel gave the title compound (13.9g) as a white foam.
δ ¹H (CDCl₃) 8.69 (d, *J*4.5 Hz, 1H), 8.0 (d, J 9.2 Hz, 1H), 7.47 (t, *J* 4.3 Hz, 1H), 7.35 (dd, *J* 9.2, 2.7 Hz, 1H), 7.22 (dd, *J* 9.6. 2.7 Hz, 1H), 6.09 (m, 1H), 5.3 (m, 1H). 5.0 (m, 2H), 3.91 (s, 3H), 0.86 (t, *J* 6.3 Hz, 3H); EI *M*⁺ 424 (found: M⁺424.3096.C₂₇H₄₀N₂O₂ requires 424.3090).

### Example 14. [3R,4R]-3-Ethyl-1-heptyl-4-[3-(R,S)-hydroxy-3-(6-hydroxyquinolin-4-yl)propyl]piperidine

Lithium wire (700mg) was dissolved with stirring in dry tetrahydrofuran (25ml) containing triphenylphosphine (5g) and the mixture stirred at room temperature for 3 hours. The product from Example 4 (426mg) was dissolved in dry tetrahydrofuran (3ml) and 0.694ml of a 1.04M solution of diphenylphosphine in benzene was added followed by 1.87ml of the deep red solution resulting from the reaction of the lithium wiere with triphenylphosphine (as described above).The resulting bright red solution was heated under argon at reflux for 3 hours. A further 0.694ml of a 1.04M solution of diphenylphosphine in benzene and 1.87ml of the solution resulting from the reaction of the lithium wire with triphenylphosphine were added and heating under reflux was continued for 17 hour. The red solution was diluted with chloroform (20ml) and water (10ml) was added. The pH of the reaction mixture was adjusted to 9 with concentrated hydrochloric acid and the mixture shaken. The aqueous layer was removed and the process repeated with water (1 x 10ml) and brine (1x10ml). The organic layer was dried over anhydrous magnesium sulphate, filtered and evaporated to give an orange oil. Column chromatography on silica gel eluting with ethanol/chloroform/0.880 aqueous ammonia solution (15/86/1) gave the title compound (256mg) as a pale cream foam. δ_{H} (CDCl₃) 8.51 (m, 1H), 7.93 (m, 1H), 7.48 (m, 1H), 7.28 (m, 1H), 5.16 (brm, 1H); EI MH⁺ 413 (found: MH⁺ 413.3166. C₂₆H₄₁N₂O₂ requires 413.3168).

### Example 15. [3R,4R]-1-Heptyl-3-(2-hydroxyethyl)-4-[3-(R,S)-hydroxy-3-(6-methoxyquinolin-4-yl)propyl]piperidine

The product from Example 13c (424 mg) was treated with 9-borabicyclo[3.3.1]nonane (2.2 ml of a 0.5M solution in tetrahydrofuran) and the reaction mixture was refluxed for 6 hours. The cooled reaction mixture was then treated with ethanol (3 ml), 6 M aqueous sodium hydroxide solution (1 ml) and 30% hydrogen peroxide solution (2 ml). The reaction mixture was stirred at room temperature for 1 hour and extracted with ethyl acetate (3 x 10 ml). The combined organic layers were dried over anhydrous magnesium sulphate and evaporated to give an oil. Column chromatography on silica gel gave the title compound (293 mg) as an oil.
δ_{H} (CDCl₃) 8.71 (m, 1H), 8.02 (d, *J* 9.2Hz, 1H), 7.50 (t, *J* 4.1 Hz, 1H), 7.38 (dd, *J* 9.2, 2.8 Hz, 1H), 7.23 (dd, *J* 4.1, 2.8 Hz, 1H), 5.32 (m, 1H), 3.93 (s, 3H), 3.8 (m, 4H), 3.64 (m, 2H), 2.28 (m, 2H); EI M⁺ 442 (found: M⁺ 442.3195. C₂₇H₄₂N₂O₃ requires 442.3195).
The individual diastereomers were separated by preparative HPLC using a Chiralpak AD column.

### Reference Example 16. [3R,4R]-3-Ethyl-1-heptyl-4-[3(R,S)-hydroxy-3-(6-[5-phthalimidopentyloxy]-quinolin-4-yl)propyl]piperidine.

The product from Example 14 (52mg) was dissolved in dry dimethylformamide (2ml), *N*-5-bromopentylphthalimide (41mg) and potassium carbonate (19mg) were added and the mixture stirred and heated to 80° for 4 hours. The mixture was allowed to cool to room temperature, diluted with water (20ml), the pH adjusted to 12 with potassium carbonate and extracted with ethyl acetate (3 x 50ml). The combined organic layers were dried over anhydrous magnesium sulphate, filtered and evaporated to give a red oil. Colum chromatography on silica-gel eluting with chloroform:ethanol: 0.880 aqueous ammonia solution (84:15:1) gave the title compound (44mg).
δ_{H} (CDCl₃) 8.71 (d, *J* 4.5 Hz, 1H), 7.98 (d,*J* 9.2 Hz, 1H), 7.82 (m_{,} 2H), 7.71 (m, 2H)_{,} 7.48 (m, 1H), 7.31 (m, 1H), 7.23 (m, 1H), 5.32 (m, 1H), 4.05 (m, 2H), 3.75 (t, *J* 7.0 H_{z,} 2H); EI M⁺ 627 (found: M⁺ 627.4023.C₃₉H₅₃N₃O₄ requires 627.4036).

### Example 17. [3R,4R]-3-Ethyl-1-heptyl-4-[3-(R,S)-hydroxy-3-(6-[5-aminopentyloxy]-quinolin-4-yl)propyl]piperidine.

The product from Reference Example 16 (4.68g) was dissolved in methanol (20ml) and the solutuion cooled to -40°. Methyl hydrazine (0.794ml) was added and the mixture stirred at -40° for 15 minutes and 2 hours at room temperature. The mixture was evaporated to a red-brown oil, dissolved in methanol (5ml) and fresh methyl hydrazine (800ul) added. After 45 minutes stirring, the reaction mixture was evaporated to low volume and kept under high vacuum for 2 hours to give a red foam. Column chromatography on silica-gel eluting with chloroform:ethanol: 0.880 aqueous ammonia solution (83.5:15:1.5) gave the title compound (3.14g) as a white foam.
δ_{H} (CDCl₃) 8.71 (d, *J* 4.5 Hz, 1H), 8.0 (d, *J* 9.2 Hz, 1H), 7.49 (t, *J* 4.1 Hz, 1H), 7.35 (m, 1H), 7.26 (m, 1H), 5.30 (m. 1H), 4.05 (t, *J* 6.3 Hz, 2H), 2.71 (br t, *J* 6.5 Hz, 2H); CI MH⁺ 498.

### Example 18. [3R,4R]-3-Ethyl-1-heptyl-4-[3-(R,S)-hydroxy-3-(6-[2-amino-2-oxo-1,1-dimethyl]ethoxyquinolin-4-yl)Propyl]-4-yl]piperidine.

The product from Example 14 (1.39g) was dissolved in dry dioxan (17ml) and sodium hydride (149mg of 60% dispersion in oil) added. The mixture was stirred at room temperature for 1 hour and then 2-bromo-2-methyl-pmpionamide (560mg) was added and the mixture stirred and heated to 100° for 4 hours. The reaction mixture was allowed to cool to room temperature, the solid sodium bromide filtered off and the dioxan was removed *in vacuo*. The residue was dissolved in chloroform (20ml) and washed with water (2 x 20ml), dried over anhydrous magnesium sulphate, filtered and evaporated to give a brown foam. Column chromatography on silica-gel eluting with chloroform:ethanol:0.880 aqueous ammonia solution (83.5:15:1.5) gave the title compound (853mg) as a white foam.
δ_{H} (CDCl₃) 8.79 (d, *J* 4.5 Hz, 1H), 8.04 (d, *J* 9.15 Hz, 1H), 7.51 (d, *J* 4.5 Hz, 1H), 7.45 (t, *J* 2.7 Hz, 1H), 7.36 (dd, *J* 9.1, 2.6 Hz, 1H), 6.61 (br s, 1H), 5.69 (br s, 1H), 5.26 (br m, 1H), 1.62 (s, 6H); El M⁺497 (found: M⁺ 497.3620.C₃₀H₄₇N₃O₃ requires 497.3617).

### Reference Example 19. [3R,4R]-3-Ethyl-1-heptyl-4-[3-(R,S)-hydroxy-3-(6-[2-hydroxy-2-methyl-propionamido]quinolin-4-yl)propyl]piperidine.

The product from Example 18 (574mg) was dissolved in dry, 1,3-dimethyl-3,4,5,6-tetrahydro-2(1H)-pyrimidinone (0.74ml) in dry dimethylformamide (7.4ml) and sodium hydride (52mg, 60% dispersion in oil) was added. The mixture was stirred and heated to 100° for 3 hours, allowed to cool to room temperature, diluted with water (20 ml) and extracted with chloroform (3x 50 ml). The combined organic layers were dried over anhydrous magnesium sulphate, filtered and evaporated to give a brown oil. Column chromatography on silica-gel eluting with chloroform : ethanol:0.880 aqueous ammonia (83.5:15:1.5) gave the title compound (257mg).
δ_{H} (CDCl₃) 9.02 (s, 1H), 8.73 (d, *J* 4.5 Hz, 1H, 8.51 (dd, *J* 8.7, 2.1 Hz, 1H), 8.0 (d, *J* 9.0 Hz, 1H), 7.64-7.50 (m, 2H), 5.37 (m, 1H), 2.54 (br s, 1H), 1.61 (s, 6H).

### Example 20. [3R,4R]-3-Ethyl-1-heptyl-4-[3-(R,S)-hydroxy-3-(6-aminoquinolin-4-yl)propyl]piperidine

The product from Reference Example 19 (723mg) was dissolved in dioxan (25ml) and 5M hydrochloric acid (25ml) was added. The mixture was stirred and heated to 100° for 2 hours, allowed to cool to room temperature, 10% aqueous sodium carbonate was added to pH12, and extracted with dichloromethane (3 x 50ml). The combined organic layers were dried over anhydrous magnesium sulphate, filtered and evaporated to give a solid. Column chromatography on silica-gel, eluting with chloroform:ethanol: 0.880 aqueous ammonia solution (83.5:15:1.5) gave the title compound (562mg) as a pale brown foam.
δ_{H} (CDCl₃) 8.59 (d, *J* 4.5 Hz, 1H), 7.89 (d, *J* 8.9 Hz, 1H), 7.41 (m, 1H), 7.11 (dd, *J* 8.9, 2.3 Hz, 1H), 7.02 (dd, *J* 8.8, 2.4 Hz, 1H), 5.21 (m, 1H), 4.05 (br s, 2H), 2.56 (br s, 2H); El M⁺ 411 (found: M⁺ 411.3233.C₂₆H₄₁N₃O requires 411.3249).

### Example 21. [3R,4R]-3-Ethyl-1-heptyl-4-[3-(R,S)-hydroxy-3-(6-azidoquinolin-4-yl)propyl] piperidine.

The product from Example 20 (20mg) was dissolved in 2M hydrochloric acid (1ml) and the solution was cooled to 0° with stirring. Solid sodium nitrite (14mg) was added to the yellow solution which became colouress. The mixture was stirred at 0° for 10 minutes and then sodium azide (40mg) was added. The mixture was allowed to warm to room temperature and stirred for 1 hour. The mixture was diluted with saturated aqueous sodium carbanate solution (5ml) and extracted with dichloromethane (3 x 15ml). The combined organic layers were dried over anhydrous magnesium sulphate, filtered and evaparated to give the title compound (15mg) as a yellow oil. δ_{H} (CDCl₃) 8.78 (d, *J* 4.5 Hz, 1H), 8.10 (d, *J* 9.0 Hz, 1H), 7.56 (m, 2H), 7.39 (dd, *J* 9.0, 2.4 Hz, 1H), 5.32 (m, 1H), 2.53 (br s, 1H); EI M⁺ 437 (found: M⁺ 437.3168.
C₂₆H₃₉N₅O requires 437.3155)

### Example 22. [3R,4R]-3-Ethyl-1-heptyl-4-(3-(6-hydroxyquinolin-4-yl)propyl]piperidine.

### (a) [3R,4R]-3-Ethyl-4-[3-(6-hydroxyquinolin-4-yl)propyl]piperidine.

The product from Example 12a (7.0g, 0.022mole) in 47% hydrobromic acid (100ml) was heated at 150°C for 18 hours. The solvent was removed *in vacuo* and the residue basified with saturated sodium hydrogen carbonate solution and evaporated *in vacuo.* The residue was dissolved in methanol, evaporated *in vacuo* and purified by column chromatography eluting with 20% (9:1 methanol:.880 ammonia)/dichloromethane to afford the title compound (5.5g, 82%) as a grey solid.
δ_{H} (CDCl₃) 9.92 (s, 1H), 8.77-8.00 (br s, 1H), 8.53 (d, *J* 6 Hz, 1H), 7.84 (d, *J* 10 Hz, 1H), 7.3-7.19 (m, 3H), 3.43-3.15 (m ,2H), 3.11-2.76 (m, 5H) 1.91-1.50 (m, 5H), 1.49-1.13 (m, 4H), 0.92-0.75 (m, 3H)

### (b). Title compound.

The product from Example 22a (3.5g, 0.012 mole) in methanol (100ml) was treated with heptaldehyde (1.6ml, 0,012 mole) and stirred at room temperature for 18 hours. Sodium triacetoxyborohydride (3.72g, 0.018mole) was added and the mixture stirred at room temperature for 4 hours. The solvent was removed in vacuo and the residue partitioned between water and dichloromethane. The dichloromethane layer was dried (magnesium sulphate) and evaporated in vacuo. The residue was purified by column chromatography eluting with 2 to 5% (9:1 methanol/ .880 ammonia)/dichloromethane to afford the title compound (1.7g, 37%) as an orange gum.
δ_{H} (CDC1₃) 8.60 (d,*J* 6 Hz, 1H), 8.00 (d, *J* 6 Hz, 1 H), 7.50 (br s, 1H), 7.50-7.31 (m ,2H), 7.15 (d, *J* 6 Hz, 1H), 3.00-2.43 (m, 7H), 1.90-0.95 (m, 21H), 0.95-0.74 (m, 6H)
MS (+ve ion electrospray) m/z 397 (MH+).

### Example 23. [3R,4R]-3-Ethyl-1-heptyl-4-[3-(6-propyloxyquinolin-4-yl)propyl]piperidine.

The product from Example 22b (0.17g, 0.43mmol) was alkylated with 1-bromopropane(0.044ml, 0.47mmol) by the method of Reference Example 16. Purification by column chromatography eluting with 1 to 2% (9:1 methanol/ .880 ammonia)/dichloromethane afforded the title compound (0.06, 32%) as an orange gum.
δ_{H} (CDCl₃) 8.65 (d, *J* 6 Hz, 1H), 8.00(d, *J* 10 Hz, 1H), 7.45-7.10 (m, 3H), 4.05(t, *J* 7 Hz, 2H), 3.14-2.86(m, 2H), 2.70-0.72 (m, 37H)
MS (+ve ion electrospray) m/z 439 (MH+)

### Example 25. [3R,4R]-3-Ethyl-1-heptyl-4-[3-(6-(5-aminopentyloxy)-quinolin-4-yl)propyl]piperidine.

The product from Example 22b (0.10g, 0.16 mmol) in methanol (2ml) was treated with methyl hydrazine as described in Example 17. Purification by column chromatography eluting with 2 to 5% (9:1 methanol/ .880 ammonia)/dichloromethane to afforded the title compound (0.11 g, 47%) as a colourless oil.
δ_{H} (CDCl₃) 8.68 (d, *J* 5 Hz, 1H), 8.01 (d, *J* 8 Hz, 1H), 7.42-7.15 (m, 3H), 4.10 (t, *J* 7 Hz, 2H), 3.09-2.86 (m, 2H), 2.83-2.67 (m, 2H), 2.64-1.14 (m, 34H), 0.90 (t, *J* 7 Hz, 6H)
MS (+ve ion electrospray) m/Z 482 (MH+)

### Reference Example 26. [3R,4R]-3-Ethenyl-1-(2-t-butyloxycarbonylaminoethyl)-4-[3-(6-methoxyquinolin-4-yl)propyl]piperidine

### (a) [3R,4R]-3-Ethenyl-4-[3-(6-methoxyquinolin-4-yl)propyl]piperidine

Example 13a (50.0g, 154mmol) was reduced as described in Example 12a The product was purified by chromatography on silica gel eluting with 90:9:1 chloroform/ethanol/35% ammonia solution to give the title compound (38.75g, 80%).
δ_{H} (CDCl₃) *inter alia* 8.68 (1H, d, *J* 4.5Hz), 8.02 (1H, d, *J* 9.3Hz), 7.39 (1H, dd, *J* 9.3, 2.7Hz), 7.22 (1H, d, *J* 2.7Hz), 7.19 (1H, d, *J* 4.5Hz), 6.10 (1H, m), 5.06 (2H, m), 3.96 (3H, s). MS (+ve ion electrospray) m/z 311 (MH⁺).

### (b) [3R.4R] -3-Ethenyl-1-(2-t-butyloxycarbonylaminoacetyl)-4-[3-(6-methoxyquinolin-4-yl)propyl]piperidine

*N*-tert-Butyloxycarbonylglycine (0.14g, 0.008mole) in dry dichloromethane (15ml) was cooled to 0°C and treated with *N*-methylmorpholine (0.096g, 0.00096mole), 1-hydroxybenzotriazole hydrochloride (0.258g, 0.0019mole) and 1-(3-dimethylaminopropyl)-3-ethylcarbodiimide hydrochloride (0.32g, 0.0019mole) and the mixture stirred at 0°C for 30 minutes. The product from Reference Example 26a (0.5g, 0.0016mole) was added and stirred at room temperature for 1 hour. The mixture was washed with saturated aqueous sodium bicarbonate solution, 2M hydrochloric acid and saturated brine solution, dried (magnesium sulphate) and evaporated *in vacuo* . The residue was purified by column chromatography eluting with 5% methanol/dichloromethane to afford the title compound (0.62g, 83%) as a yellow solid. δ_{H} (CDCl₃) *inter alia* 8.82-8.40 (m, 2H), 7.66-7.20 (m, 4H), 5.89-5.45 (m, 2H), 5.22-5.00 (m, 2H), 4.00 (s, 3H), 1.42 (s, 9H)
MS (+ve ion electrospray) m/z 468 (MH+)

### (c) Title compound.

The product from Reference Example 26b (0.61g, 0.0013mole) in dry tetrahydrofuran (10ml) was added dropwise to a suspension of lithium aluminium hydride (0.20g, 0.0052mole) and stirred at room temperature for 16 hours. The mixture was cooled to 0°C and treated dropwise with water (0.2ml), 2m sodium hydroxide solution (0.3ml) and water (0.5ml). The mixture was filtered through celite and the filtrate evaporated *in vacuo.* The residue was purified by column chromatography eluting with 2 to 5% (9:1 methanol/.880 ammonia)/dichloromethane to afford the title compound (0.42g, 71%) as an orange oil.
δ_{H} (CDCl₃) 8.66 (d, *J* 7 Hz, 1H), 8.01 (d, *J* 10 Hz, 1H), 7.36 (dd, *J* 3 and 12 Hz, 1H), 7.12 (m, 2H), 6.20-6.01 (m, 1H), 5.10-4.87 (m, 3H), 3.95 (s, 3H), 3.78-3.65 (m, 1H), 3.33-3.11 (m, 3H), 2.97 (t, *J* 7 Hz, 2H), 2.88-2.68 (m, 2H), 2.41-2.25 (m, 3H), 2.18-1.94 (m, 2H), 1.94-2.10 (m, 14H)
MS (+ve ion electrospray) m/z 354 (MH+)

### Example 27. [3R,4R]-3-Ethenyl-1-(2-phenoxyethyl)4-[3-(6-methoxyquinolin-4-yl)propyl]piperidine

### (a) [3R,4R]-3-Ethenyl-1-(2-phenoxyacetyl)-4-[3-(6-methoxyquinolin-4-yl)propyl]piperidine.

The product from Reference Example 26a (0.30g, 0.0096mole) in dry dichloromethane (20ml) was treated with triethylamine (0.11g, 0.001mole) and the mixture cooled to 0°C. Phenoxyacetyl chloride (0.18g, 0.001 mole) was added and the mixture stirred at room temperature for 2 hours. The mixture was washed with water, dried (magnesium sulphate) and evaporated *in vacuo* to afford the title compound (0.37g, 86%) as a yellow gum.
δ_{H} (CDCl₃) *inter alia* 8.67 (d, *J* 7 Hz, 1H), 8.03 (d, *J* 10Hz, 1H), 7.40-7.12 (m, 6H), 7.05-6.84 (m, 2H), 5.91-5.48 (m, 1H), 5.19-4.92 (m, 2H), 4.27-4.50 (m, 2H), 3.95 (s, 3H)
MS (+ve ion electrospray) m/z 445 (MH+)

### (b) [3R,4R]-3-Ethenyl-1-(2-phenoxyethyl)-4-[3-(6-methoxyquinolin-4-yl)propyl]piperidine

The title compound was prepared from the product from Example 27a using the method of Reference Example 26c.
^{δ}H (CDCl₃) 8.66 (d, *J* 7 Hz, 1H), 8.01 (d, *J* 10 Hz, 1H), 7.41-7.13 (m, 5H), 6.98-6.82 (m, 3H), 6.20-6.01 (m, 1H), 5.08-4.97 (m, 2H), 4.11-4.00 (m, 2H), 3.95 (s, 3H), 3.04-2.62 (m, 6H), 2.40-2.15 (m, 3H), 1.87-1.25 (m, 7H)
MS (+ve ion electrospray) m/z 431 (MH+)

### Example 29. [3S,4R]-3-Ethenyl-1-heptyl-4-[3-(6-methoxyquinolin-4-yl)propyl]piperidine

Reference Example 26a (1.04 g) in water (5 ml) was treated with 5M hydrochloric acid to pH 3.5 followed by a 37% solution of formaldehyde (0.10 g) and the solution was heated under reflux, under argon, for 17 hours. The reaction mixture was evaporated to dryness, dissolved in water and made basic with an excess of 2M sodium hydroxide and extracted with dichloromethane (3 x 20 ml). The organic fraction was washed with water, dried over anhydrous sodium sulphate and evaporated to afford an oil. The oil was dissolved in dry dimethylformamide (5 ml) and anhydrous potassium carbonate (1.84 g) was added, followed by n-heptyl iodide (0.90 g) and the mixture was stirred at room temperature under argon for 17 hours. The reaction mixture was evaporated to dryness, brine was added, and the solution was extracted with dichloromethane (3 x 20 ml). The organic fraction was washed with brine, dried over sodium sulphate, and evaporated to dryness to afford an oil. The crude product was chromatographed on a SepPak 10g silica cartridge eluting with ethyl acetate:hexane (1:1) to afford the title compound as a pale brown oil (0.095 g).
δ_{H} (CDCl₃) 8.65 (d, *J* 4Hz 1H), 8.0 (d, *J* 8Hz, 1H), 7.34 (dd, *J* 8,2 Hz 1H), 7.21 (d, *J* 2Hz, 1H), 7.17 (d, *J* 4Hz), 5.54 (m, 1H), 5.02 (m, 2H), 3.94 (s, 3H), 2.94 (m, 4H), 2.80 (br d, *J* 10 Hz, 2H), 2.27 (m, 2H), 2.02 (m, 2H), 1.10-1.90 (m, 16H), 0.87 (t, *J* 7 Hz, 3H); Found: EI MH⁺ 409. C₂₇H₄₀N₂O requires MH, 409.

### Example 30 [3R,4R]-3-Ethenyl-1-heptyl-4-[3-(6-methoxyquinoline)-4-yl)propyl]piperidine.

Prepared from Reference Example 26a by method of Example 3 in 66% yield.
δ ¹H (CDCl₃) 8.57 (d, 1H), 7.95 (d, 1H), 7.09-7.31 (m, 3H), 6.04 (m, 1H), 4.94 (m, 2H)_{,} 3.87 (s, 3H), 2.90 (t, 2H), 2.70 (m, 2H), 1.19-2.27 (m, 22H), 0.80 (m, 3H).
EI M⁺ , 409. C₂₇H₄₀N₂O requires M, 409.

### Example 31. [3R,4R]-1-Heptyl-3-(2-hydroxyethyl)-4-[3-(6-methoxyquinolin-4-yl)propyl]piperidine

Prepared from Example 30 (9.95g) using method of Example 15 to give the title compound (1.9g) (22.5%) as an oil.
δ_{H} (CDCl₃) 8.65 (d, 1H), 8.04 (d, 1H), 7.38 (d, 1H), 7.20 (m, 2H), 3.95 (s, 1H).
EI MH⁺, 427. C₂₇H₄₂N₂O₂ requires MH, 427.

### Example 32. [3R,4R]-1-Heptyl-3-(2-acetoxyethyl)-4-[3-(6-methoxyquinolin-4-yl)propyl]piperidine

Example 31 (0.100g) was dissolved in dry pyridine (5 ml) and stirred at room temperature while acetic anhydride (132 ul) and 4-dimethylaminopyridine (catalytic amount) were added. The reaction mixture was stirred at room temperature for 60 hours. The mixture was diluted with ethyl acetate (50 ml), washed with water (3 x 20 ml), dried over anhydrous magnesium sulphate, filtered and evaporated. This gave the title compound (0.083g, 75%) which did not require purification.
δ_{H} (CDCl₃) 1.98 (s,3H)
EI MH⁺, 469. C₂₉H₄₄N₂O₃ requires MH, 469.

### Example 33. [3R,4R]-1-Heptyl-3-(3-hydroxypropyl)-4-[3-(6-methoxyquinolin-4-yl)propyl]piperidine.

### (a) [3R,4R]-1-Benzyloxycarbonyl-3-ethenyl-4-[3-(6-methoxyquinolin-4-yl)propyl]piperidine.

Example (26a) (6.34g) was dissolved in ethyl acetate (70 ml) and saturated aqueous sodium bicarbonate solution (70 ml) was added. Benzyl chloroformate (3.2 ml) was added dropwise to the vigorously stirred reaction mixture while maintaining the pH at 9 by the addition of solid sodium carbonate. Vigorous stirring was continued for 14 hours overnight. Ethyl acetate (20 ml) and water (20 ml) were added and the organic layer separated. The aqueous layer was further re-extracted with ethyl acetate (100 ml), the combined organic layers dried over anhydrous magnesium sulphate, filtered and evaporated. Column chromatography on silica-gel gave the title compound (8.34g, 92%) as an oil.
δ_{H} (CDCl₃) 8.65 (d, 1H), 8.03 (d, 1H), 7.15-7.38 (m, 8H), 5.78 (m, 1H), 5.12 (m, 4H). EI MH⁺ ,445. C₂₈H₃₂N₂O₃ requires MH, 445.

### (b) [3R,4R]-1-Benzyloxycarbonyl-3-(2-hydroxyethyl)-4-[3-(6-methoxyquinolin-4-yl)propyl]piperidine.

The olefin 33a (4.89g, 0.011 moles) was hydroborated as described in Example 15. Column chromatography on silica gel gave a brown coloured oil (3.5g, 69%).
E.I. MH⁺, 463. C₂₈H₃₄N₂O₄ requires MH, 463.
δ_{H} (CDCl₃) 8.00 (1H, d), 7.25-4 (8H, m), 5.15 (2H, q).

### (c) [3R,4R]-1-Benzyloxycarbonyl-3-(2-oxoethyl)-4-[3-(6-methoxyquinolin-4-yl)propyl]piperidine.

The alcohol (33b) (1.5g, 3.24 mmol) was dissolved in dry dichloromethane (40 ml) and size 4A molecular sieves were added. Tetrapropylammonium perruthanate (0.227g, 0.65 mmol) and 4-methyl-mopholine-N-oxide (6.48 mmol) were added to the reaction mixture. This was stirred at room temperature for 1 hour. The reaction mixture was filtered through silica, washing with ethylacetate (50 ml). The organic washings were evaporated. Column chromatography on silica-gel gave a colourless oil (0.56g, 34%).
E.I. MH⁺, 461. C₂₈H₃N₂O₄ requires MH, 461.
δ_{H} (CDCl₃) 9.65 (1H, m), 8.65 (1H, d), 8.00 (1H, d), 7.1-4 (8H, m), 5.15 (2H, m).

### (d) [3R,4R]-1-Benzyloxycarbonyl-3-(prop-2-enyl)-4-[3-(6-methoxyquinolin-4-yl)propyl]piperidine.

Methyltriphenylphosphonium bromide (1.36g, 3.82 mmol) was dissolved in dry tetrahydrofuran (10ml). A solution of potassium tert-butoxide in tetrahydrofuran (1M; 3.3 ml, 3.3 mmol) was added under argon. After 0.25 hours Example 33c (116) (0.5g, 1.09 mmol) in dry THF (10 ml) was added. The reaction mixture was allowed to stir at room temperature for 2 hours. Acetone (10 ml) and ethylacetate (30 ml) were added. The reaction mixture was centrifuged, the supernatent was decanted and the solvent evaporated. Column chromatography on silica-gel gave the olefin as an oil (0.4g, 82%). E.I. MH⁺ , 459. C₂₉H₃₄N₂O₃ requires MH, 459.
δ_{H} (CDCl₃) 8.65 (1H, d), 8.0 (1H, d), 5.75 (1H, s), 5.15 (2H, s), 5 (1H, m)

### (e) [3R,4R]-1-Benzyloxycarbonyl-3-(3-hydroxypropyl)-4-[3-(6-methoxyquinolin-4-yl)propyl]piperidine.

Example 33d (0.31g, 0.676 mmol) was hydroborated as in Example 15. Column chromatography on silica-gel gave the alcohol (0.11g, 34%).
E.I. MH⁺, 477. C₂₉H₃₆N₂O₄ requires MH, 477.
δ_{H} (CDCl₃) 8.65 (1H, d), 8.0 (1H, d), 7.4-2 (8H, m), 5. 15 (2H, m), 3.95 (3H, s).

### (f) [3R,4R]-3-(3-Hydroxypropyl)-4-[3-(6-methoxyquinolin-4-yl)propyl]piperidine.

Example 33e (0.11, 0.23 mmol) was hydrogenated under the same conditions as for Example 34b affording the amine as a brown oil (0.071 g, 90%).
E.I. MH⁺ ,343. C₂₁H₃₀N₂O₂ requires MH, 343.

### (g) Title compound.

The secondary amine 33f (0.1g, 0.3 mmol) was alkylated as in Example 3. Column chromtography on silica-gel gave the title compound as a clear oil (0.029g, 21%).
E.I. MH+ ,441. C₂₈H₄₄N₂O₂ requires MH, 441.
δ_{H} (CDCl₃) 8.65 (1H, d), 7.95 (1H, d), 7.25 (1H, dd), 7.15 (2H, m), 3.9 (3H, s).

### Example 34, [3R,4R]-1-Heptyl-3-(1-hydroxyethyl)-4-[3-(6-methoxyquinolin-4-yl)propyl]piperidine.

### (a) [3R,4R]-1-Benzyloxycarbonyl-3-(1-hydroxyethyl)-4-[3-(6-methoxyquinolin-4-yl )propyl]piperidine.

Example 33a (5.72g) was dissolved in THF (30 ml) and mercuric acetate (16.44g) dissolved in THF/water (100 ml/130 ml) added to the solution. The reaction mixture was heated at reflux for 16.5 hours and allowed to cool to room temperature. 2M Sodium hydroxide solution (64 ml) and sodium borohydride (2.00g) dissolved in 2M sodium hydroxide solution (64 ml) were then added to give a grey suspension. After stirring for 3 hours, the mixture was filtered through a plug of celite and washed well with diethyl ether and water. Saturated sodium bicarbonate solution was added and the mixture extracted with diethyl ether and water. Saturated sodium bicarbonate solution was added and the mixture extracted with diethyl ether (x2). The combined organic layers were dried over anhydrous magnesium sulphate, filtered and evaporated. Column chromatography on silica gel gave the title compound (2.32g) (39%) as a white solid.
δ_{H} (CDCl₃) 8.65 (d, 1H), 8.05 (d, 1H), 7.34 (m, 8H), 5.13 (s, 2H), 3.94 (s, 3H), 3.75 (m, 1H), 3.65 (m, 2H), 3.28-2.95 (m, 4H), 2.10 (bs, 1H), 1.20-1.95 (m, H)
EI MH⁺ 463. C₂₈H₃₄N₂O₄ requires MH, 463.

### (b) [3R,4R]-3-(1-Hydroxyethyl)-4-[3-(6-methoxyquinolin-4-yl)propyl]piperidine.

Example 34a (0.700g) was dissolved in ethanol (40 ml), 10% Pd/C (0.400g) added and the reaction mixture hydrogenated at atmospheric pressure for two hours. The mixture was filtered through a small plug of celite and the solvent evaporated to give 0.483g (97%) of the title compound. This was taken onto the next step without purification.
δ_{H} (CDCl₃) 8.65 (D, 1H), 8.03 (d, 1H), 7.38 (d, 1H), 7.23 (m, 2H), 3.94 (s, 1H).
EI MH⁺ 329.1. C₂₀H₂₈N₂O₂ requires MH, 329.

### (c) [3R,4R]-1-Heptyl-3-(1-hydroxyethyl)-4-[3-(6-methoxyquinolin-4-yl)propyl]piperidine.

Example 34b (0.463g) was converted to the title compound by the method of Example 3. Column chromatography on silica gel gave the title compound (0.335g) (56%) as a yellow oil.
δ_{H} (CDCl₃) 8.65 (d, 1H), 8.03 (d, 1H) 7.36 (d, 1H), 7.22 (m, 2H), 3.94 (s, 3H), 3.04-1.27 (m, 3H), 0.88 (m, 3H)
EI MH⁺ 427. C₂₇H₄₂N₂O₂ requires MH. 427.

### Example 35 [3R,4R]-3-Ethyl-1-(2-phenylethyl)-4-[3-(R,S).hydroxy-3-(6-methoxyquinolin-4-yl)propyl]piperidine.

Example 11a ( 500 mg. 1.52 mmol) in methanol (30 ml) was stirred with phenyl acetaldehyde (273 mg, 2.28 mmol) and A4 molecular sieves for 30 min, then sodium cyanoborohydride (141 mg, 2.28 mmol) was added and the mixture was stirred overnight. Water (30 ml) was then added and the mixture was extracted with EtOAc (3 x 30 ml). The combined extracts were washed with brine (60 ml), dried (sodium sulphate) and concentrated *in vacuo.* The residual oil was purified hy column chromatography (CH2Cl2:MeOH 2-5%) to give the title compound as an oil (610 mg).
δ_{H} (CDCl3) 8.70 (d, 1H, J=3.3Hz); 8.01 (d, 1H, J=6.6Hz); 7.49 (t. 1 H, J= 1.65Hz); 7.34 (dd, 1H. J=6.6, 1.65Hz); 7.30-7.15 (m, 6H), 5.31 (m, 1H); 3.90 (s, 3H); 2.81-1.23 (m, 1814); 0.88 (m. 3H)
MS: m/Z 433(MH)⁺

### Example 36. [3R,4R].3-Ethyl-1-(-3-phenylpropyl)-4-[3-(R,S)-hydroxy-3-(6-methoxyquinolin-4-yl)propyl]piperidine.

In a manner similar to Example 35 Example 11a ( 328 mg, 1 mmol) and phenyl propionaldehyde (210 mg, 1.50 mmol) gave the title compound as an oil (388 mg)
δH (CDCl₃): 8.72 (d, 1H, J=3.3); 8.01 (d, 1H, J=6.6Hz); 7.48 (t, 1H, J=3.3); 7.34 (dd, 1H, J=6.6,1.65); 7.29-7.14 (m, 6H); 5.32 (m, 1H); 3.92 (s, 3H); 2.70-1.20 (m, 20H); 0.88 (m, 3H)
MS: m/z 447(MH)⁺

### Example 37. 1-Heptyl-4-[2-(R,S)-hydroxy-3-(6-methoxyquinolin-4-yl)propyl]piperidine.

### (a) [3R,4R]-1-Benyloxycarbonyl-4-[2-oxo-3-(6-methoxyquinolin-4-yl)propyl]piperidine.

To a solution of lithium diisopropylamide (5.12 ml, 7.68 mmol) in THF (15 ml) at -78°C was added dropwise with stirring a solution of 6-methoxy-4-methylquinoline (1.33 g, 7.68 mmol) in THF (15 ml). The mix. was stirred at -78°c for 20 min. and then a solution of N-(benzyloxycarbony])-4-(ethoxyacetyl)-piperidine (1.17 g, 3.84 mmol) in THF (10 ml) was added dropwise. Stirring was continued for 20 min. and allowed to warm to room temp. for 1 h. Water added and the aq. layer was neutralised with acetic acid and extracted with EtOAc (X 3). the combined organic layers were washed with brine, dried and concentrated in *vacuo* to give the title compound as an oil. This was purified by column chromatography (Hexane:EtOAc) to give the product as an oil (1.05 g)
MS. : m/z 433 (M+H)⁺

### (b) 1-Benzyloxycarbonyl-4-[2-(R,S)-hydroxy-3-(6-methoxyquinolin-4-yl)propyl]piperidine.

To a solution of Example 37a (114 mg. 0.26 mmol) in MeOH (10 ml) was added excess sodium cyanoborohydride (30 mg) and the reaction mix. was stirred for 2h. Water added and extracted with EtOAc (x3). The combined extracts were washed and concentrated to give the title compound as an oil (112 mg).
MS: m/z 435(MH)+

### (c) 4-[2-(R,S)-hydroxy-3-(6-methoxyquinolin-4-yl)propyl]piperidine.

Using the method of Example 34b, Example 37b (550 mg) was hydrogenated to give the title compound as a crystalline product (386 mg) after filtration and concentration in *vacuo.*
MS: m/z 300(MH)+

### (d) Title compound.

Example 37c was alkylated as described in Example 22b to give the title compound as an oil (224 mg).
δH: (CDCl3) 8.62 (d, 1H, J=3.3); 8.00 (d, 1H, J=6.6Hz); 7.36 (dd, 1H, J=6.6,1.65); 7.25 (d, 1H, J=1.65); 7.23 (d, 1H, J=3.3); 4.16 (m, 1H); 3.94 (s, 3H); 3.22 (dd, 1H, J=8.26,1.65); 3.05 (dd, 1H, J=8.26,6.6Hz): 2.96 (m, 2H); 2.30 (m, 2H); 1.90-1.189 (m, 19H); 0.87 (t, 3H, J=4.95Hz)
MS: m/z 399(MH)⁺

### Example 38 1-Heptyl-4-[3-(6-methoxyquinolin-4-yl)prop-2-enyl]piperidine.

Example 37 (200 mg, 0.50 mmol ) in acetic anhydride (5 ml) was refluxed for 4h. Acetic anhydride was evaporated in *vacuo* and the residue partitioned between saturated potassium carbonate and EtOAc (20 ml). The organic layer was separated, dried and concentrated in vacuo. Purification by column chromatography (CH₂Cl₂:MeOH:NH₃ ; 9:1:0.1) gave the product as an oil (100 mg)
MS: m/z 381 (MH)

### Example 39 1-Heptyl-4-[3-(6-methoxyquinolin-4-yl)propyl]piperidine.

Example 38 (70 mg, 0.18 mmol) in MeOH (50 ml) was reduced with hydrogen in the presence of 10% Pd/C (30 mg) at 50 psi overnight. Concentration of the filtered solution in *vacuo* and purification by column chromatography (CH₂Cl₂:MeOH:NH₃ ; 9:1:0.2) gave the title compound as a crystalline solid (54mg).
(CDCl3) δH: 8.60 (d, 1H, J=3.3Hz ); 8.00 (d, 1H. J=6.6Hz ); 7.35 (dd, 1H, J=6.6,1.65Hz ); 7.22 (d, 1H, J= 1.65Hz); 7.18 (d. 1H. J= 3.3Hz ); 3.95 (s, 3H); 3.01 (m, 4H); 2.41 (m, 2H); 2.03 (m, 2H); 1.85-1.38 (m.10H); 1.28 (bs, 9H); 0.88 (t, 3H, J=4.95Hz )
MS: m/Z 383 (MH)⁺

### Example 40. [3R,4R]-3-Ethyl-1-heptyl-4-[3-(R.S)-hydroxy-3-(6-methoxyquinolin-4-yl)butyl]piperidine

The product from Example 3 (0.49g) was dissolved in dry THF (10ml) and cooled to -78°C under Ar. Methylmagnesium bromide solution (3M in diethyl ether, 0.5ml) was added, and the solution was allowed to warm to ambient temperature over 4h. Water (50ml) was then added, and the mixture was extracted with ethyl acetate (3 x 30ml). The organic layers were washed with brine, dried over anhydrous sodium sulphate and evaporated to give an oil. The product was chromatographed on silica gel, eluting with 0-20% methanol in dichloromethane (gradient), giving first the recovered starting material (0.33g), and then the title compound (0.053g).
δ_{H} (CDCI₃) 8.62 (d), 8.03 (d), 7.99 (d), 7.36 (m), 3.92 (s), 2.9-1.9 (br m), 1.81 (s), 1.6-1.0 (br m), 0.89 (t), 0.78 (t), 0.73 (t);
mass spectrum EI MH⁺ 441. C₂₈H₄₄N₂O₂ requires MH, 441.

### Example 41 [3R,4R]-3-Ethenyl-1-heptyl-4-[3-(R,S)-azido-3-(6-methoxyquinolin-4-yl)propyl]piperidine.

Example 13c (4.8g) was dissolved in dry toluene (15 ml) and diphenylphosphorylazide (2.9 ml) and 1,8-diazabicyclo[5.4.0]undec-7-ene (2.0 ml) were added. The reaction mixture was stirred at room temperature for 21 hours. Further diphenylphosphorylazide (2.9 ml) and 1,8-diazabicyclo[5.4.0]undec-7-ene (2.0 ml) were added and the reaction mixture heated at 50°C for 2 hours 40 minutes. The mixture was allowed to cool to room temperature and saturated sodium bicarbonate solution (50 ml) added. The mixture was extracted with ethyl acetate (3x50 ml) and the combined organic extracts dried over anhydrous magnesium sulphate, filtered and evaporated. Column chromatography on silica-gel gave the title compound (4.3g, 85%) as a dark yellow oil. δH (CDCl₃) 8.75 (d, 1H), 8.05 (d, 1H), 7.21-7.40 (m, 3H), 6.10 (m, 1H), 5.00 (m, 3H), 3.96 (s, 3H), 2.76 (t, 2H).
EI MH⁺ 450. MH of C₂₇H₃₉N₅O requires 450.

### Example 42. [3R,4R]-3-Ethenyl-1-heptyl-4-[3-(R,S)-amino-3-(6-methoxyquinoolin-4-yl)propyl]piperidine

Example 41 (4.3g) was dissolved in ethanol (100 ml) and 10% Pd/C (1.5g) added. The reaction mixture was hydrogenated at atmospheric pressure for one hour. Approximately 2.3 of the reaction was removed, filtered through a plug of celite and the solvent evaporated. Column chromatography on silica gel gave compound B (1.2g) as a yellow oil.
δH (CDCl₃) 8.75 (d, 1H), 8.05 (d, 1H), 7.48 (m, 1H), 7.39 (m, 1H), 7.30 (m, 1H), 6.08 (m, 1H), 4.98 (m, 2H), 4.61 (t, 1H), 3.94 (s, 3H). 2.75 (m, 2H).
El MH⁺ 424. MH of C₂₇H₄₁ N₃O requires 424.

### Example 43. [3R,4R]-3-Ethyl-1-heptyl-4-[3-(R,S)-amino-3-(6-methoxyquinolin-4-yl)propyl]piperidine

Example 42 was hydrogenated at atmospheric pressure for six hours. The reaction mixture was filtered through a plug of celite and the solvent evaporated. Following a column chromatography on silica gel, title compound was obtained as a yellow oil.
δH (CDCl₃) 8.75 (d, 1H), 8.05 (d, 1H), 7.30-7.49 (m, 3H), 4.63 (m, 1H), 3.93 (s, 3H).
EI MH⁺ 426. MH of C₂₇H₄₃N₃O requires 426.

### Example 44 [3R,4R]-3-Ethyl-1-heptyl-4-[3-(6-methoxyquinolin-4-yl)butyl]piperidine

### (a) [3R,4R]-1-Benzyloxycarbonyl-3-ethenyl-4-[3-oxo-3-(6-methoxyquinolin-4-yl)propyl]piperidine.

Example 13a was protected by the method of Example 33a to give the title compound.

### (b) [3R,4R]-1-Benzyloxycarbonyl-3-ethenyl-4-[3-(6-methoxyquinolin-4-yl)but-3-en]piperidine.

Example 44a was converted to the title compound by the general method of Example 33d. Chromatography eluting with 5-70% ethyl acetate in petroleum ether afforded the title compound as an oil (2.7g, 72%).
δH (CDCl₃) 8.70 (1H, d), 8.05 (1H. d), 7.25-7.40 (7H, m), 7.15 (1H, d), 5.65-5.80 (1H. m), 5.45 (1H, d), 5.00-5.20 (5H. m), 3.90 (3H, s).
E.I. MH⁺, 457. C₂₉H₃₂N₂O₃ requires MH, 457.

### (c) [3R,4R]-3-Ethyl-4-[3-(6-methoxyquinolin-4-yl)butyl]piperidine

Example 44b (0.3g, 0.66 mmol) in tetrahydrofuran (20 ml) was treated with a slurry of 10% palladium on charcoal (0.1g) in water (5 ml) and hydrogenated at atmospheric pressure for 2h. Filtration and evaporated afforded the title compound as a brown oil (0.22g, 100%).
E.I. MH⁺, 327. C₂₁H₃₀N₂O requires MH, 327

### (d) [3R,4R]-3-Ethyl-1-heptyl-4[3-(6-methoxyquinolin-4-yl)butyl]piperidine

Example 44c was alkylated as described in Example 3. Chromatography, eluting with 1:1 dichloromethane: ethyl acetate afforded the title compound as a clear oil (60 mg. 21%), as a single diastereomer at the benzylic position.
δ (CDCl₃) 8.70 (1H, d), 8.05 (1H, d), 7.35 (1H. dd), 7.30 (1H. d), 7.25 (1H. d), 1.35 (3H, d).
E.I. MH⁺, 425. C₂₈H₄₄N₂O requires MH, 425

### Example 45 [3R,4R]-3-Ethenyl-1-heptyl-4-(R,S)-acetamido-3-(6-methoxyquinolin-4-yl)propyl]piperidine

Acetic anhydride (0.1168g) dissolved in ethyl acetate (1ml) was added dropwise to a vigorously stirred solution of Example 42 (0.440g) in ethylacetate (10ml) and saturated sodium carbonate solution (10ml) at 0°C. The reaction mixture was allowed to warm to room temperature and stirred for 1.5 hours. Additional acetic anhydride (3 drops) in ethyl acetate (1ml) was added dropwise to the reaction mixture and allowed to stir at room temperature overnight. Saturated sodium carbonate solution (20ml) was added and extracted with ethyl acetate (20ml). The organic layer was dried over anhydrous magnesium sulphate, filtered and the solvent evaporated.

Column chromatography on silica gel gave the title compound as an oil (0.390g, 81%)
EI M⁺, 466. C₂₉H₄₃N₃O₂ requires MH, 466.
δH (CDCl₃) 6.10 (m, 1H) 5.74 (m, 2H) 5.04 (m, 2H) 2.00 (s, 3H)

### Example 46 [3R,4R]-1-Heptyl-3-(2-(R,S)-hydroxypropyl)-4-[3-(6-methoxyquinolin-4-yl)propyl]piperidine

### (a) [3R,4R]-1-Benzyloxycarbonyl-3-(2-(R,S)-Hydroxypropyl)-4-[3-(6-methoxyquinolin-4-yl)propyl]piperidine.

Example 33c was alkylated asdescribed in Example 40 giving the title compound in 13% yield.
E.I. MH⁺, 477. C₂₉H₃₆N₂O₄ requires MH, 477.

### (b) [3R,4R]-3-(2-(R,S)-Hydroxypropyl)-4-[3-6-[3-(6-methoxyquinolin-4-yl)propyl]piperidine.

Example 46a was hydrogenated as described for Example 34b to give the title compound in 90% yield.
E.I. MH⁺ ,343. C₂₁H₃₀N₂O₂ requires MH, 343.

### (c) Title compound

Example 46b was alkylated as described for Example 3 to give the title compound in 40% yield.
E.I. MH⁺ ,441. C₂₈H₄₄N₂O₂ requires MH, 441.
δH (CDCl₃) 8.80(d, 1H), 7.95(d, 1H), 7.30 (d, 1H), 7.14 (m, 2H), 4.1 (m, 1H), 3.9(s, 3H). The individual distereomers were separated using HPLC and a Chiralpak AD column.

### Example 47 [3R,4R]-1-Heptyl-3-(1-(R,S),2-dihydroxyethyl)-4-[3-(6-methoxyquinolin-4-yl)propyl]piperidine.

N-methylmorpholine oxide (126 mg) was dissolved in water (5 ml). Acetone (2 ml) and osmium tetroxide (6 mg) in t-butanol (2 ml) added. Example 30 (400 mg) in acetone (3 ml) was added and the reaction mixture stirred at room temperature for 19 hours.

Solid sodium sulfite was added. The mixture was stirred for 30 minutes and then filtered through a plug of celite. The solid was washed well with ethyl acetate and the filtrate dried over anhydrous magnesium sulphate, filtered and evaporated. Column chromatography on silica gel gave the target compound (48 mg) (11%) as a mixture of diastereoisomers (2:1 ratio) as an oil.
δH (CDCl₃) 8.55 (d, 1H), 7.95 (d, IH), 7.30 (d, 1H), 7.14 (m, 2H), 3.88 (m), 3.72 (m), 3.62 (m), 3.50 (m), 3.39 (m), 2.90-3.11 (m, 4H).
EI M⁺ 443. C₂₇H₄₂N₂O₃ requires MH, 443.

### Example 48. [3R,4R]-1-Heptyl-3-aminocarbonyloxyethyl-4-[3-(6-methoxyquinoIin-4-yl)propyl]piperidine.

Example 31 (1g, 2.35 mmol) was dissolved in dichloromethane (5 ml). Trichloroacetyl isocyanate (0.5g, 2.5 mmoles) in dichloromethane (1 ml) was added dropwise over 30 minutes. This was stirred at 0°C for 2 hours. Potassium carbonate (0.36g, 2.65 mmoles) in methanol (3 ml) and water (1 ml) was added and the reaction mixture was allowed to stir for an hour at 0°C. The reaction was left to stir at room temperature overnight. The mixture was partitioned between dichloromethane and dilute aqueous sodium bicarbonate and the phases repeated. The organic phase was collected, dried over sodium sulphate and evaporated. Column chromatography on silica gel gave the titlecompound as an oil (0.54g, 49%).
E.I. MH⁺, 470. C₂₈H₄₃N₃O₃ requires MH, 470.
δH (CDCl₃) 8.7 (1H, d), 8.05 (1H, d), 7.35 (1H, dd), 7.25 (2H, m).

### Example 50. [3R,4R]-3-(1-(R,S),2-Dihydroxyethyl)-1-heptyl-4-[3-(R,S)-hydroxy-3-(6-methoxyquinolin-4-yl)propyl]piperidine

Prepared as in Example 47 from 0.200g of Example 13c. Column chromatography on silica gel allowed the two pairs of diasteroisomers to be separated by preparative hplc, giving 27mg (25%) of two pairs of diastereomers.
δH (CDCl₃) 8.60 (m, 1H), 7.90 (d, 1H), 7.46 (m, 1H), 7.29 (m, 1H), 7.16 (m, 1H)_{,} 8.63 (s, 1H), 7.95 (d, 1H), 7.48 (m, 1H), 7.30 (m, 1H), 7.16 (d, 1H).
El MH⁺ 459. C₂₇H₄₂N₂O₄ requires MH, 459.

### Example 51 [3R, 4R]-3-Ethyl-1-heptyl-4-[(6-methoxyquinolinyl-4-oxy)methyl]piperidine.

### (a) 6-Methoxyquinoline-4-carboxylic acid and [3R,4S]-3-Ethenyl-4-piperidineacetic acid

The title compounds were prepared by modification of the procedure described by W.E. Daering and J.D. Chanley, *J. Amer. Chem Soc.,* 1946, **68**, 586 whereby quinone (225g, 0.70 mol) in t-butylalcohol (11) and water (10 ml) was treated with potassium t-butoxide (170g, 1.5 mol). The mixture was stirred at 30C, while air was bubbled through the mixture for 3 days. The mixture was diluted with diethylether and water and the layers separated. The aqueous phase was extracted with ethyl acetate. The combined diethyl ether and ethyl acetate extracts were dried over magnesium sulfate and evaporated to give recovered starting material (97.6g, 43% recovery). The aqueous phase was acidified to pH5 with 5M hydrochloric acid. The precipitate was collected by filtration, washed with water and methanol, then dried to give 6-methoxyquinoline-4-carboxylic acid as a yellow solid (64.6g, 46% yield);
δ_{H} [(CD₃)₂SO, 250 MHz] 6.23-5.95 (1H, m), 5.34-5.06 (2H, m), 3.37-2.92 (5H, m), 2.70 (1H, m), 2.38-2.15 (3H, m), 1.94-1.52 (2H, m): the filtrate contained [3R,4S]-3-Ethenyl-4-piperidineacetic acid.

### (b) [3R,4S]-1-(t-Butyloxycarbonyl)-3-ethenyl-4-piperidine acetic acid

[3R,4S]-3-Ethenyl-4-piperidineacetic acid (96g, 0.57 mol) in dichloromethane (900 ml) and methanol (180 ml) was stirred while triethylamine (150 ml, 1.07 mol) were added. The mixture was stirred for 18h and evaporated. The residue was diluted with ethyl acetate and 2.5 m hydrochloric acid. The layers were separated and the aqueous phase extracted twice more with ethyl acetate. The combined organic extracts were extracted three times with sodium carbonate solution. The aqueous extracts were acidified to pH 2.5 with 5M hydrochloric acid and extracted with ethyl acetate three times. The combined organic extracts were washed with brine, dried over magnesium sulfate and evaporated to give the title compound as a buff solid (81.7g, 43%);
δ_{H} (CDCl₃, 250 MHz) 5.89-5.68 (1H, m), 5.23-5.06 (2H, m), 4.12 (1H, brs), 3.95 (2H, d, *J* 13Hz), 3.04 (1H, dd, *J* 3 and 9 Hz), 2.87 (1H, brs), 2.48-2.05 (4H, m), 1.63-1.35 (2H, m), and 1.45 (9H, s).

### (c) [3R, 4S]-1-(t-Butyloxycarbonyl)-3-ethyl-4-piperidine acetic acid

Example 51b (2.0g, 7.43mmol) in tetrahydrofuran (100ml) was hydrogenated over 10% palladium on carbon (100mg) for 4 hours. The mixture was filtered through celite and the filtrate concentrated *in vacuo* to give the title compound (2.00g, 100%).
δH (CDCl₃) 4.07-3.60 (2H, m), 3.15-2.82 (2H, m), 2.41-2.18 (3H, m), 1.59-1.40 (13H, m), 1.25 (2H, m), 0.98 (3H, t, *J* 7.2Hz). MS (-ve ion electrospray) m/z 270 [M-H]-.

### (d) [3R, 4R]-4-Bromomethyl-1-(t-butyloxycarbonyl)-3-ethylpiperidine

Example 51c (1.35g, 5.0mmol) in ethyl acetate (30ml) at 0°C was treated with 4-methylmorpholine (0.6ml, 5.5mmol) and isobutyl chloroformate (0.71ml, 5.5mmol). After 30 minutes at O°C the resulting suspension was filtered and the filtrate was concentrated. The residue was dissolved in bromotrichloromethane (20ml) and was added dropwise over 5 minutes to a degassed refluxing suspension of 2-mercaptopyridine N-oxide sodium salt (894mg, 6.0mmol) in bromotrichloromethane (20ml). After heating under reflux for 1 hour the mixture was cooled and the solution concentrated. The product was purified by chromatography on silica gel eluting with 10% ethyl acetate in hexane to give the title compound as a colourless solid (960mg, 62%).
δH (CDCl₃) 4.20-3.82 (2H, m), 3.34 (2H. d, *J* 7.8Hz), 2.92-2.69 (2H, m), 2.02 (1H, m)_{,} 1.72 (1H. m), 1.57 (1H, m), 1.52-1.40 (10H, m), 1.20 (2H, m), 0.99 (3H, t, *J* 7.5Hz).

### (e) [3R, 4R]-4-Acetoxymethyl-1-(t-butyloxycarbonyl)-3-ethylpiperidine

Example 51d (730mg. 2.38mmol) in acetonitrile (10ml) was added potassium acetate (2.0g. 20.4mmol) and 18-crown-6 (200mg, 0.75mmol). The reaction mixture was heated to reflux for 60 hours and then diluted with chloroform (40ml) and water (5ml). The aqueous phase was re-extracted with chloroform (4 x 40ml) and the combined organic phases dried over magnesium sulphate and concentrated *in vacuo.* The product was purified by chromatography on silica gel eluting with 20% ethyl acetate in hexane to give the title compound (300mg, 44%).
δH (CDCl₃) 4.02 (2H, d, *J* 6.9Hz), 4.00-3.70 (2H, m), 3.05-2.78 (2H, m), 2.08 (3H, s), 2.01 (1H, m), 1.61-1.41 (12H, m), 1.22 (2H, m), 0.96 (3H, t, *J* 7.3Hz).
MS (+ve ion electrospray) m/z 287 (MH⁺).

### (f) [3R, 4R]-1-(t-Butyloxycarbonyl)-3-ethyl-4-(hydroxymethyl)piperidine

Example 51e (110mg, 0.38mmol) was dissolved in methanol (5ml) and treated with 1M aqueous sodium hydroxide (1ml). The reaction mixture was stirred for 18 hours at room temperature and then concentrated *in vacuo.* The residue was partitioned in chloroform and water and the pH of the aqueous phase adjusted to 7 by the addition of dilute aqueous hydrochloric acid. The organic phase was separated and the aqueous phase re-extracted with chloroform. The combined organic phases were dried over magnesium sulphate and concentrated *in vacuo* to yield the title compound (90mg, 97%). δ ¹H (CDCl₃) 4.15-3.83 (2H, m), 3.58 (2H, m), 2.98-2.73 (2H, m), 1.85 (1H, m), 1.65-1.40 (13H, m), 1.25 (2H, m), 0.99 (3H, t, *J* 7.4Hz).

### (g) [3R, 4R]-1-(t-Butyloxycarbonyl)-3-ethyl-4-[(6-methoxyquinolinyl-4-oxy)methyl]piperidine

Example 51f (244mg, 1.00mmol) was dissolved in dry DMSO (2ml) and treated with sodium (27mg, 1. 17mmol) under argon. The reaction mixture was stirred for 2 hours, treated with 4-chloro-6-methoxyquinoline (244mg, 1.26mmol) and then heated at 60°C for 5 days. The mixture was diluted with water and the product extracted into ethyl acetate (x 3), dried over magnesium sulphate and concentrated *in vacuo.* The product was purified by chromatography on silica gel eluting with ethyl acetate to give the title compound (100mg, 25%).
δH (CDCl₃) 8.62 (1H, d, *J* 5.1Hz), 7.95 (1H, d, *J* 9.1Hz), 7.43 (1H, d, *J* 2.7Hz), 7.38 (1H, dd, *J* 9.1, 2.7Hz), 6.72 (1H, d, *J* 5.1Hz), 4.18 (2H. m), 3.94 (3H, s), 3.03 (2H, m), 2.42 (1H, m), 1.87 (1H, m), 1.75-1.43 (13, m), 1.37 (2H, m), 1.02 (3H, t. *J* 7.3Hz). MS (+ve ion electrospray) m/z 401 (MH⁺).

### (h) [3R.4R]-3-Ethyl-4-[(6-methoxyquinolinyl-4-oxy)methyl]piperidine bis-trifluoroacetate.

Example 51g ( 100mg. 0.25mmol) was dissolved in dichloromethanc (2ml). cooled to 0°C and treated with trifluoroacetic acid (2ml). The reaction mixture was allowed to warm to room temperature and stirred for a further 3 hours The mixture was concentrated *in vacuo,* re-suspended in toluene and concentrated (x 3) to yield the title compound (130mg, 100%).
δH (d₆DMSO) 9.09 (1H, d, *J* 6.5Hz), 8.52 (2H, br s, exch), 8.11 (1H, d, *J* 9.3Hz), 7.78 (1H,dd, *J* 9.3, 2.7Hz), 7.58 (1H, d, *J* 6.6Hz), 7.53 (1H, d, *J* 2.7Hz), 5.02 (1H, br s, exch)_{,} 4.56 (2H, m), 3.96 (3H, s), 3.18-3.03 (4H, m), 2.62-1.83 (4H, m), 1.44 (2H, m), 0.97 (3H, t, *J* 7.3Hz). MS (+ve ion electrospray) m/z 301 (MH+).

### (i) Title compound.

Example 51h (120mg, 0.23mmol) in DMF (3ml) was treated with potassium carbonate (125mg, 0.91mmol) and 1-iodoheptane (41ul, 0.25mmol). The mixture was stirred at room temperature for 3 hours and then concentrated *in vacuo.* The residue was redissolved in ethyl acetate, washed with water (x 2), dried over magnesium sulphate and concentrated *in vacuo.* The product was purified by chromatography on silica gel eluting with 95:4.5:0.5 chloroform/ ethanol/ 35% ammonia solution to give the title compound (80mg, 87%).
δH (CDCl₃) 8.62 (1H, d, *J* 5.2Hz), 7.94 (1H. d, *J* 9.2Hz), 7.47 (1H, d, *J* 2.7Hz), 7.35 (1H, dd, *J* 9.2, 2.7Hz), 6.72 (1H, d. *J* 5.2Hz), 4.16 (2H, m), 3.94 (3H. s), 2.62 (2H, m), 2.41-2.20 (5H, m), 1.95-1.25 (15H. m 0.98 (3H, t, *J* 7.4Hz), 0.88 (3H, t, *J* 6.8Hz). MS (+ve ion electrospray) m/z 399 (MH⁺).

### Example 52 [3R,4S]-3-Ethenyl-1-heptyl-4-[2-(6-methoxyquinolin-4-yl)-oxyethyl]piperidine.

### (a) Methyl [3R,4S]-3-ethenyl-4-piperidine acetate hydrochloride

Thionyl chloride (20ml) was added dropwise to a solution of Example 51b (10.0g, 37.1mmol) in methanol (400ml). The mixture was heated at reflux for 4 hours and then concentrated *in vacuo.* The residue was diluted with toluene and concentrated (x 3) to yield the title compound (8.10g. 100%)
δH (d₆ DMSO) 9.18 (1H, br s, exch ) 8.82 (1H, br s, exch), 6.03 (1H, m), 5.14 (2H, m). 3.59 (3H, s), 3.20-2.91 (4H, m). 2.62, (1H, m), 2.33-2.20 (3H, m), 1.80-1.52 (3H, m), MS (+ve ion electrospray) m/z 184 (MH⁺ ).

### (b) Methyl [3R,4S]-1-heptyl-3-ethenyl-4-piperidine acetate

Example 52a (8.1g, 36.9mmol) was alkylated as described in Example 12h The product was purified by chromatography on silica gel eluting with 50% ethyl acetate in hexane to give the title compound (9.71kg, 94%)
δH (CDCl₃) 6.08 (1H, m), 5.03 (2H, m), 3.68,(3H, s), 2.79-2.62 (2H. m). 2.42-1.20 (20H, m), 0.89 (3H, t. *J* 6.7Hz), MS (+ve ion electrospray) m/z 282 (MH⁺)

### (c) [3R,4S]-1-Heptyl-3-ethenyl-4-(2-hydroxyethyl)-piperidine.

Lithium aluminium hydride (750mg, 19.7mmol) was added to a solution of Example 52b (2.5g, 9.8mmol) in tetrahydrofuran (100ml) at 0°C and the mixture stirred for 1 hour at that temperature. Water (0.75ml) was added, followed by 10% aqueous sodium hydroxide solution ( 1.1 ml) and then water (1.9ml). The mixture was stirred for 1 hour and then filtered. The filtrate was concentrated *in vacuo* to yield the title compound as a orange oil (2.5g, 100%).
δH (CDCl₃) 6.12 (1H, m), 5.05 (2H, m), 3.67 (2H, t, *J* 6.5Hz), 2.76 (2H, m), 2.38-1.20 (21H, m), 0.89 (3H, t, *J* 6.8Hz). MS (+ve ion electrospray) m/z 254 (MH⁺).

### (d) [3R,4S]-3-Ethenyl-1 -heptyl-4-[2-(6-methoxyquinolin-4-yl)oxyethyl]piperidine.

Diethyl azodicarboxylate (140ul, 0.88mmol) was added to a solution of 4-hydroxy-6-methoxyquinoline (100mg, 0.58mmol), Example 52c (160mg, 0.64mmol) and triphenylphosphine (224mg, 0.86mmol) in tetrahydofuran (10ml). The reaction mixture was stirred at room temperature for 18 hours and then diluted with diethyl ether and dilute aqueous hydrochloric acid. The aqueous phase was separated, washed with diethyl ether (x 2) and then basified with aqueous potassium carbonate solution. The product was extracted with dichloromethane (x 2), dried over magnesium sulphate and concentrated *in vacuo.* The product was purified by chromatography on silica gel eluting with 97:2.7:0.3 chloroform/ ethanol/ 35% ammonia solution to give the title compound (65mg, 55%).
δH (CDCl₃) 8.61 ( 1H, d. *J* 5.3Hz), 7.95 ( 1H, d, *J* 9.2Hz), 7.46 (1H, d, *J* 2.8Hz), 7.36 (1H, dd, *J* 9.2, 2.8Hz), 6.70 (1H, d, *J* 5.3Hz), 6.19 (1H. m), 5.14 (2H, m), 4.23 (2H, m), 3.96 (3H, s), 2.80 (2H, m), 2.47-2.05 (5H, m), 1.88 (3H, m), 1.69 (2H, m), 1.46 (2H, m), 1.34-1.21 (8H, m), 0.88 (3H, t, *J* 6.8Hz). MS (+ve ion electrospray) m/z 411 (MH⁺).

### Example 53 1-Heptyl-4-[(6-methoxyquinolin-4-yl)oxyimethyl]piperidine

### (a) Ethyl 1-heptylpiperidine-4-carboxylate

Ethyl isonipecotate (5.0g, 3 1.8mmol) was dissolved in 1,2-dichloroethane (100ml) and treated with 1 -heptaldehyde (4,4ml, 31.5mmol). The reaction mixture was stirred for 10 minutes and then sodium triacetoxyborodride (6.7g, 31.7mmol) was added. After stirring for 18 hours, the reaction was diluted with dichloromethane and aqueous sodium carbonate solution. The organic phase was dried over magnesium sulphate, filtered and concentrated *in vacuo.* The product was purified by chromatography on silica gel eluting with ethyl acetate to give the title compound (2.60g, 32%).
δH (CDCI₃) 4.14 (2H, q, *J* 7.1Hz), 2.90 (2H, m), 2.32-2.23 (3H, m), 2.04-1.69 (7H, m), 1.49 (2H, m), 1.33-1.21 (10H, m), 0.89 (3H, t, *J* 6.9Hz). MS (+ve ion electrospray) m/z 256 (MH⁺).

### (b) 1-Heptyl-4-hydroxymethylpiperidine

Lithium aluminium hydride (1 .Og, 26.3mmol) was added to a solution of ethyl 1-heptylpiperidine-4-carboxylate (2.2g, 8.6mmol) in tetrahydrofuran (100ml) at 0°C. The reaction mixture was allowed to warm to room temperature and stirred for a further 66 hours. Water (Iml) was added, followed by 10% aqueous sodium hydroxide solution (1.5ml) and then water (2.5ml). The mixture was stirred for 1 hour and then filtered. The filtrate was concentrated *in vacuo* to yield the title compound as a yellow oil (1.84g, 100%).
δH (CDCl₃) 3.48 (2H, d, *J* 6.4Hz), 2.96 (2H, m), 2.30 (2H, m), 1.95-1.20 (18H, m), 0.89 (3H, t, *J* 6.8Hz). MS (+ve ion electrospray) m/z 213 (MH⁺).

### (c) 1-Heptyl-4-[(6-methoxyquinolin-4-yl)oxymethyl]piperidine

1-Heptyl-4-hydroxymethylpiperidine was treated with 4-chloro-6-methoxyquinoline (90mg, 0.47mmol) using the general method of Example 51g to give the title compound (84mg, 48%).
δH (CDCl₃) 8.61 (1H, d, *J* 5.2Hz). 7 96 (1H, d, *J* 9.1Hz), 7.47 (1H, d, *J* 2.8Hz), 7.35 (1H, dd, *J* 9.1, 2.8Hz), 6.70 (1H. d. *J* 5.2Hz), 4.05 (2H, d, *J* 6.4Hz), 3.95 (3H, s), 3.03 (2H, m), 2.37 (2H, m), 2.09-1.22 (17H, m), 0.85 (3H, t, *J* 6.7Hz). MS (+ve ion electrospray) m/z 371 (MH⁺).

### Example 54 [3R,4R]-3-Ethyl-1-heptyl-4-[(6-methoxyquinolin-4-yl)methylthiomethyl]piperidine

### (a) [3R,4R]-1-(t-Butyloxycarbonyl)-3-ethylpiperidine-4-methylthioacetate

Example 51f(612mg 2mmol) and potassium thioacetate (342mg, 3mmol) in acetone (20ml) was heated under reflux under argon for 2 hours. After cooling to room temperature the solution was concentrated and the residue purified by rapid chromatography on silica gel eluting with 10% ethyl acetate in hexanes to give the title compound as a gum (500mg. 83%).
δH (CDCl₃) 4.20-3.80 (2H, m), 3.00-2.70 [4H, m including δ 2.85 (2H. d. *J* 7.5Hz], 2.34 (3H, s), 1.80-1.10 [17H.m including δ 1.45 (9H, s)], 0.97 (3H, t, *J* 7.5Hz).

### (b) 4-Chloromethyl-6-methoxyquinoline

4-Hydroxymethyl-6-methoxyquinoline {A. Knoll, Patent DE 88044, 1950} (2.0g, 10.6mmol) was suspended in dichloromethane (100ml) and treated with thionyl chloride (3ml, 41.1mmol). The reaction mixture was stirred at room temperature for 18 hours and then concentrated *in vacuo.* The residue was re-suspended in toluene and concentrated (x3). The resulting solid was partitioned between dichloromethane and saturated sodium hydrogen carbonate solution, the organic layer was separated washed with brine, dried over magensium sulphate to give the title compound as a colourless solid.
δH (CDCl₃) 8.75 (1H, d, J 5.0Hz), 8.06 (1H, d, J 9.2Hz), 7.44-7.38 (2H, m), 7.30 (1H, d, J 2.8Hz), 4.96 (2H, s), 3.98 (3H, s), MS (+ve ion electrospray) m/z 208, 210 (MH⁺).

### (c) [3R,4R]-3-Ethyl-4-[(6-methoxyquinolin-4-yl)methylthiomethyl]piperidine

A solution of [3R,4R]-1-(*t*-butyloxycarbonyl)-3-ethylpiperidine-4-methylthioacetate (400mg, 1.33mmol) in *N,N*-dimethylformamide (5ml) and sodium methoxide (143mg, 2.66mmol) was stirred at room temperature under argon for 20 minutes. A solution of 4-chloromethyl-6-methoxyquinoline (269mg, 1.33mmol) was then added and the mixture was heated at 80°C for 2 hours. After cooling to room temperature the mixture was diluted with water and ethyl acetate. The organic layer was separated, washed with brine, dried over magnesium sulphate and concentrated. The residue was purified by chromatography on silica gel eluting with 50-100% ethyl acetate in hexanes to give [3R,4R]-1-(t-butyloxycarbonyl)-3-ethyl-4-[(6-methoxyquinolin-4-yl)methylthiomethyl]piperidine (154mg, 48%).

This material was deprotected by the general method of Example 51h to give the title compound (39mg, 34%) as a gum.
δH (CDCl3) 8.69 (1H, d, *J* 4.3Hz), 8.03 (1H, d, *J* 9.1Hz), 7.41-7.25 (3H, m), 4.07 (3H, s), 3.96 (2H, s), 3.00-2.30 (6H, m), 1.95-1.05 (7H, m), 0.85 (3H, t, *J* 7.1Hz). MS (+ve ion electrospray) m/z 331 (MH⁺).

### (d) Title compound

Example 54c (33mg, 0.1 mmol) was alkylated by the method of Example 22b to give the title compound (28mg, 65%) as a gum.
δH (CDCl₃) 8.69 (1H, d, *J* 4.3Hz), 8.03 (1H, d, *J* 9.1Hz), 7.40-7.26 (3H, m), 4.06 (2H, s), 3.96 (3H, s). 2.60-1.05 (24H, m), 0.85 (6H, m). MS (+ve ion electrospray) m/z 429 (MH⁺).

### Example 55 [3R,4R]-1-Heptyl-3-ethenyl-4-[{(6-methoxyquinoline-4-yl)carbonylamino}methyl]piperidine

### (a) [3R,4R]-1-tert-Butoxycarbonyl-3-ethenyl 4-{[(2-trimethylsilylethoxy)carbonylamino]methyl}-piperidine.

To a solution of Example 5 1c,(5.4g, 20 mmol) 2-trimethylsilylethanol (4.7 ml_{,} 22 mmol) and triethylamine (3.1 ml, 22 mmol) in xylene was added diphenylphosphoryl azide (3.1 ml, 22 mmol) and the mixture was heated under reflux for 7 h. After cooling, the mixture was washed with saturated aqueous sodium bicarbonate and brine, dried and evaporated. The crude product was chromatographed on silica gel (400g) eluted with 4:1 hexane/ethyl acetate to give the title compound, 3.04g (40%).
m.s. 407 (M.Na⁺), 351, 307.

### (b) [3R,4R]-Aminomethyl-1-tert-butoxycarbonyl-3-ethenylpiperidine.

To a solution of tetrabutylammonium fluoride (1M in tetrahydrofuran, 33 ml) was added Example 55a (3.03g, 7.9 mmol), and the mixture was stirred at 30°C for 64 h. Solvent was removed *in vacuo* and the residue was dissolved in dichloromethane and washed with saturated aqueous ammonium chloride. The organic phase was dried and evaporated, and the residue was chromatographed on silica gel (400g) eluted with 5-20% methanol/dichloromethane, with addition of 1% triethylamine in the later stages. Eluted product was dissolved in water and extracted with dichloromethane. The organic extract was washed with water, dried and evaporated to give the title compound, 0.39g (21%). m.s. 241 (MH⁺), 185, 141.

### (c) [3R,4R]-1-tert-Butoxycarbonyl-3-ethenyl-4-[{(6-methoxyquinoline-4-yl)carbonylamino}methyl]piperidine.

A mixture of Example 55b (0.39g, 1.6 mmol), 6-methoxy-4-quinoline carboxylic acid (0.32g, 1.6 mmol), 1-(3-dimethylaminopropyl)-3-ethylcarbodiimide hydrochloride (0.30g, 1.6 mmol) and 1-hydroxybenzotriazole monohydrate (50 mg) in dimethylformamide (10 ml) was stirred for 6h at room temperature. Solvent was removed *in vacuo* and the residue was partitioned between ethyl acetate and sat. aqueous sodium bicarbonate. The organic phase was washed with water, dried and evaporated. The residue was chromatographed on silica gel (40g) eluted with ethyl acetate to give the title compound, (0.56g) (82%).
m.s. 426 (MH+), 370, 326

### (d) [3R.4R]-3-Ethenyl-4-[{(6-methoxyquinoline-4-yl)carbonylamino}methyl]piperidine

Example 55c was deprotected with trifluoroacetic acid by the method of Example 51h to give the title compound, 0.77g (100%).
m.s. 326 (MH⁺)

### (e) Title compound

Example 55d (0.60g) was alkylated by the method of Example 3 to give the title compound, 0.30g (64%).
¹H NMR (CDCl₃) δ: 0.88 (3H, m), 1.27 (8H, m), 1.44 (2H, m), 1.65 (2H, m), 1.93 (1H, m), 2.05 (1H, m), 2.16-2.33 (2H, m), 2.46 (1H, m), 2.78-2.87 (2H, m), 3.38 (1H, quintet, J=7), 3.49 (1H, quintet, J=7), 3.92 (3H, s), 5.12 (2H, d, J=13), 6.16 (1H, s), 6.25 (1H, dd, J=18, 9), 7.34 (1H, d, J=4), 7.40 (1H. dd. J=9,2), 7.54 (1H, d, J=2), 8.01 (1H. d, J=9), 8.76 (1H, d, J=4).
m.s. 424 (MH⁺)

### Example 56 [3R,4R]-3-Ethenyl-1-heptyl-piperidine-4-[N-(6-methoxyquinolin-4-yl)]propionamide.

### (a) [3R,4R]-Methyl 3-[1-t-butyloxycarbonyl-3-ethenyl-piperidin-4-yl]propanoate

Example 51b (1.5g, 5.58 mmol) in ethyl acetate at 0°C was treated with N-methylmorpholine (0.72 ml, 6.56 mmol), then isobutylchloroformate (0.90 ml, 6.94 mmol). The mixture was stirred for 0.75h, then filtered and kept cold while a solution of diazomethane in ether (generated from diazolol (10.75g, 50 mmol)] was added. The mixture was stirred for 18h, then the solvent removed by blowing a stream of argon through the mixture. The crude product was purified on silica gel 60 eluting with 1:2 ethylacetate, hexane to give [3(R),4(S)-1-(t-butyloxycarbonyl)-3-ethenyl-4-piperidinylmethyl]diazomethylketone as a yellow oil (0.83g, 50%); vₘₐₓ (CH₂Cl₂) 2101, 1734, 1692, 1642 cm⁻¹. The diazoketone (0.83g, 2.83 mmol) in methanol (30 ml) was treated with silver benzoate (2.05 mmol) and triethylamine (4.7 ml, 33.6 mmol). The mixture was stirred for 3 days with the exclusion of light. The mixture was evaporated and the amide product purified by chromatograghy on silica gel eluting with 3.7 ethylacetate, hexane to give the title coumpound as a yellow oil (0.61g, 73% yield);
δ_{H} (CDCl₃, 250 MHz) 5.91-5.70 (1H, m), 5.18-5.04 (2H, m), 4.11 (1H, brs), 3.98 (1H, d, *J* 13Hz), 3.67 (3H, s), 2.96 (1H, dd. *J*3 andHz(1H. br s), 2.32 (2H. m), 1.56 (2H, m) and 1.44 (9H, s).

### (b) [3R,4R]-3-[1-t-Butyloxycarbonyl-3-ethenyl piperidin-4-yl]propanoic acid.

Example 56a (0.1 5g, 0.505 mmol) in dioxan (5 ml) with 40% aqueous sodium hydroxide solution (0.1 ml) was stirred at room temperature for 18h, then heated at 80°C for 6h. The mixture was diluted with ethyl acetate and acidified with 5N hydrochloric acid. The layers were separated and the aqueous phase extracted twice more with ethyl acetate. The combined organic extracts were washed with brine, dried over magnesium sulfate and evaporated to give the title compound as a pale yellow oil (0.14g, 98% yield); δ_{H} (CDCl₃, 250 MHz) 5.90-5.67 (1H, m), 5.16-5.04 (2H, m), 4.12 (1H, m), 3.99 (1H, d, *J* 11 Hz), 2.95 (1H, dd, *J* 3 and 13Hz), 2.77 (1H, brs), 2.36 (3H, m), and 1.57 (2H, m); m/z (negative electrospray) [M-H]⁺292.

### (c) [3R,4R]-3-Ethenyl-1-(t-butyloxycarbonyl)-piperidine-4-[N-(6-methoxyquinolin-4-yl)]propionamide

Example 56b (0.14g, 0.50 mmol) in dichloromethane (20 ml) was treated with oxalyl chloride (0.07 ml, 0.80 mmol) and N,N-dimethylformamide (1 drop). The mixture was stirred for 1h, then evaporated to dryness. The residue was diluted with toluene and dichloromethane and evaporated.

The acid chloride was dissolved in dichloromethane (10 ml) and added to a mixture of 4-amino-6-methoxyquinoline (0.09g, 0.44 mmol) and triethylamine (0.14 ml, 1.0 mmol) in dichloromethane (10 ml). The mixture was stirred for 2h, then diluted with dichloromethane and washed successively with water and brine. The solution was dried over magnesium sulfate and evaporated to give the title compound as an oil (O.087g, 40% yield);
δ_{H} (CDCl₃, 250 MHz) 8.71 (1H. d. *J* 5Hz), 8.16 (1H, s), 8.03 (1H, d, *J* 9Hz), 7.82 (1H, s), 7.39 (1H, dd, *J* 2.54 and 9Hz), 7.02 (111, d, *J* 2.5 Hz), 5.93-5.74 (1H, m), 5.25-5.08 (2H, m), 4.25-3.90 (2H, m), 3.94 (3H. s). 2.96 (1H, d, *J* 14.5 Hz), 2.79 (1H, brs), 2.55 (1H, m), 2.36 (1H, m), 1.73 (4H, s) and 1.45 (9H, s).

### (d) Title compound

Example 56cwas deprotected with trifluoroacetic acid (2ml) as described in Example 51h. The crude amine was alkylated as described in Example 12b. The crude product was purified on silica gel eluting with 1-4% methanol in dichloromethane to give a colourless glass (0.047g, 58% yield);
δH (CDCl₃, 250 MHz)8.69 ( 1H, m). 8. 19-8.04 (1H, m), 8.00 (1H, d, *J* 10 Hz), 7.30 (1H, dd, *J* 2.5 and 9.5 Hz), 7.06 (1H, d, *J* 3 Hz), 6.21-6.02 (1H, m), 5.13-5.01 (2H, m), 3.90 (3H, s), 2.89-2.68 (2H, m), 2.53-2.42 (2H, 242-2.09 (6H, m), 2.09-1.94 (1H, m), 1.72-1.60 (2H, m), 1.59-1.37 (4H, m), 1.27 (7H, s) and 0.88 (3H, m); m/z (positive electrospray) MH⁺ 438.

### Example 57 [3R,4R]-3-Ethenyl-1-heptyl-piperidine-4-[N-(6-methoxyquinolin-4-yl)]propylamine.

Example 56d (0.019g, 0.045 mmol) in tetrahydrofuran (4 ml) was treated with 1M lithium aluminium hydride (0.15 ml) and heated at reflux for 2h. The mixture was then allowed to cool and diluted with water (0.2 ml) and 40% aqueous sodium hydroxide (0.1 ml). The mixture was evaporated and the crude product purified on silica gel eluting with 5% methanol in chloroform containing 0.5% 0.88 ammonia to give the title compound as an oil (0.014g, 76% yield);
δ_{H} (CDCl₃, 250 MHz) 8.45 (1H. d, *J* 5Hz), 7.91 (1H. d, *J* 9Hz), 7.40-7.22 (1H, m), 6.95 (1H, s), 6.40 (1H, d, *J* 5Hz), 6.37-6.04 (1H, m), 5.22-4.96 (2H, m), 4.80 (1H, s), 3.92 (3H, s), 3.28 (2H, m), 2.80 (2H, m), 2.45-1.90 (4H, m), 1.90-1.10 (9H, m), 0.88 (3H, m); m/z (positive electrospray) MH⁺ 424.

### Example 58 [3R,4S]-3-Ethenyl-1-heptyl-piperidine-4-[N-(6-methoxyquinolin-4-yl)]acetamide.

The title compound was prepared by the methods of Examples 56c and 56d from Example 51 b and 4-amino-6-methoxyquinoline.

The crude product was chromatographed on silica gel, eluting with 0-5% methanol in dichloromethane (gradient), giving the title compound as a colourless glass. δ_{H} (CDCl₃) 8.68 (d), 8.39 (s), 8.16 (d), 7.97 (d), 7.33 (dd), 7.08 (d), 6.17 (m), 5.15-5.0 (m), 3.83 (s), 2.9-2.6 (m), 2.5-1.95 (m), 1.75-1.2 (m), 0.88 (t);
mass spectrum EI MH⁺ 424 (C₂₆H₃₇N₃O₂).

### Example 59 [3R,4R]-3-Ethenyl-1-heptyl-piperidine-4-[N-(6-methoxyquinolin-4-yl)]ethylamine.

The product from Example 58 (0.33g) was reduced by the method of Example 57. Chromatographed on silica gel, eluting with 0-12% (9:1 ethanol / 880 ammonia) in dichloromethane (gradient), gave the title compound (0.24g) as a yellow gum which solidified slowly on standing.
δ_{H} (CDCl₃) 8.42 (d), 7.90 (d), 7.28 (dd), 7.00 (d), 6.40 (d), 6.15 (m), 5.2-5.0 (m), 3.88 (s), 3.72 (m), 3.4 (b), 3.30 (m), 2.77 (m), 2 .4-2.0 (m), 1.75-1.2 (m), 0.88 (t);
mass spectrum El MH⁺ 410 (C₂₆H₃₉N₃O).

### Example 60 [3R,4S]-3-Ethenyl-1-heptyl-4-[2-(R,S)-hydroxy-2-(6-methoxyquinolin-4-yl)ethyl]piperidine

### (a) [3R,4R]-1-(t-Butoxycarbonyl)-3-ethenyl-4-[1-(R,S)-methoxycarbonyl-2-oxo-2-(6-methoxyquinolin-4-yl)ethyl]piperidine and [3R,4R]-1-(methoxycarbonyl)-3-ethenyl-4-[1-(R,S)-methoxycarbonyl-2-oxo-2-(6-methoxyquinolin-4-yl)ethyl]piperidine

Sodium amide (90%; 2.0 g) was added cautiously to a mixture of methyl [6-methoxy-quinolin-4-yl] carboxylate (8.3 g; 0.038 mol) and methyl [1-*tert*-butyloxycarbonyl)-3-(R)-ethenyl-piperidin-4-(S)-yl]-acetate (prepared from the reaction of Example 51b with diazomethane in ether) (8.5 g; 0.03 mol) in dry toluene (15 ml) under argon and the mixture was heated at 120 °C (oil bath) with stirring for 3 days. The cooled product was treated with aqueous ammonium chloride and extracted with dichloromethane (4 x 100 ml). The organic fraction was washed with water, dried over anhydrous sodium sulphate, and evaporated to dryness *in vacuo* to afford a brown oil that was chromatographed on a Biotage silica column (90 g) eluting with 10% - 30% ethyl acetate in hexane. The first product collected was [3R,4R]-1-(t-Butoxycarbonyl)-3-ethenyl-4-[1-(R,S)-methoxycarbonyl-2-oxo-2-(6-methoxyquinolin-4-yl)ethylJpiperidine (3.0 g). Found: El MH⁺ 469. C₂₆H₃₂N₂O₆ requires M 468.

Further elution afforded [3R,4R]-1-methoxycarbonyl-3-ethenyl-4-[1-(R.S)-methoxycarbonyl-2-oxo-2-(6-methoxyquinolin-4-yl)ethyl]piperidine (3.0 g). Found: El MH⁺ 427. C₂₃H₂₆N₂O6 requires M, 426

### (b) [3R,4S]-3-Ethenyl-4-[2-oxo-2-(6-methoxyquinolin-4-yl)ethyl]piperidine dihydrochloride

### Method A

A solution of [3R,4R]-1-(t-Butoxycarbonyl)-3-ethenyl-4-[1-(R,S)-methoxycarbonyl-2-oxo-2-(6-methoxyquinolin-4-yl)ethyl]piperidine (Example 60a, 2.9 g) in tetrahdrofuran (50 ml) and 2M hydrochloric acid (200 ml) was heated under reflux for 4.5 hours and evaporated to dryness *in vacuo.* The residue was azeotroped with dry toluene (x 2) to afford the title compound as a hygroscopic foam (2.24 g).

### Method B

A solution of [3R.4R]-1-methoxycarbonyl-3-ethenyl-4-[1-(R,S)-methoxycarbonyl-2-oxo-2-(6-methoxyquinolin-4-yl)ethyl]piperidine(Example 60a, 3.0 g) in 5M hydrochloric acid (100 ml) was heated under reflux for 18 hours. The product was evaporated to dryness *in vacuo*, basified with 2N sodium hydroxide solution, and extracted with toluene (3 x 100 ml). The organic traction was washed with brine, dried with anhydrous sodium sulphate and evaporated to dryness to afford the title compound (1.96 g) as the oily free base.
δ¹H (CDCl₃) 8.85 (d, *J* 4Hz 1H), 8.03 (d, *J* 8Hz, 1H), 7.75 (d, *J* 2 Hz 1H), 7.55 (d, *J* 4Hz, 1H), 7.40 (dd, *J* 8,2 Hz, 1H), 6.15 (m, 1H), 5.10 (m, 2H), 3.95 (s, 3H), 2.30 - 3.20 (m,), 1.60 (m, 3H). Found: EI MH⁺ 311. C₁₉H₂₂N₂O₂ requires M 310.

### (c) [3R,4S]-3-Ethenyl-1-heptyl-4-(2-oxo-2-(6-methoxyquinolin-4-yl)ethyl]piperidine

Example 60b (1.4 g; 3.68 mmol) was alkylated as described in Example 3. The product was chromatographed on a Biotage silica column (90 g) eluting with ethyl acetate-hexane (1:1) to afford the title compound (1.33 g; 88.5 %).
81H (CDCl₃) 8.85 (d, *J* 4Hz 1H). 8.03 (d, *J* 8Hz, 1H), 7.75 (d, *J* 2 Hz 1H), 7.56 (d, *J* 4Hz, 1H), 7.41 (dd, *J* 8,2 Hz, 1H), 6.17 (m, 1H), 5.10 (m, 2H), 3.94 (s, 3H), 2.10 - 3.15 (m), 1.20 - 1.80 (m), 0.88 (t, *J* 6 Hz, 3H). Found: EI MH+ 409. C₂₆H₃₆N₂O₂ requires MH, 409.

### (d) Title compound.

Example 60c was reduced as described in Example 2 to afford an oil (0.21 g) containing the title compound as a 1:1 mixture of diastereomers.
δ¹H (CDCl₃) 8.85 (d, *J* 4Hz 1H), 8.03 (d, *J* 8Hz, 1H), 7.75 (d, *J* 2 Hz 1H), 7.56 (d, J 4Hz, 1H), 7.41 (dd, *J* 8,2 Hz, 1H), 6.17 (m, 1H), 5.10 (m, 2H), 3.94 (s, 3H), 2.10 - 3.15 (m), 1.20 - 1.80 (m), 0.88 (t, *J* 6 Hz, 3H). Found: EI MH⁺ 411. C₂₆H₃₈N₂O₂ requires MH,411.

### Example 61 [3R,4R]-3-Ethenyl-1-heptyl-4-[2-(6-methoxyquinolin-4-yl)ethyl]piperidine

Example 60c ( 0.30 g) was reduced as described in Example 12a to afford the title compound (0.030 g) as an oil.
δ¹H (CDCl₃) 8.64 (d, *J* 4Hz 1H). 8.00 (d. *J* 8Hz. 1H), 7.35 (dd, *J* 8,2 Hz, 1H), 7.15 (m, 2H), 6.17 (m, 1H), 5.10 (m, 2H), 3.94 (s, 3H), 2.00 - 3.15 (m), 1.20 - 1.80 (m), 0.88 (t, *J* 6 Hz, 3H). Found: EI MH⁺ 395. C₂₆H₃₈N₂O₂ requires M 394.

### Example 62 [3R,4R]-3-Ethyl-1-heptyl-4-[2-(6-methoxyquinolin-4-yl)ethyl)piperidine.

### (a) [3R,4R]-3-Ethyl-4-[2-(6-methoxyquinolin-4-yl)ethyl]piperidine.

Example 60b ( 0.30 g) was reduced as described in Example 12a for 16h. The product was chromatographed on a Biotage silica gel column (40 g) eluting with ethanol-dichloromethane-35% ammonia (10:80:1 to afford firstly Example 61 then the title compound (0.089 g) as an oil.
δ¹H (CDCl₃) 8.65 (d, *J* 4Hz 1H), 8.01 (d, *J* 8Hz, 1H), 7.35 (dd, *J* 8,2 Hz, 1H), 7.20 (m, 2H), 3.94 (s, 3H), 2.50-3.10 (m), 1.20 - 1.85 (m), 0.91 (t, *J* 7 Hz, 3H). Found: EI MH⁺ 299. C₁₉H₂₆N₂O requires M 298

### (b) [3R,4R]-3-Ethyl-1-heptyl-4-[2-(6-methoxyquinolin-4-yl)ethyl]piperidine.

Example 62a was alkylated as described in Example 3. The product was chromatographed on a Sep Pak silica column (10 g) eluting with ethyl acetate-hexane (1:1) to afford the title compound (0.083 g).
δ¹H (CDCl₃) 8.68 (d, *J* 4Hz 1H), 8.03 (d, *J* 8Hz, 1H), 7.35 (dd, *J* 8,2 Hz, 1H), 7.15 (m, 2H), 3.95 (s, 3H), 2.10 - 3.15 (m), 1.20 - 1.80 (m), 0.89 (m, 6H). Found: EI MH⁺ 397. C₂₆H₄₀N₂O₂ requires M 396.

### Example 63. 1-Heptyl-4-[2(R,S)-hydroxy-2-(6-methoxy-4-quinolinyl)ethyl]-piperidine

### (a) 4-[2(R,S)-hydroxy-2-(6-methoxy-4-quinolinyl)ethyl]piperidine

The title compound was prepared by the addition of sodium borohydride to a solution of4-[2-(6-methoxy-4-quinolinyl)-2-oxo-ethyl]piperidine [EP 31753A1] in in *iso*propanol as described for Example 2.

### (b) Title compound

A suspension of Example 63a (314mg, 1mmol), potassium carbonate (552mg, 4mmol) and 1-bromoheptane (0.1 8ml, 1.15mmol) in dry DMF (10ml)was heated to 80°C under an argon atmosphere for 3h. The reaction mixture was then partitioned between ethyl acetate and water and the organic layer washed four times with water. The organic layer was dried (magnesium sulphate) and concentrated to afford a brown oil (336mg). The crude material was purified by silica chromatography eluting with a 90:9:1 mixture of dichloromethane:methanol:0.88 ammonia to afford the *title* compound (258mg, 63%) which was then crystallised from ethyl acetate: MS (electrospray) 385MH⁺; NMR (CDCl₃) 8.73 (1H, d, J 4.5Hz). 8.02 (1H, d, J 9.1 Hz), 7.53 (1H, d, J 4.5Hz). 7.36 (1H, dd, J 9.2, 2.7Hz), 7.18 (1H, d, 2.7Hz). 5.47 (1H, m), 3.92 (3H, s), 2.97 (2H, m), 2.30 (2H, m), 2.08-1.89 (3H, m), 1.75 (4H, m), 1.6-1.2 (12H, m), 0.87 (3H, t. J 6.4Hz).

### Example 64. [3S,4R]-3-Ethenyl-1-heptyl-4-[2-(6-methoxyquinolin-4-yl)ethyl]piperidine.

### (a) [3S,4R]-3-Ethenyl-4-[2-(6-methoxyquinolin-4-yl)ethyl]piperidine.

A solution of Example 61 (0.14 g) in water (1.0 ml) was epimerised as described in Example 29. The product was chromatographed on a Sep Pak silica gel column (10 g) eluting with ethanol-dichloromethane -35% ammonia (10:80:1) to afford the title compound (0.065 g) as an oil.
δ¹H (CDCl₃) 5.50 (m, 1H), 5.05 (m, 2H), Found: EI MH⁺ 297. C₁₉H₂₄N₂O requires MH, 297.

### (b) Title compound.

Example 64a was alkylated as described in Example 3 to afford the title compound (0.017 g) as an oil.
δ¹H (CDCl₃) 8.65 (d, *J* 4Hz 1H), 7.95 (d, *J* 8Hz, 1H), 7.35 (dd, *J* 8,2 Hz, 1H), 7.18 (m, 2H), 5.58 (m, 1H), 5.07 (m, 2H), 3.93 (s, 3H), 2.80 - 3.15 (m), 1.20 - 2.35 (m), 0.88 (t, *J* 6 Hz, 3H). Found: El MH⁺ 395. C₂₆H₃₈N₂O₂ requires M 394.

### Example 65 N-(6-Methoxy-4-quinolinyl)-1-heptyl-4-piperidinecarboxamide

Example 53a (375mg, 1.47 mmol) in THF (10ml) containing aqueous sodium hydroxide was heated under reflux for 2 hours. After cooling the solution was acidified with conc.HCl and the solvent evaporated. The residue was suspended in dichloromethane (10ml), then oxalylchloride (0.39ml, 4.4mmol) and DMF (1 drop) were added. The mixture was stirred at room temperature for 1 hour, and the solution was evaporated in vacuo. The solid was dissolved in dichloromethane (4ml) and added to a solution of 4-amino-6-methoxyquinoline (256mg, 1.47mmol) and 4-dimethylaminopyridine (359mg, 2.94mmol) in dichloromethane (10ml). The mixture was stirred at room temperature for 1h, and the solvent was evaporated *in vacuo.* The residue was dissolved in ethyl acetate and the solution washed with aqueous sodium bicarbonate. The organic layer was dried over magnesium sulphate and evaporated in vacuo. The crude product was purified by column chromatography eluting with 2 to 10% (9:1 methanol/.880 ammonia)/dichloromethane to afford the title compound (1 10mg, 20%) as an off-white solid.
δ¹H (CDCl₃)8.71 (1H, d, *J* 5.0 Hz). 8.17 (1H, d,*J* 5.0 Hz), 8.03 (1 H, d. *J* 9.3 Hz). 7.83 (1H, br s), 7.39 (1H, dd. *J* 2.5. 9.3 Hz). 6.99 (1 H. d. *J* 2.5 Hz). 3.96 (3H, s). 3.10-3.00 (2H, m). 2.56-1.22 (21H, m), 0.94-0.80 (3H, t, *J*6.1)
MS (+ve ion electrospray) m/z 384 (MH+)

### Example 66 (3Z)-(4R)-3-Ethylidene-1-heptyl-4-[3-(6-methoxyquinolin-4-yl)propyl]piperidine.

### (a) (3Z)-(4R)-3-Ethylidene-4-[3-(6-hydroxyquinolin-4-yl)propyl]piperidine

Lithium ribbon (2.8g, 400mmol) was dissolved in dry THF (100ml) containing triphenylphasphine (20g, 76mmol) and the mixture stirred at room temperature for 6 hours.

Reference Example 26a (5.0g, 16.1mmol) was dissolved in dry THF (50ml) and 22.5ml of a 1.04M solution of diphenylphosphine in benzene was added, followed by 60ml of the deep red solution resulting from the reaction of lithium ribbon with triphenylphosphine.

The resulting mixture was heated at reflux under argon for 60 hours. After cooling, the reaction was diluted with chloroform (230ml) and water (120ml). The pH of the mixture was adjusted to 9 by the addition of concentrated hydrochloric acid. After separating, the organic phase was washed with brine, dried over magnesium sulphate and concentrated *in vacuo.* The product was partially purified by chromatography on silica gel eluting with 80:18:2 chloroform/ ethanol/35% ammonia solution to give partially pure title compound (838mg).
δ ¹H (CDCl₃) *inter alia* 5.18 (1H, q, *J* 6.7Hz), 1.60 (3H, d, *J* 6.7Hz). MS (-ve ion electrospray) m/z 295 [M-H]⁻.

### (b) (3Z)-(4R)-3-Ethylidene-1-heptyl-4-[3-(6-hydroxyquinolin-4-yl)propyl]piperidine

Partially purified Example 66a was alkylated as described in Example 22b. The product was purified by chromatography on silica gel eluting with 95:4.5:0.5 chloroform/ethanol/35% ammonia solution to give the title compound (112mg).
δ ¹H (CDCl₃) 8.60 (1H, d, *J* 4.4Hz), 7.98 (1H, d, *J* 9.0Hz), 7.32 (1H, dd, *J* 9.0, 2.6Hz), 7.27 (1H, d, *J* 2.6Hz), 7.13 (1H, d, *J* 4.4Hz), 5.25 (1H, br s, exch), 5.17 (1H, q, *J* 6.7Hz), 3.25 (1H, d, *J* 12.2Hz), 2.94-2.70 (4H, m), 2.47-2.39 (2H, m), 2.00 (1H, m), 1.79 (1H, m), 1.71-1.52 (5H, m) 1.61 (3H, d, *J* 6.7Hz),1.40-1.21 (11H, m), 0.85 (3H, t, *J* 7.0Hz). MS (+ve ion electrospray) m/z 395 (MH⁺).

### (c) Title compound

(3*Z*)-(4*R*)-3-Ethylidene-1-heptyl-4-[3-(6-hydroxyquinolin-4-yl)propyl]piperidine (49mg, 0.12mmol) was dissolved in methanol:acetonitrile (1:9, 6ml). *N,N*-Diisopropylethylamine (22ul, 0.12mmol) was added, followed by trimethylsilyldiazomethane (62ul, 0.12mmol). The reaction mixture was stirred for 18 hours at room temperature and then concentrated *in vacuo.* The product was purified by chromatography on silica gel eluting with 95:4.5:0.5 chloroform/ ethanol/35% ammonia solution to give the title compound (10mg, 20%).
δH (CDCl₃) 8.67 (1H, d, *J* 4.4Hz), 8.00 (1H, d, *J* 9.0HZ), 7.37 (1H, dd, *J* 9.0, 2.6Hz), 7.22 (1H, d, *J* 2.6Hz), 7.20 (1H, d, *J* 4.4Hz), 5.26 (1H, q, *J* 6.7Hz), 3.96 (3H, s), 3.16-2.99 (3H, m), 2.79 (2H, m), 2.45-2.32 (3H, m), 2.05 (1H, m), 1.92-1.23 (16H, m), 1.65 (3H, d, *J* 6.7Hz), 0.88 (3H, t, *J* 6.8Hz). MS (+ve ion electrospray) m/z 409 (MH+).

### Example 67. [3R,4S] -l-Cinnamyl-4-[2-(6-naethoxyqumolin-4-yl)-oxyethyl]piperidine.

### (a) 6-Methoxy-4-chloroquinoline.

To POCl₃ (5 ml, 53.7 mmol) cooled in ice 6-methoxyquinoline N-oxide (1 g, 5.7 mmol) was added in portions. After addition the mixture was refluxed gently for 30 mins. The cooled mixture was poured with stirring to crushed ice (25 g). Successive partial neutralisations with conc. aq. ammonia precipitated first the 2-chloro derivative and then the 4-chloro derivative. The 6-methoxy-4-chloroquinoline was partitioned between water and ethyl acetate, the combined organic extracts were dried over NaSO₄ and concentrated *in vacuo.* Yield, 0.31 g, 28%.
**MS.**: m/z 193/195 (M+H)⁺

### (b) [3R,4S] -1-t-Butoxycarbonyl-4-[2-(6-methoxyquinolin-4-yl)-oxyethyl]piperidine.

To a solution of 2-(1-t-butoxycarbonyloxypiperidin-4-yl) ethanol (0.1 g, 0.44 mmol) in dry DMSO (0.5 ml), sodium metal (0.01 g, 0.44 mmol) was added under argon and left to stir for 1 hour. After 1 hour Example 67a (0.08 g, 0.44 mmol) was added and microwaved for successive 2 minute bursts'at 50W. After 8 mins the mixture was allowed to cool and was poured into water (50 ml). The mixture was then extracted with ether (3x50 ml). The organic extracts were then combined and dried over NaSO₄ and concentrated under vacuum. The crude product was purified by column chromatography on silica gel 60, pre-absorbing onto silica and eluting with 0-30% EtOAc/Hexane. Yield, 0.07 g.
**MS.** : m/z 386 (M+H)+

### (c) [3R.4S ]-4-[2-(6-methoxyquinolin-4-yl)-oxyethyl]piperidine

Example 67b (0.065 g. 0.17 mmol) was dissolved in DCM (10 ml), TFA (5 ml) was added dropwise over -15-20mins, with stirring at 0°C. After stirring O/N saturated potassium bicarbonate solution was added until pH-9. The solution was then extracted with DCM (3x100 ml), washed with brine (100 ml), dried over NaSO₄ and then concentrated under vacuum. Yield, 0.322 g. 65%.
**MS.**: m/z 286 (M+H)⁺

### (d) Title compound

REM resin [1.2 mmol/g] (0.664 g, 0.79 mmol), and Example 67c (0.251 g, 0.88 mmol) were suspended in dry DMF (8 ml) and agitated on a rotator for ~96 hours. The resin was then washed with DMF (3x15 ml), DCM (3x15 ml), 3:1 DCM/MeOH (15 ml), 1:1 DCM/MeOH (15 ml), 1:3 DCM/MeOH (15 ml), MeOH (3x15 ml) and then left O/N under high vac.

This resin was suspended in dry DMF (10 ml), cinnamyl bromide (1 ml, 6.7 mmol) was added. This was then agitated on a rotator for 24 hours. The resin was then washed with DMF (10 ml). DCM (10 ml), 3:1 DCM/MeOH (10 ml), 1:1 DCM/MeOH (10 ml), 1:3 DCM/MeOH (10 ml), MeOH (2x10 ml) and then left O/N under high vac.

This resin was then suspended in dry DCM (5 ml) and triethylamine (1.5 ml, 11 mmol) was added. The solution was then agitated on a rotator at room temp O/N. The resin was then washed with DCM (3x10 ml). The washings were combined and poured into saturated KCO₃ (30 ml) and extracted with ethyl acetate (3x30 ml), dried and then concentrated under vacuum. The crude product was purified by column chromatography (CH2Cl2:MeOH:NH3; 9:1:0.1) to give the title compound ( 0.016 g).
¹H NMR (CDCl₃):1.64 (4H, m); 1.85 (1H, d, J = 12.8Hz); 1.94 (2H, q, J = 6.4Hz): 2.05 (2H, m); 3.08 (2H, m); 3.22 (2H, m); 3.92 (3H, s); 4.25 (2H, t, J = 6.4Hz); 6.33 (1H, m); 6.53 (1H, ); 5.69 (1H, d, J = 5.2Hz); 7.30 (4H, m); 7.44 (1H, d, J = 2.8Hz); 7.94 (1H, d_{,} J = 9.3Hz); 8.60 (1H, d, J = 4.8Hz).
**MS.**: m/z 402 (M+H)+

### Example 68 [3R,4R]-3-(2-Acetoxyethyl)-1-heptyl-4-[3-(6-methoxy-quinolin-4-yl)propyl]piperidine

Example 31 (0.100g) was dissolved in dry pyridine (5 ml) and stirred at room temperature while acetic anhydride (132 ul) and 4-dimethylaminopyridine DMAP (catalytic amount) were added. The reaction mixture was stirred at room temperature for 60 hours. The mixture was diluted with ethyl acetate (50 ml), washed with water (3 x 20 ml), dried over anhydrous magnesium sulphate. filtered and evaporated. This gave the title compound (0.083g, 75%).
δH (CDCl₃) 1.98 (s, 3H)
El MH⁺ ,469. C₂₉H₄₄N₂O₃ requires MH, 469.

### Example 69 [3R,4R]-3-Ethyl-1-heptyl-4-[3-(6-{2-hydroxyethyloxy}quinolin-4-yl)propyl]piperidine

### (a) [3R,4R]-3-Ethyl-1-heptyl-4-[3-(6-{tert-butyloxycarbonyl}methyloxyquinolin-4-yl)propyl]piperidine

The title compound (0.18g, 52%) was prepared from Example 22a using the general alkyation method of Example 23 and t-butylbromoacetate.
MS (+ve ion electrospray) m/Z 511 (MH+)

### (b) Title compound

Example 69a (0.18g, 0.00035 mole) was reduced as described in Reference Example 26c. Column chromatography eluting with 2% (9:1 methanol/.880 ammonia)/dichloromethane afforded the title compound (0.09g, 58%) as a colourless gum.
MS (+ve ion electrospray) m/z 441 (MH+)

### Example 70 (3R,4R]-3-(Ethylaminocarbonyloxyethyl)-1-heptyl-4-(3-(6-methoxyquinolin-4-yl)propyl]piperidine

Example 31 (0.5g, 0.7mmol) was dissolved in toluene (2ml) and ethylisocyanate (0.06ml, 0.77mmol) added dropwise. After stirring at 50°C for 3 h, sat. sodium bicarbonate solution (50ml) was added and the products extracted with ethyl acetate. Drying, evaporation to dryness and column chromatography gave the product as aclear oil (0.323g, 67%).
E.I. MH⁺, 498. C₃₇H₂₇N₃O₃ requires MH, 498.

### Example 71 [3R,4R]-3-Ethenyl-1-heptyl-4-[3-(R,S)-aminocarbonylamino-3-(6-methoxyquinolin-4-yl)propyl]piperidine

Example 43 (0.250g) was dissolved in hydrochloric acid (0.2 ml of 5M and diluted to 10 ml) and THF (3 ml). Potassium cyanate (0.053g) was added and the reaction mixture heated at 100 °C for 50 minutes. Saturated sodium bicarbonate solution (40 ml) was added and the mixture extracted with ethyl acetate (2x50 ml). The combined organic extracts were dried over anhydrous magnesium sulphate, filtered and the solvent evaporated. Column chromatography on silica-gel gave the title compound as a cream coloured gel (0.076g, 28%).
E.I. MH⁺ 467. C₂₈H₄₂N₄O₂ requires MH, 467.

### Example 72 [3R,4R]-3-Ethyl-1-heptyl-4-[3-(6-(4-aminobutyloxy)-quinolin-4-yl)propyl]piperidine

The title compound was prepared from Example 14 and N-5-bromobutylphthalimide by an analogous process to those described in Reference Example 16 and Example 17.
E.I. MH⁺, 468. C₃₀H₄₉N₃O requires MH, 468.

### Example 73 [3R, 4R]-1-Heptyl-3-(1-(R)- and 1-(S)-hydroxy-2-methoxyethyl)-4-[3-(6-methoxyquinolin-4-yl) propyl] piperidine

### a) [3R, 4R]-1-Benzyloxycarbonyl-3-(1-(R, S )-2-dihydroxyethyl)-4-[3-(6-methoxyquinolin-4-yl) propyl] piperidine

Example 33a was dihydroxylated using osmium tetroxide as in the method of Example 47 giving the title compounds (2:1 mixture of inseparable isomers) in 80% yield.
EI M⁺H 479 C₂₈H₃₄N₂O₅ requires 478.

### b) [3R, 4R]-1-Benzyloxycarbonyl-3-(2-(R, S)-oxiranyl)-4-[3-(6-methoxyquinolin-4-yl) propyl] piperidine.

This was prepared from Example 73a by a modification of a related procedure by S. Takano *et al.* Synthesis, 1983, 116.

The above diol (Example 73a) (9.0g 18.8 mmol) was dissolved in toluene (150ml) then triphenylphosphine (7.4g, 28.2 mmol) and diethylazodicarboxylate (4.9g_{,} 28.2 mmol) were added. The mixture was heated to reflux under argon for 2.5 days. Evaporation and chromatography on silica eluting with a gradient of ethyl acetate/hexane (70/30) to neat ethyl acetate afforded a white solid (20.0g). Analysis of this material showed it to contain ca. 9g of the title compound, the balance being triphenylphosphine oxide.

E.I MH⁺ 461 C₂₈H₃₂N₂O₄ requires 460

### c) [3R, 4R]-1-Benzyloxycarbonyl-3-(1-(R, S)-hydroxy-2-methoxyethyl)-4-[3-(6-methoxyquinolin-4-yl) propyl] piperidine.

Epoxide (Example 73b) (3.7g, approximately 1.9g of epoxide, 4.0 mmol) was dissolved in methanol (40 ml) and treated at 0°C with boron trifluoride etherate (1.5 ml, 1.8g, 12.0 mmol) The mixture was stirred tor 5 h at room temperature then evaporated to dryness giving a white foam (5.0 g).
E. I. M⁺H 493, C₂₉H₃₆N₂O₅ requires 492.

### d) [3R, 4R]-3-(1-(R, S)-Hydroxy-2-methoxyethyl)4-[3-(6-methoxyquinolin-4-yl) propyl] piperidine.

The above methyl ethers (Example 73c) (5.0g)were hydrogenated using the procedure of Example 34b to give the title compounds as yellow oil after chromatographic purification ( 1.14g, approximately80% from the epoxide).
E.I. M⁺H 359. C₂₁H₃₀N₂O₃ requires 358.

### e) Title compounds.

The title compounds were prepared as a separable mixture by alkylating with heptyl iodide (1.1 equivalents) and using potassium carbonate (1.2 equivalents) in N,N-dimethyl formamide at room temperature for 3 hours. Evaporation extractive workup (ethyl acetate-water) and chromatography on silica afforded the individual diastereomers in a combined yield of 40%.
E.I. M⁺H 457, C₂₈H₄₄N₂O₃ requires 456.

### Example 74 [3R, 4R]-1-Heptyl-3-(1-(R)- and 1-(S)-hydroxy-2-methylthioethyl)-4-[3-(6-methoxyquinolin-4-yl) propyl]piperidine

### a) [3R, 4R]-1-Benzyloxycarbonyl-3-(1-(R,S)-hydroxy-2-methylthioethyl)-4-[3-(6-methoxyquinolin-4-yl) propyl] piperidine.

Epoxide 73b(2g, equivalent to 1g of epoxide, 2.2 mmol) was dissolved in N,N-dimethyl formamide (25ml) and treated with sodium thiomethoxide (0.31g, 4.4 mmol). After 4.5h the mixture was evaporated and the residue partitioned between ethyl acetate-water/saturated aqueous sodium bicarbonate (2/1 ratio by volume ). The organic extract was dried and evaporated. The residue was purified by chromatography on silica eluting with an ethyl acetate-hexane gradient.
E.I. M⁺H 509, C₂₉H₃₆N₂O₄S requires 508.

### b) [3R, 4R]-3-(1-(R,S)-hydroxy-2-methylthioethyl)-4-[3-(6-methoxyquinolin -4-yl) propyl] piperidine.

This was prepared by hydrogenation of the above compound, Example 74a, according to the method of Example 34b. with the modification that "Palladium Black" catalyst was required instead of the usual 10% palladium on charcol.
E.I. M⁺H 375, C₂₁H₃₀N₂O₂S requires 374.

### c) Title compounds.

The title compounds were prepared by N-heptylation of Example 74b by the procedure of Example 73e followed hy chromatography on silica eluting an ethanol/ethyl acetate gradient to give the individual diastereomers (32mg and 22 mg) as oils in a combined yield of 23%.
E.I. M⁺H 473, C₂₈H₄₄N₂O₂S require 472.

### Example 75 [3R, 4R]-1-(5-Methylhexyl)-3-(1-(R)- and 1-(S),2-dihydroxyethyl)-4-[3-(6-methoxyquinolin-4-yl)propyl]piperidine

The title compounds were prepared from the corresponding N-benzyloxycarbonyl protected piperidines (Example 73a) by hydrogenolytic removal of the N-benzyloxycarbonyl protecting group ( according to the procedure of Example 34b) ) and alkylation with 5-methylhexylbromide using potassium carbonate as base in N,N-dimethylforamide followed by chromatography on silica eluting with a 0 to 5% gradient of ethanol in ethyl acetate. This gave 140mg and 170mg of the individual diastereomers. E.I M⁺H 443, C₂₇H₄₂N₂O₇ requires 442.

### Example 76 (3R, 4R]-3-Ethyl-1-heptyl-4-[3-(6-(3-aminopropyl)oxyquinolin-4-yl) propyl]piperidine

This was prepared from Example 22 by alkylation with N-3-bromopropylphthalimide following the procedure of Reference Example 16, then removal of the phthalimide protecting group with methylhydrazine following the procedure of Example 17.
E.I. M⁺H 454, C₂₉H₄₇N₃O requires 453.

### Example 77 [3R, 4R]-3-Ethyl-1-heptyl-4-[3-(6-(2-aminoethyl)oxyquinolin-4-yl) propyl]piperidine

This was prepared from Example 22 by the process of Example 76, with the variation that N-2-bromoethylphthalimide was used in placed of N-bromopropylphthalimide
E.I. M⁺H 440, C₂₈H₄₅N₃O requires 439.

### Example 78 (3R,4R]-3-Ethyl-1-heptyl-4-[3-(6-(3-guanidinopropyl)oxyqninolin-4-yl) propyl] piperidine

### a) [3R,4R]-3-Ethyl-l-heptyl-4-[3-(6-N', N"-bis-t-buytloxycarbonylguanidinopropyloxy quinolin-4-yl) propyl) piperidine.

A solution of Example 76 (0.41g, 0.9 mmol) was dissolved in N,N-dimethylformamide (1.2 ml), then treated with N,N-bis-t-butoxycarbonyl-thiourea (0.3g, 1.1 mmol) and a suspension of 2-chloro-1-methyl-pyridinium iodide (0.27g, 1.1 mmol) Mukaiyama's reagent) in N,N-dimethylformamide (2.5 ml). After 12h the reaction mixture was diluted with water and extracted with ether. The organic extract was dried and evaporated. Chromatography on silica eluting with ethyl acetate afforded the title compound as a clear oil ( 0.49g, 79%).

### b) Title compound.

The protected guanidine (Example 78a) (0.48g, 0.7 mmol) was dissolved in 1:1 trifluoroacetic acid:dichloromethane (20ml) containing phenol (0.7g, 0.7 mmol). After 6h the mixture was evaporated to dryness and chromatographed on silica eluting with aqueous ammonia:methanol:dichloromethane (1.5:15:85) affording the title compound as an oil (0.095g, 27%).
E.I. M⁺H 496, C₃₀H₄₉N₅O requires 495.

### Example 79 [3R, 4R]-3-Ethyl-1-heptyl-4-[3-(6-(piperidine-4-yl)methoxyquinolin-4-yl) propyl]piperidine

### a) 1-Benzyloxycarbonyl-4-hydroxymethyl-piperidine.

A solution of 4-hydroxymethyl-piperidine (1.0g, 8.7 mmol) in dichloromethane (20ml) was treated triethylamine (1.3ml, 9.6 mmol) then benzylchloroformate ( 1.4ml, 9.6 mmol). After 14h the reaction mixture was diluted with dichloromethane and washed with dilute aqueous sodium bicarbonate solution. The organic extract was dried and evaporated. Chromatography on silica eluting with 1:1 ethyl acetate:hexane afforded the product as a clear oil ( 0.97g, 44%).

### b) 1-Benzyloxycarbonyl-4-iodomethyl-piperidine.

A solution of triphenylphosphine (1.4g, 5.1 mmol), imidazole ( 0.35g, 5.1 mmol) and iodine (1,3g, 5.1 mmol) in dichloromethane (15ml) was stirred at room temperature for 0.5h then a solution of Example 79a ( 0.96g, 3.9 mmol) in dichloromethane (4ml) was added. After 3h the mixture was centifuged and the supernatant washed with dilute aqueous sodium sulphite. The dichloromethane was dried and evaporated. Chromatography on silica eluting with an ethyl acetate -hexane gradient afforded the title compound as a yellow oil ( 1.2g, 85%)

### c) [3R,4R]-3-Ethyl-1-heptyl-4[3-(6-(1-benzyloxycarbonyl piperidine-4-yl) methoxyquinolin-4-yl) propyl] piperidine.

This was prepared from the above iodide (Example 79b) and Example 22 using the procedure of Reference Example 16.

### d) [3R, 4R]-3-Ethyl-l-heptyl-4-[3-(6-(piperidine-4-yl) methoxy quinolin-4-yl) propyl] piperidine.

The title compound was prepared by hydrogenolytic removal of the N-benzyloxycarbonyl protecting group from Example 79c ( 0.06g, 0.09 mmol) by the procedure of Example 34b with the variation that the reaction time was 4h. Filtration, evaporating, and chromatography on silica eluting with aqueous ammonia-methanol-dichloromethane ( 0.5-5-95) afforded the title product as a clear oil (8 mg, 17%).
E.I. M⁺H 494, C₂₃H₅₁N₃O requires 493.

### Example 80. [3R, 4S]-1-Heptyl-3-vinyl 4-[3-(6-methoxyquinolin-4-yl)-(R,R)-oxiran-2-ylmethyl]piperidine

### (a) N-Heptylquininium bromide

Quinine (3.24g,10mmol) in benzene (10ml) and ethanol (2ml) was heated at 80° for 3 days with 1 -bromoheptane (1.57ml, 10mmol). The solvent was removed and the residue triturated with hexane to give the quarternary salt; MH⁺ 423.

### (b) Title compound

The product from Example 80a in benzene (10ml) was stirred vigorously overnight with 40% sodium hydroxide (10ml). The mixture was diluted with ethyl acetate, washed with water, brine, dried over magnesium sulfate and purified on silica gel eluting with methanol-ethyl acetate mixtures to give the title compound (0.79g, 19%),
MH⁺ 423.

### Example 81. [3R, 4S]-1-Heptyl-4-[(2S)-hydroxy-3-(6-methoxyquinolin-4-yl)propyl]-3-vinylpiperidine

The product of Example 80b (0.10g, 0.24mmol) in ether (15ml) was treated with lithium aluminium hydride (0.027g, 3 equiv) and stirred for 4h. The reaction was quenched with dilute sodium hydroxide, filtered and evaporated. Purification on silica gel eluting with methanol-chloroform-ammonia mixtures gave the title compound (0.110g, 64%), MH⁺ 425.

### Example 82. [3R, 4S]-1-Heptyl-3-vinyl-4-[3-(6-methoxyquinolin-4-yl)-(S,S)-oxiran-2-yl-methyl]piperidine

### (a) N-Heptylquinidinium bromide

Quinidine (10g, 31mmol) was suspended in toluene (30ml) and ethanol (5ml). 1-Bromoheptane (4.84ml, 31mmol) was added and the mixture heated at 80° overnight. After cooling the solvent was evaporated and the residue triturated with hexane to give the quartemary salt.

### (b) Title compound.

The product from Example 82a in t-butanol (150ml) was treated with potassium t-butoxide (10g, 3 equiv) and heated at 80° for 1h. The mixture was cooled, evaporated and the product purified on silica gel eluting with ethyl acetate to give the title compound (7.27g, 55%) MH⁺ 423.

### Example 83. [3R, 4S]-3-Ethyt-1-heptyl-4-[2-(S)-hydroxy-3-(6-methoxyquinolin-4-yl)propyl]piperidine

The product from Example 80b (0.115g, 0.27 mmol) in ethanol (15ml) was hydrogenated over 10% palladium on charcoal for 2 days. The mixture was filtered and evaporated to give the title compound (0.061g, 53%) MH⁺427.

### Example 84. [3R, 4S]-1-Heptyl-4-[N-methyl-N-(6-methoxyquinolin-4-yl)aminoethyl]-3-vinylpiperidine

### (a) [3R, 4S]-1-Heptyl-4-(methylaminocarboxymethyl)-3-vinylpiperidine

Example 51b in ethyl acetate (50ml) was treated with 4-methylmorpholine (1.44 ml, 13.1 mmol) and isobutylchloroformate (1.8 ml, 13,9 mmol) . After stirring for 1h the mixture was filtered and cooled in ice. Methylamine gas was bubbled through the mixture for 0.5h and the mixture stirred for a further 3h, then filtered, evaporated and purified on silica eluting with ethyl acetate to give a golden oil. Treatment of the oil with trifluoroacetic acid (9 ml) in dichloromethane (15 ml) for 1h, followed by evaporation and treatment with 1-iodoheptane (2.07 ml, 13.2 mmol) in dimethylformamide (15ml) in the presence of potassium carbonate (2.92g, 20.8 mmol) gave the title compound (2.61g), νₘₐₓ (film) 1647, 1558, and 1466 cm⁻¹.

### (b) [3R, 4S]- 1 Heptyl-4-(2-methylaminoethyl)-3-vinyl piperidine

The product from Example 84a (0.56g, 2.0 mmol) in tetrahydrofuran (15ml) was treated with lithium aluminium hydride (0.23g, 3 equiv) and heated at reflux for 3h. After allowing to cool the reaction was quenched with dilute sodium hydroxide, filtered through celite, and evaporated to give an oil (0.25g. 50%) MH⁺ 267.

### (c) Title compound.

The product from Example 84h (0.24g, 0.9 mmol) was heated with 4-chloro-6-methoxyquinoline (0.09g, 0.45 mmol) at 160 C for 18h. The mixture was cooled. diluted with dichloromethane, washed with sodium carbonate solution, dried and evaporated. Purification on silica gel eluting with methanol-chloroform-ammonia (5:95:0.5) gave a brown oil (0.172g, 88%), MH⁺424

### Example 85. [3R,4R]-1-Heptyl-3-(1-(R,S)-hydroxyethyl)-4-[3-(6-methoxyquinolin-4-yl)propyl]piperidine

### (a) [3R,4R]-3-(1-(R,S)-Hydroxyethyl)-4-[3-(6-methoxyquinolin-4-yl)propyl]piperidine

A solution of [3R, 4R]-1-Benzyloxycarbonyl-3-(2-(R, S)-oxiranyl)-4-[3-(6-methoxyquinolin-4-yl) propyl] piperidine (Example 73b) (3g, ca 1.5g, ca 3.3 mmol - balance is triphenylphosphine oxide) in ethanol (100 ml) was hydrogenated over 10% palladium on charcol (3g) for 4h. Filtration and evaporation gave the crude product as a brown oil (3g).

### (b) [3R,4R]-1-Heptyl-3-(1-(R,S)-hydroxyethyl)-4-[3-(6-methoxyquinolin-4-yl)propyl]piperidine

A solution of the crude product of Example 85a(1.5g) was dissolved in N,N-dimethylformamide (3 ml) and treated with potassium carbonate (0.28g, 2.0 mmol) then heptyliodide ((0.32 ml, 0.45g, 2.0 mmol). After 3h the mixture was evaporated to dryness then partitioned between ethyl acetate and water. The organic extract was dried and evaporated. Chromatography on silica eluting with aqueous ammonia:methanol:dichloromethane (0.2:2:100) afforded the individual diastereomers (27 mg, 8%) and (55 mg, 16% - yields over two stages). This latter diastereomer is the same compound as Example 34, prepared by different chemistry.
E.I. MH+ 427, C₂₇H₄₂N₂O₂ requires 426.

### Example 86. [3R,4R]-1-Heptyl-3-(1-(R,S)-hydroxy-1-methylethyl)-4-[3-(6-methoxyquinolin-4-yl)propyl]piperidine

### (a) [3R,4R]-1-Benzyloxycarbonyl-3-(1-acetyl)-4-[3-(6-methoxyquinolin-4-yl)propyl]piperidine

A solution of [3R,4R]-1-benzyloxycarbonyl-3-(1-hydroxyethyl)-4-[3-(6-methoxyquinolin-4-yl)propyl]piperidine (Example 34a) (0.4g, 0.9 mmol) in dichloromethane (10 ml) was treated with 4 A molecular sieves, tetrapropylammonium perruthenate (60 mg) and then N-methylmorpholine N-oxide (0.2g). After 1h, the mixture was diluted with ethyl acetate and filtered through celite. Evaporation and chromatography on silica eluting with aqueous ammonia:methanol:dichloromethane (1.5:15:100) gave the product as an oil (0.382g, 96%).
E.I. MH+ 461. C₂₈H₃₂N₂O₄ requires 460.

### (h) [3R,4R]-1-Benzyloxycarbonyl-3-(1-R,S)-1-hydroxy-1-methylethyl)-4-[3-(6-methoxyquinolin-4-yl)propyl]piperidine

A solution of the product of Example 86a (0.12g, 0.26 mmol) in tetrahydrofuran (2 ml) at -78⁰ C under argon was treated with a solution of methylmagnesium bromide in tetrahydrofuran (3M; 0.1 ml, 0.3 mmol) and the mixture allowed to warm to room temperature over 4h. The solution was re-cooled to -78⁰ C and an equivalent portion of methylmagnesium bromide was added. The mixture was allowed to warm to room temperature over 12h then partitioned between ethyl acetate and dilute aqueous sodium bicarbonate solution. The organic extract was dried and evaporated. Chromatography on silica gave the title compound as an oil (52mg, 42%).
E.I. MH+ 477, C ₂₉H₃₆N₂O₄ requires 476.

### (c) [3R,4R]- 3-(1-(R,S)-1-hydroxy-1-methylethyl)-4-[3-(6-methoxyquinolin-4-yl)propyl]piperidine

The title compound was prepared from the compound of Example 86b in 59% yield by hydrogenation in ethanol over palladium on charcol for 2.5h.
E.I. MH+ 343, C₂₁H₃₀N₂O₂ requires 342.

### (d) [3R,4R]-1-Heptyl-3-(1-(R,S)-hydroxy-1-methylethyl)-4-[3-(6-methoxyquinolin-4-yl)propyl]piperidine

The title compound (6mg) was prepared in 25% yield from the above [3R,4R]- 3-(1-(R,S)-1-hydroxy-1-methylethyt)-4-[3-(6-methoxyquinolin-4-yl)propyl]piperidine by N-heptylation in N,N-dimethylformamide at room temperature for 4.5h with potassium carbonate (1.2 equivalents) and heptyliodide (1.1 equivalents), followed by workup and chromatography.
E.I. MH+ 441, C₂₈H₄₄N₂O₂ requires 440.

### Example 87 [3R,4R]-1-Heptyl-3-hydroxymethyl-4-[3-(6-methoxyquinolin-4-yl)propyl]piperidine.

### (a) [3R,4R]-1-Benzyloxycarbonyl-3-formyl-4-[3-(6-methoxyquinolin-4-yl)propyl]piperidine

A solution of Example 73a (3.0g. 6.3 mmol)was dissolved in methanol:water (75 ml:25 ml) at 5° C then treated with sodium periodate (4g, 18.8 mmol).After 4h the mixture was concentrated. treated with ethyl acetate, filtered through celite and evaporated. Chromatography on silica eluting with a 0 to 2% methanol in dichloromethane gradient gave the title compound as an oil (2.2g. 80%).

E.I. MH+ 447, C₂₇H₃₀N₂O₄ requires 446

### (b) [3R,4R]-1-Benzyloxycarbonyl-3-hydroxymethyl-4-[3-(6-methoxyquinolin-4-yl)propyl]piperidine

A solution of Example 87a (1g, 2.2 mmol) in methanol (12 ml) was treated at 0° C with sodium borohydride (60mg, 1.6 mmol). After 1h the mixture was evaporated and the residue partitioned between half-saturated aqueous sodium bicarbonate solution and ethyl acetate. The organic extract was dried and evaporated . The crude product was chromatographed on silica eluting with a 0 to 2% gradient of methanol in dichloromethane affording the title compound as an oil (0.62g, 62%).

E.I. MH+ 449, C₂₇H₃₂N₂O₂ requires 448.

### (c) [3R,4R] -3-Hydroxymethyl-4-[3-(6-methoxyquinolin-4-yl)propyl]pipendine

This was prepared by hydrogenation of Example 87baccording to the standard procedure as for Example 43, in approximately 90% crude yield.

E.I. MH⁺ 315, C19H26N2O2 requires 314

### (d) Title compound

This was prepared by N-heptylation of Example 87c according to the standard procedure of Example 73e, giving after chromatography on silica eluting with aqueous ammonia:ethanol:chloroform (1.5:15:500) the title compound as an oil (0.36g, 73%).

E.I MH+ 413, C₂₆H₄₀N₂O₂ requires 412.

### Example 88 [3R,4R]-1-(6-Methylheptyl)-3-(1-(R) and 1-(S),2-dihydroxyethyl)-4-[3-(6-methoxyquinolin-4-yl)propyl)piperidine.

These were prepared as the individual diastereomers (faster running diastereomer, 110mg: slower running diastereomer 100mg:combined yield, 22%) by the same method as Example 75 from Example 73a using 6-methylheptylbromide as alkylating agent.
E.I M⁺H 457, C₂₈H₄₄N₂O₃ requires 456.

### Example 89 [3R, 4S]-1-Heptyl-4-((2S)-hydroxy-3-(6-methoxyquinolin-4-yl)propyl]-3-(2-hydroxyethyl)piperidine

[3R, 4S]-1-Heptyl-4-[(2S)-hydroxy-3-( 6-methoxyquinolin-4-yl)propyl]-3-vinylpiperidine (Example 81) (0.59g. 1.39) mmol)in tetrahydrofuran (20 ml) was treated with 5M 9-borabicyclo[3.3.1]nonane (8.3 ml. 4.17 mmol) and heated at reflux overnight. The mixture was allowed to cool and was treated with ethanol (8 ml), 2M sodium hydroxide solution (4 ml), and 30% hydrogen peroxide (3 ml). After being stirred for 1 hour the mixture was decanted from an insoluble sludge and extracted with ethyl acetate to give the crude produce, which was purified on silica gel eluting with 15 % methanol in ethyl acetate to give a golden oil (0.235g, 38%). MH⁺ 443.

### Example 90 [3R, 4S]-1-Heptyl-3-aminocarbonyloxymethyl-4-[3-(6-methoxyquinolin-4- yl)propyl]piperdine

A solution of [3R, 4S]-1-heptyl-3-hydroxymethyl-4-[3-(6- methoxyquinolin-4-yl)propyl]piperdine (Example 87), (0.2g, 0.49 mmol) in dichloromethane (3.5 ml) was treated with a solution of trichloroacetyl isocyanate (0.1g. 0.55 mmol) in dichloromethane (1 ml) at 0° C. After 18h at room temperature the mixture was treated with a solution of potassium carbonate (0.23g) in methanol:water (1 ml:2 ml) and the mixture was stirred at room temperature for 0.5h. More dichloromethane and water were added, and the organic extract was dried and evaporated. The crude product was purified by chromatography on silica eluting with a gradient of 880 ammonia:methanol:dichloromethane giving the title compound as a clear oil (0.046g, 21%).
E.I. MH+ 456 C27H41N3O3 requires 455.

### Example 91 [3R, 4R]-1-Heptyl-4-[3-(6-methoxyquinolin-4-yl)propyl]-3-(2-carbamoylethyl)piperidine oxalate

### a) (3R, 4R)-3-(2-Hydroxyethyl)-4-[3-(6-methoxyquinolin-4-yl) propyl] piperidine.

This was prepared in approximately quantitative yield from the N-benzyloxycarbonyl piperidine (Example 33b) by hydrogenation in ethanol over 10% palladium on charcoal.
E.I. MH⁺ 329, C₂₀H₂₈N₂O₂ requires 328.

### b) (3R, 4R)-1-t-Butyloxycarbonyl-3-(2-hydroxyethyl)A-[3-(&methoxyquinolin-4-yl) propyl] piperidine.

The above piperidine (Example 91a) was dissolved in dichloromethane-N,N-dimethylformamide and treated with triethylamine (1.2 eq), di-*t*-butylcarbonic anhydride (1.1 equivalents ) and N,N-dimethylammopyridine (catalytic quantity). After stirring overnight the mixture was evaporated and purified by chromatography on silica eluting with a gradient of ethyl acetate/hexane, giving the product as an oil ( 3.8g. 34%)
E.I MH+ 429, 329 (loss of CO₂C₄H₉), C₂₅H₃₆N₂O₄ requires 428.

### c) (3R,4R)-1-t-Butyloxycarbonyl-3-[2-(4-methylphenyl)sulfonyloxyethyl]-4-[3-(6-methoxyquinolin-4-yl) propyl] piperidine

The above alcohol (Example 91b) (2.2g, 5.1 mmol) was dissolved in dichloromethane (50ml), then triethylamine (0.85ml, 0.62g, 6.1 mmol), N,N'-dimethylaminopyridine (catalytic) and 4-methylphenylsulfonyl chloride (1.1 g, 5.6 mmol) were added. After 20h the mixture was diluted with more dichloromethane and washed with water. The organic extract was dried (Na₂S0₄) and evaporated.
Chromatography on silica eluting with ethyl acetate:hexane (1:1) afforded the product as a yellow oil ( 1.8g, 61%).
E.I. MH+ 583, C₃₂H₄2N₂0₆S requires 582.

### d) (3R, 4R)-1- t-Butoxycarbonyl-3-(2-cyanoethyl)-4-[3-(6-methoxyquinolin-4-yl) propyl] piperidine.

The above tosylate (Example 91c) (1.8g, 31mmol) was dissolved in N,N-dimethylformamide (15ml) and treated with sodium cyanide ( 0.3g, 6.2 mmol). The mixture was stirred at room temperature for 16 h then at 40° for 1h. The mixture was evaporated to dryness and the residue was partitioned between ethyl acetate and water. The organic extract was dried (MgSO₄) and evaporated to give the product as an oil, (67%).
E.I. MH+ 438, C₂₆H₃₅N₃O₃ requires 437.

### e) (3R, 4R)-3-(2-Cyanoethyl)-4-[3-(6-methoxyquinolin-4-yl) propyl] piperidine.

The above nitrile (Example 91d) (0.9g) was treated with 1:1 trifluoroacetic acid:dichloromethane (25 ml)at 0°C. After 1h the reaction mixture was evaporated and the residue partitioned between ethyl acetate and saturated aqueous sodium bicarbonate solution.. The organic extract was dried and evaporated to give the title compound as an oil in approximately quantitative yield.
E.I MH⁺ 338, C₂₁H₂₇N₂O₂ requires 337.

### f) (3R, 4R)-1-Heptyl-3-(2-cyanoethyl)-4-[3-(6-methoxyquinolin-4-yl) propyl] piperidine.

The title compound was prepared from the above piperidine (Example 91e) by heptylation at room temperature, using the procedure of Example 12b, giving the purified product as an oil (0.55g, 62%).
E.I. MH+ 436, C₂₈H₁₄N₃0 requires 435.

### g) [3R, 4R]-1-Heptyl-3-(3-carboxyethyl)-4-[3-(6-methoxyquinolin-4-yl) propyl] piperidine.

Hydrolysis of the corresponding cyanoethyl compound (Example 91f) (0.35g, 0.8mmol) with concentrated hydrochloric acid and dioxane (9ml of each) at reflux for 11h followed by evaporation and chromatography on silica (eluting with 1.5:15:50 aqueous ammonia:methanol:chloroform afforded the title compound (0.23g, 55% as an oil.
E.I. MH⁺, 455, C₂₈H₄₂N₂O₂ requires 454.

### h) Title compound

The above carboxylic acid (Example 91g) in dichloromethane was treated with 1-(3-dimethylaminopropyl)-3-ethylcarbodiimide hydrochloride (0.051g, 0.26 mmol) and concentrated ammonia solution (1 ml). The mixture was agitated overnight then reduced in volume and purified on silica gel eluting with 20% methanol in ethyl acetate to give the title compound. This was converted to the oxalate salt by treatment with 0.1M oxalic acid in ether. MH⁺ 454.

### Table 1

The following compounds of the prior art are prepared by processes analogous to those described in the Examples

### Table 2

The following novel compounds were prepared by processes analogous to those described in the Examples

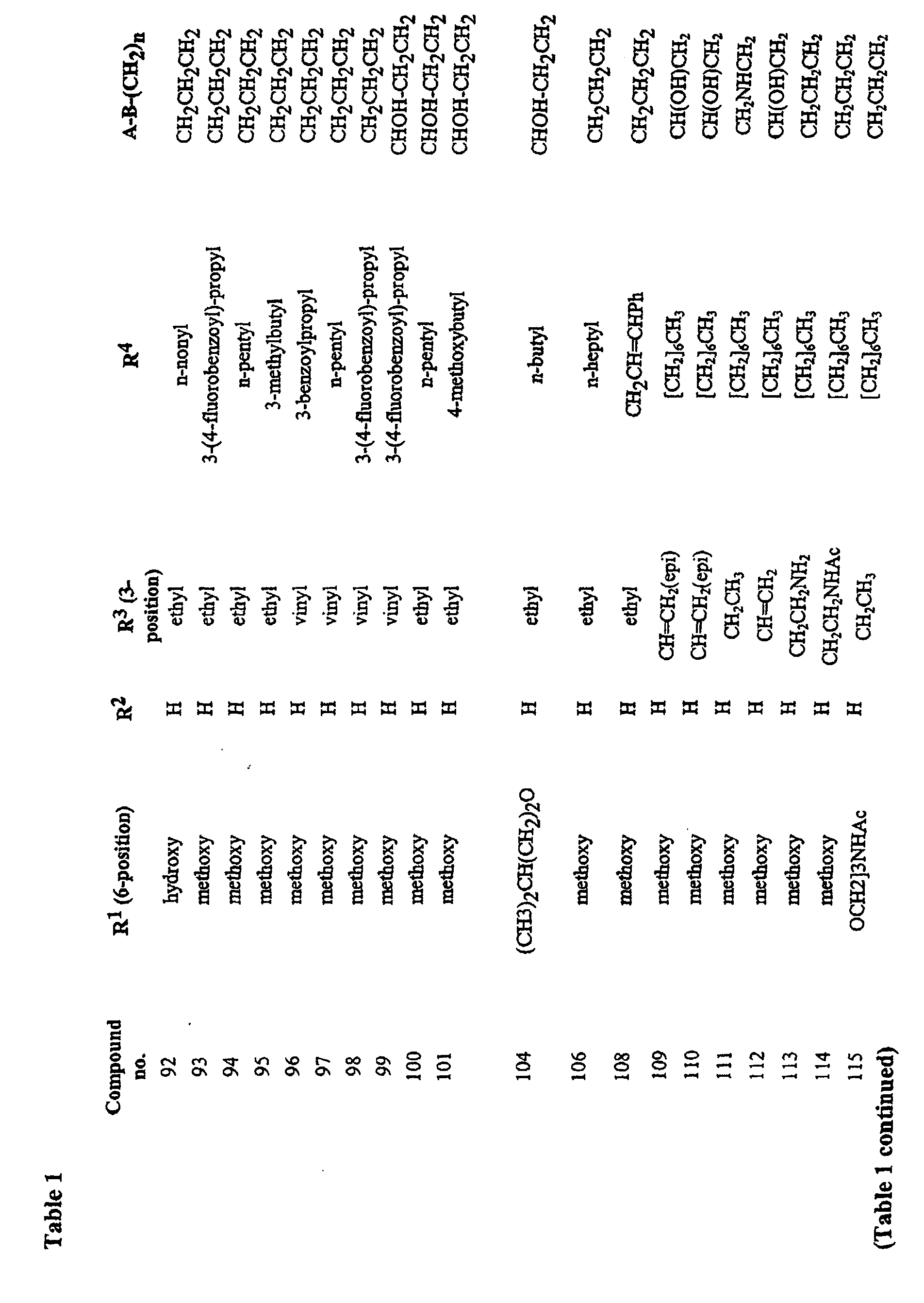

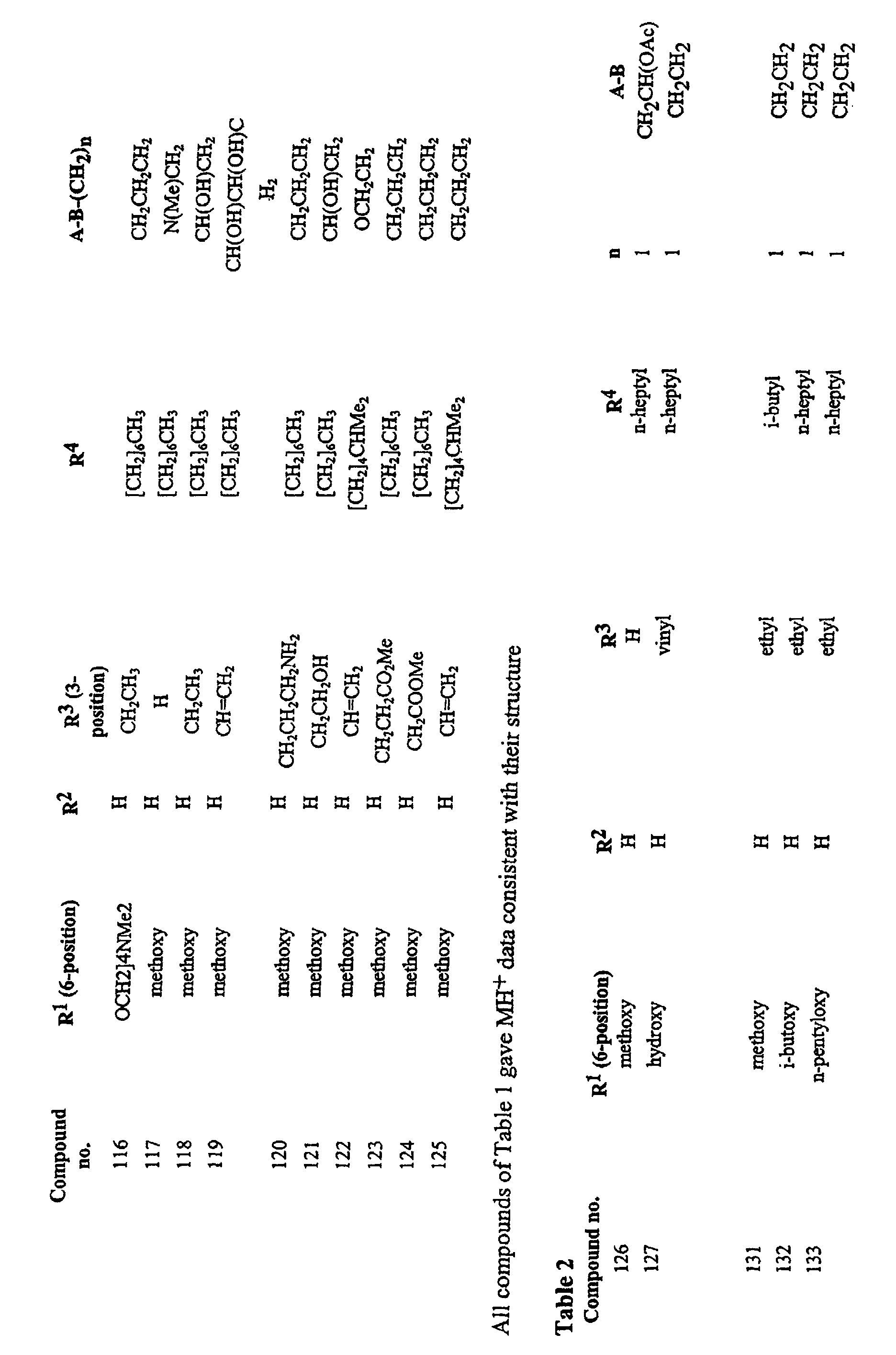

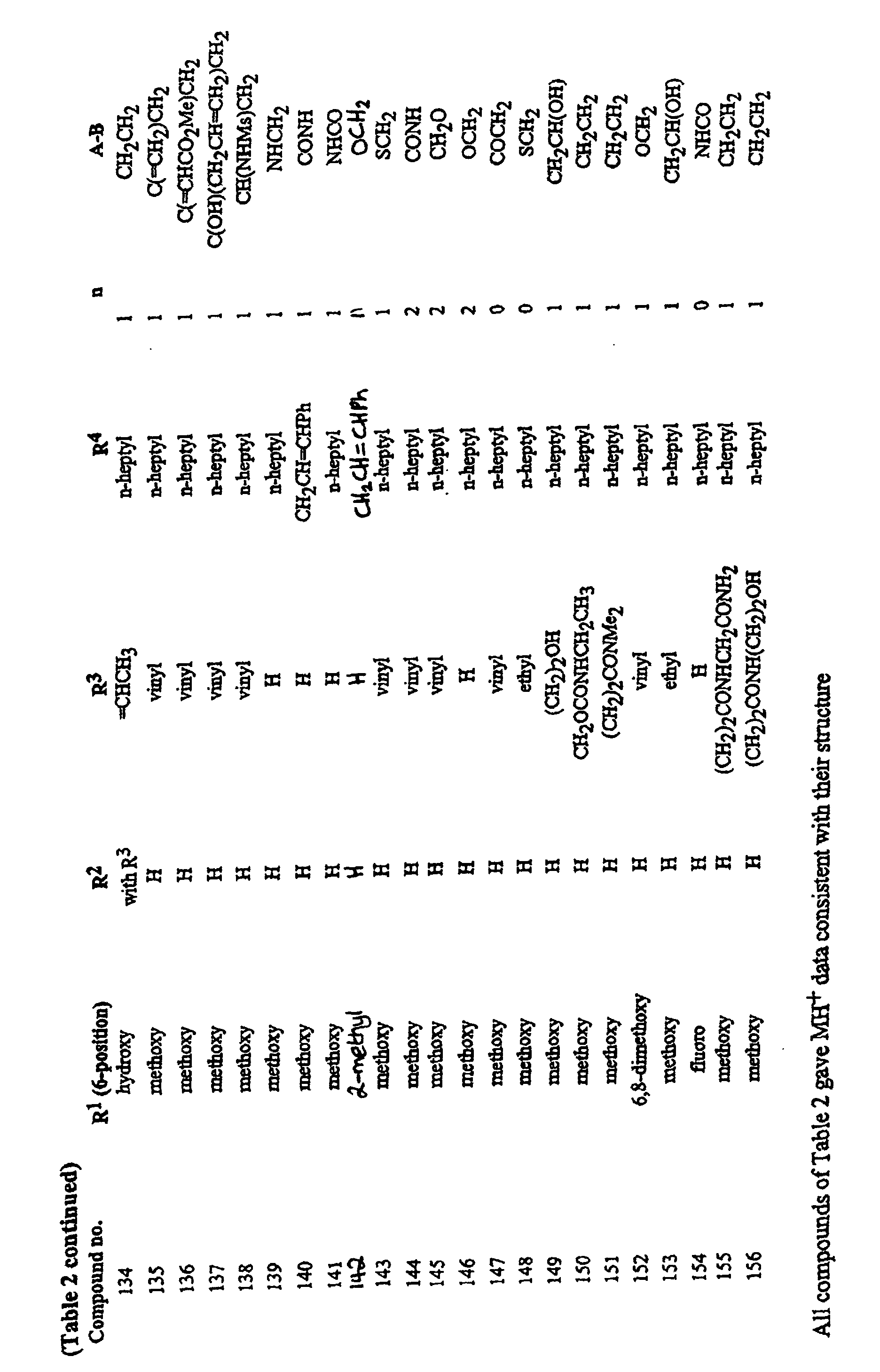

### Biological Activity

The MIC (µg/ml) of compounds E2 and 105 against various organisms was determined and compared with Vancomycin.

Example 2 was tested as a 1:1 mixture of its isomers about the CH(OH) unit and separately as isomer (I) and isomer (II).

| Organism | Vancomycin | Example 2 | | |
|---|---|---|---|---|
| | | (1:1) | (1) | (II) |
| E. coli ESS | >64 | 4 | 8 | 2 |
| Staph. aureus "south" | 2 | 0.25 | 2 | 0.125 |
| Staph. aureus V573 | 1 | 0.25 | 2 | 0.125 |
| Staph. aureus Russell | 2 | 0.5 | 2 | 0.125 |
| Staph. epidermis 11047 | 2 | 2 | 4 | 1 |
| B.subtilis 6633 | 0.5 | 64 | >64 | 64 |
| Strep. faecalis I | 2 | 4 | 4 | 2 |

Examples 4, 6, 8, 10, 11, 12, 15,25, 31, 32, 33, 34, 36, 41, 42, 43, 44, 46, 47, 48, 50, 60, 63, 64, 65,66, 73, 74, 75, 76, 78, and 80 to 91 and compound 155 of Table 2 have MIC of less than or equal to 1µg/ml against one or more of a range of gram positive and gram negative bacteria.

The remaining compounds E21 to E79 showed an MIC of less than or equal to 16µg/ml against one or more of a range of gram positive and gram negative bacteria

The following compounds showed an MIC of less than 100 µg/ml against certain of the organisms listed above:
E2, E3, E5, E7, E9, E13, E14, E17, E18, E20, and compounds 92-101 and 104-125 of Table 1.

## Claims

1. The use of a compound of formula (I) in the free base form or a pharmaceutically acceptable acid addition or quaternary ammonium salt or N-oxide thereof 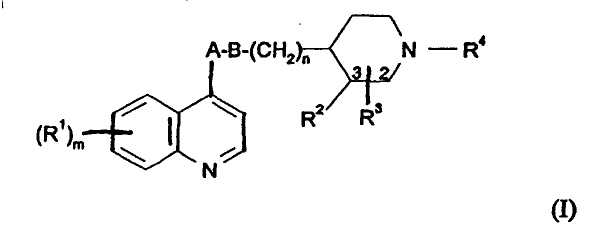 wherein:
m is 1 or 2 ;
each R¹ is independently hydroxy; (C₁₋₆) alkoxy optionally substituted by (C₁₋₆)alkoxy, amino, piperidyl, guanidino or amidino optionally N-substituted by one or two (C₁₋₆)alkyl, acyl or (C₁₋₆)alkylsulphonyl groups, NH₂CO, hydroxy, thiol, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆)alkyl; halogen; (C₁₋₆)alkyl; (C₁₋₆)alkylthio; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups; either R² is hydrogen; and
R³ is in the 2- or 3-position and is hydrogen or (C₁₋₆)alkyl or (C₂₋₆)alkenyl optionally substituted with 1 to 3 groups selected from:
thiol; halogen; (C₁₋₆)alkylthio; trifluoromethyl; azido; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; aminocarbonyl wherein the amino group is optionally substituted by (C₁₋₆)alkyl, hydroxy(C₁₋₆)alkyl, aminocabonyl(C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; oxo; (C₁₋₆)alkylsulphonyl; (C₂₋₆)alkenylsulphonyl; or aminosulphonyl wherein the amino group is optionally substituted by (C₁₋₆)alkyl or (C₂₋₆)alkenyl; or
R³ is in the 3-position and R² and R³ together are a divalent residue =CR^{5¹}R^{6¹} where R^{5¹} and R^{6¹} are independently selected from H, (C₁₋₆)alkyl, (C₂₋₆)alkenyl, aryl(C₁₋₆)alkyl and aryl(C₂₋₆)alkenyl, any alkyl or alkenyl moiety being optionally substituted by 1 to 3 groups selected from those listed above for substituents on R³;
R⁴ is a group -CH₂-R⁵ in which R⁵ is selected from:
(C₃₋₁₂)alkyl; hydroxy(C₃₋₁₂)alkyl; (C₁₋₁₂)alkoxy(C₃₋₁₂)alkyl; (C₁₋₁₂)alkanoyloxy(C₃₋₁₂)alkyl; (C₃₋₆)cycloalkyl(C₃₋₁₂)alkyl; hydroxy-, (C₁₋₁₂)alkoxy- or (C₁₋₁₂)alkanoyloxy-(C₃₋₆)cycloalkyl(C₃₋₁₂)alkyl; cyano(C₃₋₁₂)alkyl; (C₂₋₁₂)alkenyl; (C₂₋₁₂)alkynyl; tetrahydrofuryl; mono- or di-(C₁₋₁₂)alkylamino(C₃₋₁₂)alkyl; acylamino(C₃₋₁₂)alkyl; (C₁₋₁₂)alkyl- or acyl-aminocarbonyl(C₃₋₁₂)alkyl; mono- or di-(C₁₋₁₂)alkylammo(hydroxy) (C₃₋₁₂)alkyl; optionally substituted phenyl(C₁₋₂)alkyl, phenoxy(C₁₋₂)alkyl or phenyl(hydroxy)(C₁₋₂)alkyl; optionally substituted diphenyl(C₁₋₂)alkyl; optionally substituted phenyl(C₂₋₃)alkenyl; optionally substituted benzoyl or benzoylmethyl; optionally substituted heteroaryl(C₁₋₂)alkyl;and optionally substituted heteroaroyl or heteroaroylmethyl;
or R⁴ is 3-benzoylpropyl or 3-(4-fluorobenzoyl)propyl;
n is 0, 1 or 2 ;
A is NR¹¹, O, S(O)ₓ or CR⁶R⁷ and B is NR¹¹,O, S(O)ₓ or CR⁸R⁹ where x is 0, 1 or 2 and wherein:
each of R⁶, R⁷, R⁸ and R⁹ is independently selected from: H; thiol; (C₁₋₆)alkylthio; halo; trifluoromethyl; azido; (C₁₋₆)alkyl; (C₂₋₆)alkenyl; (C₁₋₆)alkoxycarbonyl; (C₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₁₋₆)alkenyl;
or R⁶ and R⁸ together represent a bond and R⁷ and R⁹ are as above defined;
or R⁶ and R⁸ together represent -O- and R⁷ and R⁹ are both hydrogen;
or R⁶ and R⁷ or R⁸ and R⁹ together represent oxo;
and each R¹¹ is independently H, trifluoromethyl, (C₁₋₆)alkyl, (C₁₋₆)alkenyl, (C₁₋₆)alkoxycarbonyl, (C₁₋₆)alkylcarbonyl, aminocarbonyl wherein the amino group is optionally substituted by (C₁₋₆)alkoxycarbonyl, (C₁₋₆)alkylcarbonyl, (C₁₋₆)alkenyloxycarbonyl, (C₂₋₆)alkenylcarbonyl, (C₁₋₆)alkyl or (C₁₋₆)alkenyl and optionally further substituted by (C₁₋₆)alkyl or (C₁₋₆)alkenyl;
wherein:
'heterocyclic' as used herein is an aromatic or non-aromatic, single or fused, ring containing up to four hetero-atoms in each ring selected from oxygen, nitrogen and sulphur, and having from 4 to 7 ring atoms which rings may be unsubstituted or substituted by up to three groups selected from amino, halogen, (C₁₋₆)aIkyl, (C₁₋ ₆)alkoxy, halo(C₁₋₆)alkyl, hydroxy, carboxy, carboxy salts, (C₁₋₆)alkoxycarbonyl, (C₁₋₆)alkoxycarbonyl(C₁₋₆)alkyl, aryl, and oxo groups, and wherein any amino group forming part of a single or fused non-aromatic heterocyclic ring as defined is optionally substituted by (C₁₋₆)alkyl optionally substituted by hydroxy, (C₁₋₆)alkoxy, thiol, (C₁₋₆)alkylthio, halo, triftuommethyl, acyl or (C₁₋₆)alkylsulphonyl;
'heteroaryl' is an aromatic heterocyclic group referred to above;
'aryl' is phenyl or naphthyl, each optionally substituted with up to five groups selected from halogen, mercapto, (C₁₋₆)alkyl, phenyl, (C₁₋₆)alkoxy, hydroxy(C₁₋₆)alkyl, mercapto (C₁₋₆)alkyl, halo(C₁₋₆)alkyl, hydroxy, amino, nitro, carboxy, (C₁₋₆)alkylcarbonyloxy, (C₁₋₆)alkoxycarbonyl, formyl, or (C₁₋₆)alkylcarbonyl groups;
'acyl' is an (C₁₋₆)alkoxycarbonyl, formyl or (C₁₋₆) alkylcarbonyl group;
provided that A and B cannot both be selected from NR¹¹, O and S(O)ₓ and when one of A and B is CO the other is not CO, O or S(O)ₓ, in the manufacture of a medicament for use in the treatment of bacterial infections in mammals.

2. A compound of formula (IA) which is a compound of formula (I) as defined in claim 1 wherein R³ is hydroxy(C₁₋₆)alkyl or 1,2-dihydroxy(C₂₋₆)alkyl optionally substituted on the hydroxy group(s).

3. A compound of formula (IB) which is a compound of formula (I) as defined in claim 1 wherein at least one R¹ is (C₂₋₆) alkoxy substituted by optionally N-substituted amino, guanidino or amidino or(C₁₋₆)alkoxy substituted by piperidyl, A is CH₂, CHOH, CH(NH₂), C(Me)(OH) or CH(Me) and B is CH₂, CHOH or CO.

4. A use according to claim 1 wherein R¹ is in the 6-position on the quinoline nucleus and is methoxy, amino(C₃₋₅)alkyloxy, nitro or fluoro and m is 1.

5. A use according to claim 1 or 4 wherein R³ is (C₁₋₆) alkyl, (C₁₋₆) alkenyl, optionally substituted 1-hydroxy-(C₁₋₆) alkyl

6. A use according to claim 5 wherein R³ is hydroxymethyl, 1- hydroxyethyl or 1,2-dihydroxyethyl wherein the 2-hydroxy group is optionally substituted with alkylcarbonyl or aminocarbonyl where the amino group is optionally substituted.

7. A use according to any one of claims 1 and 4 to 6 wherein R³ is in the 3-position.

8. A use according to any one of claims 1 and 4 to 7 wherein A is NH, NCH₃, O, CH₂, CHOH, CH(NH₂), C(Me)(OH) or CH(Me) and B is CH₂, CHOH, CO or S or A is CR⁶R⁷ and B CR⁸R⁹ and R⁶ and R⁸ together represent -O- and R⁷ and R⁹ are both hydrogen, and n is 0 or 1.

9. A use according to claim 8 wherein:
A is NH, B is CO and n is 1 or 0;
A is O, B is CH₂ and n is 1 or 0;
A is CH₂ or CH₂OH, B is CH₂, and n is 1 or 0;
A is NCH₃, CH(NH₂), C(Me)(OH) or CH(Me), B is CH₂ and n is 1
A is CR⁶R⁷ and B CR⁸R⁹ and R⁶ and R⁸ together represent -O- and R⁷ and R⁹ are both hydrogen and n is 1.

10. A use according to any one of claims 1 and 4 to 9 wherein R⁴ is (C₅₋₁₀)alkyl, unsubstituted phenyl(C₂₋₃)alkyl or unsubstituted phenyl(C₃₋₄)alkenyl.

11. A use according to any one of claims 1 and 4 to 10 wherein R⁵ is unbranched at the α and, where appropriate, β positions.

12. A compound of formula (I) as defined in claim 1 selected from:
[3R,4R]-3-Ethyl-1-hexyl-4-[3-oxo-3-(6-methoxyquinolin-4-yl)propyl]piperidine;
[3R,4R]-3-Ethyl-1-hexyl-4-[3-(R,S)-hydroxy-3-(6-methoxyquinolin-4-yl)propyl]piperidine;
[3R,4R]-3-Ethyl-1-heptyl-4-[3-oxo-3-(6-methoxyquinolin-4-yl)propyl]piperidine;
[3R,4R]-3-Ethyl-1-heptyl-4-[3-(R,S)-hydroxy-3-(6-methoxyquinolin-4-yl)propyl]piperidine;
[3R,4R] 3-Ethyl-1-octyl-4-[3-oxo-3-(6-methoxyquinolin-4-yl)propyl]piperidine;
[3R,4R]-3-Ethyl-1-octyl-4-[3-(R,S)-hydroxy 3-(6-methoxyquinolin-4-yl)propyl]piperidine;
[3R,4R]-3-Ethyl-1-decyl-4-[3-oxo-3-(6-methoxyquinolin-4-yl)propyl]piperidine;
[3R,4R]-3-Ethyl-l-decyl-4-[3-(R,S)-hydroxy-3-(6-methoxyquinolin-4-yl)propyl]piperidine;
[3R,4R]-3-Ethyl-1-dodecyl-4-[3-oxo-3-(6-methoxyquinolin-4-yl)propyl]piperidine;
[3R,4R] 3-Ethyl-1-dodecyl-4-[3-(R,S)-hydroxy-3-(6-methoxyquinolin-4-yl)propyl]piperidine;
[3R,4R]-3-Ethyl-1-cinnamyl-4-[3-(R,S)-hydroxy 3-(6-methoxyquinolin-4-yl)propyl]piperidine;
[3R,4R]-3-Ethyl-1-heptyl-4-[3-(6-methoxyquinolin-4-yl)propyl]piperidine;
[3R,4R]-3-Ethenyl-1-heptyl-4-[3-(R,S)-hydroxy-3-(6-methoxyquinolin-4-yl)propyl]piperidine;
[3R,4R]-3-Ethyl-1-heptyl-4-[3-(R,S)hydroxy-3-(6-hydroxyquinolin-4-yl)propyl]piperidine;
[3R,4R]-1-Heptyl-3-(2-hydroxyethyl)-4-[3-(R,S)-hydroxy-3-(6-methoxyquinolin-4-yl)propyl]piperidine;
[3R,4R]-3-Ethyl-1-heptyl-4-[3-(R,S)-hydroxy-3-(6-[5-aminopentyloxy]-quinolin-4-yl)propyl]piperidine;
[3R,4R]-3-Ethyl-1-heptyl-4-[3-(R,S)-hydroxy-3-(6-[2-amino-2-oxo-1,1-dimethyl]ethoxyquinolin-4-yl)propyl]piperidine;
[3R,4R]-3-Ethyl-1-heptyl-4-[3-(R,S)-hydroxy-3-(6-aminoquinolin-4-yl)propyl]piperidine;
[3R,4R]-3-Ethyl-1-heptyl-4-[3-(R,S)-hydroxy-3-(6-azidoquinolin-4-yl)propyl]piperidine;
[3R,4R]-3-Ethyl-1-heptyl-4-[3-(6-hydroxyquinolin-4-yl)propyl]piperidine;
[3R,4R]-3-Ethyl-1-heptyl-4-[3-(6-propyloxyquinolin-4-yl)propyl]piperidine;
[3R,4R]-3-Ethyl-1-heptyl-4-[3-(6-(5-aminopentyloxy)-quinolin-4-yl)propyl]piperidine;
[3R,4R]-3-Ethenyl-1-(2-phenoxyethyl)-4-[3-(6-methoxyqiunolin-4-yl)propyl]piperidine;
[3S,4R]-3-Ethenyl-1-heptyl-4-[3-(6-methoxyquinolin-4-yl)propyl]piperidine;
[3R,4R]-3-Ethenyl-1-heptyl-4-[3-(6-methoxyquinolin-4-yl)propyl]piperidine;
[3R,4R]-1-Heptyl-3-(2-hydroxyethyl)-4-[3-(6-methoxyquinolin-4-yl)propyl]piperidine;
[3R,4R]-1-Heptyl-3-(2-acetoxyethyl)-4-[3-[6-methoxyquinolin-4-yl)propyl]piperidine;
[3R,4R]-1-Heptyl-3-(3-hydroxypropyl)-4-[3-(6-methoxyquinolin-4-yl)propyl]piperidine;
[3R,4R]-1-Heptyl-3-(1-hydroxyethyl)-4-[3-(6-methoxyquinolin-4-yl)propyl]piperidine;
[3R,4R]-3-Ethyl-1-(2-phenylethyl)4-[3-(R,S)-hydroxy-3-(6-methoxyquinolin-4-yl)propyl]piperidine;
[3R,4R]-3-Ethyl-1-(3-phenylpropyl)-4-[3-(R,S)-hydroxy-3-(6-methoxyquinolin-4-yl)propyl)piperidine;
Heptyl-4-[2-(R,S)-hydxoxy-3-(6-methoxyquinolin-4-yl)propyl]piperidine;
1-Heptyl-4-[3-(6-methoxyquinolin-4-yl)prop-2-enyl]piperidine;
1-Heptyl-4-[3-(6-methoxyquinolin-4-yl)propyl]piperidine;
[3R,4R]-3-Ethyl-1 -heptyl-4-[3-(R,S)-hydroxy-3-(6-methoxyquinolin-4-yl)butyl]piperidine;
[3R,4R]-3-Ethenyl-1-heptyl-4-[3-(R,S)-azido-3-(6-methoxyquinolin-4-yl)propyl]piperidine;
[3R,4R]-3-Ethenyl-1-heptyl-4-[3-(R,S)-amino-3-(6-methoxyquinolin-4-yl)propyl]piperidine;
[3R,4R]-3-Ethyl-1-heptyl-4-[3-(R,S)-amino-3-(6-methoxyquinolin-4-yl)propyl]piperidine;
[3R,4R]-3-Ethyl-1-heptyl-4-[3-(6-methoxyquinolin-4-yl)butyl]piperidine;
[3R,4R]-3-Ethenyl-1-heptyl-4-[3-(R,S)-acetamido-3-(6-methoxyquinolin-4-yl)propyl]piperidine;
[3R,4R]-1-Heptyl-3-(2-(R,S)-hydroxypropyl)-4-[3-(6-methoxyquinolin-4-yl)propyl]piperidine;
[3R,4R]-1-Heptyl-3-(1-(R,S),2-dihydroxyethyl)-4-[3-(6-methoxyquinolin-4-yl)propyl]piperidine ;
[3R,4R]-1 -Heptyl-3-aminocarbonyloxyethyl-4-[3-(6-melhoxyquinolin-4-yl)propyl]piperidine;
[3R,4R]-3-(1-(R,S),2-Dihydroxyethyl)-1-heptyl-4-[3-(R,S)-hydroxy-3-(6-methoxyquinolin-4-yl)propyl]piperidine;
[3*R*, 4*R*]-3-Ethyl-1-heptyl-4-[(6-methoxyquinolinyl-4-oxy)methyl]piperidine;
[3R,4S]-3-Ethenyl-1-heptyl-4-[2-(6-methoxyquinolin-4-yl)-oxyethyl]piperidine;
1-Heptyl-4-[(6-methoxyquinolin-4-yl)oxymethyl]piperidine;
[3R,4R]-3-Ethyl-1-heptyl[(6-methoxyquinolin-4-yl)methylthiomethyl]piperidine;
[3R,4R]-1-Heptyl-3-ethenyl-4-[{(6-methoxyquinoline-4-yl)carbonylamino}methyl]piperidine;
[3R,4R]-3-Ethenyl-1-heptyl-piperidine-4-[N-(6-methoxyquinolin-4-yl)]propionamide;
[3R,4R]-3-Ethenyl-1-heptyl-piperidine-4-[N-(6-methoxyquinolin-4-yl)]propylamine;
[3R,4S]-3-Ethenyl-1-heptyl-piperidin-4-[N-(6-methoxyquinolin-4-yl)]acetamide;
[3R,4R]-3-Ethenyl-1-heptyl-piperidine-4-[N-(6-methoxyquinolin-4-yl)]ethylamine;
[3R,4S]-3-Ethenyl-1-heptyl-4-[2-(R,S)-hydroxy 2-(6-methoxyquinolin-4-yl)ethyl]piperidine;
[3R,4R]-3-Ethenyl-1-heptyl-4-[2-(6-methoxyquinolin-4-yl)ethyl]piperidine;
[3R,4R]-3-Ethyl-1-heptyl-4-[2-(6-methoxyquinolin-4-yl)ethyl]piperidine;
1-Heptyl-4-[2(*R*,*S*)-hydroxy-2-(6-methoxy-4-quinolinyl)ethyl]-piperidine;
[3S,4R]-3-Ethenyl-1-heptyl-4-[2-(6-methoxyquinolin-4yl)ethyl]piperidine;
N-(6-Methoxy-4-quinolinyl)-1-heptyl-4-piperidinecarboxamide;
(3Z)-(4*R*)-3-Ethylidene-1-heptyl-4-[3-(6-methoxyquinolin-4-yl)propyl]piperidine;
[3R,4S]-1-Cinnamyl-4-[2-(6-methoxyquinolin-4-yl)-oxyethyl]piperidine;
[3R,4R]-3-(2-Acetoxyethyl)-1-heptyl-4-[3-(6-methoxy-quinolin-4-yl)propyl]piperidine;
[3R,4R]-3-Ethyl-1-heptyl-4-[3-(6-{2-hydroxyethyloxy}quinolin-4-yl)propyl]piperidine;
[3R,4R]-3-(Ethylaminocarbonyloxyethyl)-1-heptyl-4-[3-(6-methoxyquinolin-4-yl)propyl]piperidine;
[3R,4R]-3-Ethenyl-1-heptyl-4-[3-(R,S)-aminocarbonylamino-3-(6-methoxyquinolin-4-yl)propyl]piperidine;
[3R,4R]-3-Ethyl-1-heptyl-4-[3-(6-(4-aminobutyloxy)-quinolin-4-yl)propyl]piperidine;
[3R,4R]-1-Heptyl-3-(1-(R)- and 1-(S)-hydroxy-2-methoxyethyl)-4-[3-(6-methoxyquinolin-4-yl) propyl] piperidine;
[3R, 4R]-1-Heptyl-3-(1-(R)- and 1-(S)-hydroxy-2-methylthioethyl)-4-[3-(6-methoxyquinolin-4-yl) propyl]piperidine;
[3R, 4R]-1-(5-Methylhexyl)-3-(1-(R)- and 1-(S),2-dihydroxyethyl)-4-[3-(6-methoxyquinolin-4-yl)propyl]piperidine;
[3R, 4R]-3-Ethyl-1-heptyl-4-[3-(6-(3-aminopropyl)oxyquinolin-4-yl) propyl]piperidine;
[3R, 4R]-3-Ethyl-1-heptyl-4-[3-(6-(2-aminoethyl)oxyquinolin-4-yl) propyl]piperidine;
[3R, 4R]-3-Ethyl-1-heptyl-4-[3-(6-(3-guanidinopropyl)oxyquinolin-4-yl)propyl] piperidine;
[3R, 4R]-3-Ethyl-1-heptyl-4-[3-(6-(piperidine-4-yl)methoxyquinolin-4-yl) propyl]piperidine;
[3R, 4S]-1-Heptyl-3-vinyl-4-[3-(6-methoxyquinolin-4-yl)-(R-R)-oxiran-2-ylmethyl]piperidine;
[3R, 4S]-1Heptyl-4-[(2S)-hydroxy-3-(6-methoxyquinolin-4-yl)propyl]-3-vinylpiperidine;
[3R, 4S]-1-Heptyl-3-vinyl-4-[3-(6-methoxyquinolin-4-yl)-(S,S)oxiran-2-yl-methyl]piperidine;
[3R, 4S]-3-Ethyl-1-heptyl-4-[2-(S)-hydroxy-3-(6-methoxyquinolin-4-yl)propyl]piperidine;
[3R, 4S]-1-Heptyl-4-[N-methyl-N-(6-methoxyquinolin-4-yl)aminoethyl]-3-vinylpiperidine;
[3R,4R]-1-Heptyl-3-(1-(R,S)-hydroxyethyl)4-[3-(6-methoxyquinolin-4- . yl)propyl]piperidine;
[3R,4R]-1-Heptyl-3-(1-(R,S)-hydroxy-1-methylethyl)-4-[3-(6-methoxyquinolin-4-yl)propyl]piperidine;
[3R,4R]-1-Heptyl-3-hydroxymethyl-4-[3-(6-methoxyquinolin-4-yl)propyl]piperidine;
[3R,4R]-1-(6-Methylheptyl)-3-(1-(R) and 1-(S),2-dihydroxyethyl)-4-[3-(6-methoxyquinolin-4-yl)propyl]piperidine;
[3R, 4S]-1-Heptyl-4-[(2S)-hydroxy-3-(6-methoxyquinolin-4-yl)propyl]-3-(2-hydroxyethyl)piperidine;
[3R,4S]-1-Heptyl-3-aminocarbonyloxymethyl-4-[3-(6-methoxyquinolin-4-yl)propyl]piperidine and
[3R, 4R]-1-Heptyl-4-[3-(6-methoxyquinolin-4-yl)propyl]-3-(2-carbamoylethyl)piperidine;
or a pharmaceutically acceptable derivative of any of the foregoing compounds.

13. A process for preparing a compound of formula (I), or a pharmaceutically acceptable derivative thereof, according to claim 2, 3 or 12 which process comprises:
(a) reacting a compound of formula (IV) with a compound of formula (V): 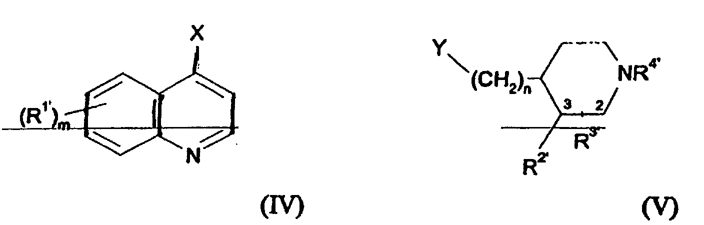 wherein m, n, R¹, R², R³ and R⁴ are as defined in formula (I), and X and Y may be the following combinations:
(i) X is M and Y is CH₂CO₂R^{x}
(ii) X is CO₂R^{y} and Y is CH₂CO₂R^{x}
(iii) one of X and Y is CH=SPh₂ and the other is CHO
(iv) X is CH₃ and Y is CHO
(v) X is CH₃ and Y is CO₂R^{x}
(vi) X is CH₂CO₂R^{y} and Y is CO₂R^{x}
(vii) X is CH=PR_{z3} and Y is CHO
(viii) X is CHO and Y is CH=PR^{z}₃
(ix) X is halogen and Y is CH=CH₂
(x) one of X and Y is COW and the other is NHR^{11'}
(xi) one of X and Y is (CH₂)ₚ-V and the other is (CH₂)_{q}NHR^{11'}, (CH₂)_{q}OH, (CH₂)_{q}SH or (CH₂)_{q}SCOR^{x} where p+q=1
(xii) one of X and Y is CHO and the other is NHR^{11'}
(xiii) one of X and Y is OH and the other is -CH=N₂
in which V and W are leaving groups, R^{x} and R^{y} are (C₁₋₆)alkyl and R^{z} is aryl or (C₁₋₆)alkyl;
(b) rearranging a compound of formula (II): 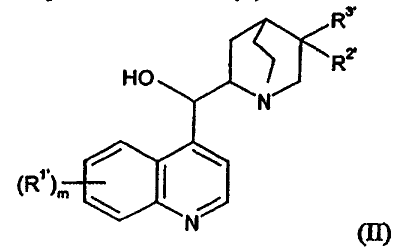 to give a compound of formula (III) which is a compound of formula (1) where R³ is in the 3-position, n is 1, A-B is COCH₂ or disubstituted epoxide and R² is H, and thereafter optionally reducing to a compound of formula (VII) which is a compound of formula (I) where n is 1, A-B is CHOHCH₂ or CH₂CHOH and R² is H;
(c) photooxygenating a compound of formula (VI): 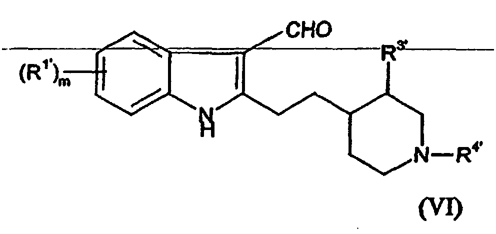 or
(d) reacting a compound of formula (IV) with a compound of formula (Vb): 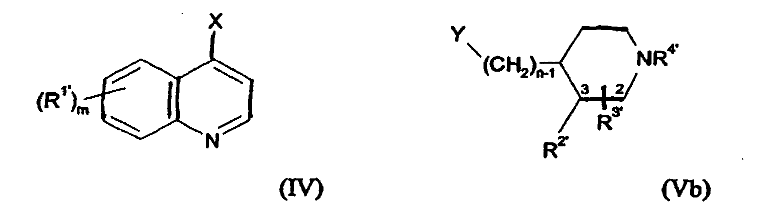
wherein m, n, R¹, R², R³ and R⁴ are as defined in formula (1), X is CH₂NHR^{11'} and Y is CHO or COW or X is CH₂OH and Y is -CH=N₂;
in which R^{1 1}', R^{1'}, R^{2'}, R³' and R⁴' are R¹¹, R¹, R², R³ and R⁴ or groups convertible thereto, and thereafter optionally or as necessary converting R^{11'}, R^{1'}, R²', R^{3'} and R⁴' to R^{11'}, R¹, R², R³ and R⁴, converting A-B to other A-B, interconverting R¹¹, R¹, R², R³ and/or R⁴ and forming a pharmaceutically acceptable derivative thereof.

14. A pharmaceutical composition comprising a compound according to claim 2, 3 or 12, and a phannaceutically acceptable carrier.

## Patentansprüche

1. Verwendung einer Verbindung der Formel (I) in Form der freien Base oder eines pharmazeutisch verträglichen Säureadditions- oder quartären Ammoniumsalzes oder N-Oxids davon 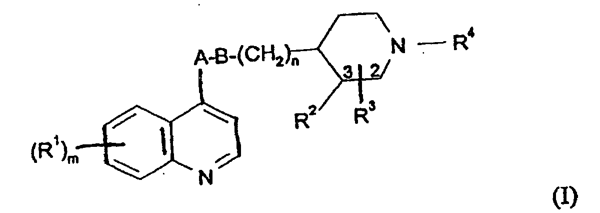 wobei: m 1 oder 2 ist;
R¹ jeweils unabhängig voneinander eine Hydroxygruppe, ein gegebenenfalls durch einen (C₁₋₆)-Alkoxy-, Amino-, Piperidyl-, Guanidino- oder Amidinorest, gegebenenfalls N-substituiert durch einen oder zwei (C₁₋₆)-Alkyl, Acyl- oder (C₁₋₆)-Alkylsulfonylreste, einen NH₂CO-, Hydroxy-, Thiol-, (C₁₋₆)-Alkylthio-, Heterocyclylthio-, Heterocyclyloxy-, Arylthio-, Aryloxy-, Acylthio-, Acyloxy- oder (C₁₋₆)-Alkylsulfonyloxyrest substituierter (C₁₋₆)-Alkoxyrest; ein (C₁₋₆)-Alkoxy-substituierter (C₁₋₆)-Alkylrest; ein Halogenatom; ein (C₁₋₆)-Alkyl-; (C₁₋₆)-Alkylthio-, Nitro-; Azido-; Acyl-; Acyloxy-; Acylthio-; (C₁₋₆)-Alkylsulfonyl-; (C₁₋₆)-Alkylsulfoxid-; Arylsulfonyl-; Arylsulfoxidrest oder ein gegebenenfalls durch einen oder zwei (C₁₋₆)-Alkyl-, Acyloder (C₁₋₆)-Alkylsulfonylreste N-substituierter Amino-, Piperidyl-, Guanidino- oder Amidinorest ist;
R² entweder ein Wasserstoffatom ist; und
R³ sich in der 2- oder 3-Position befindet, und ein Wasserstoffatom oder ein (C₁₋₆)-Alkyl- oder (C₂₋₆)-Alkenylrest ist, gegebenenfalls mit 1 bis 3 Resten substituiert, ausgewählt aus:
einem Thiol-; Halogen-; (C₁₋₆₎-Alkylthio-; Trifluormethyl-; Azido-; (C₁₋₆)-Alkoxycarbonyl-; (C₁₋₆)Aylcarbonyl-; (C₂₋₆)-Alkenyloxycarbonyl; (C₂₋₆)-Alkenylcarbonylrest; einem gegebenenfalls durch einen (C₁₋₆)-Alkyl-, (C₂₋₆)-Alkenyl-, (C₁₋₆)-Alkoxycarbonyl-, (C₁₋₆)-Alkylcarbonyl-, (C₂₋₆)-Alkenyloxycarbonyl, (C₂₋₆₎-Alkenylcarbonyl- oder Aminocarbonylrest, wobei der Aminorest gegebenenfalls durch einen (C₁₋₆)-Alkyl-, (C₂₋₆)-Alkenyl-, (C₁₋₆)-Alkylcarbonyl- oder (C₂₋₆)-Alkenylcarbonylrest substituiert ist, substituierten Hydroxyrest, einem gegebenenfalls durch einen (C₁₋₆)-Alkoxycarbonyl-, (C₁₋₆)-Alkylcarbonyl-, (C₂₋₆)-Alkenyloxycarbonyl-, (C₂₋₆)-Alkenylcarbonyl-, (C₁₋₆)-Alkyl, (C₂₋₆)-Alkenyl-, (C₁₋₆)-Alkylsulfonyl-, (C₂₋₆)-Alkenylsulfonyl- oder Aminocarbonylrest, wobei der Aminorest gegebenenfalls durch einen (C₁₋₆)-Alkyl- oder (C₂₋₆)-Alkenylrest substituiert ist, mono- oder disubstituierten Aminorest; einem Aminocarbonylrest, wobei der Aminorest gegebenenfalls durch einen (C₁₋₆)-Alkyl-, Hydroxy-(C₁₋₆)-alkyl-, Aminocarbonyl-(C₁₋₆)-alkyl-, (C₂₋₆)-Alkenyl-, (C₁₋₆)-Alkoxycarbonyl-, (C₁₋₆)-Alkylcarbonyl, (C₂₋₆)-Alkenyloxycarbonyl- oder (C₂₋₆)-Alkenylcarbonylrest substituiert ist, und gegebenenfalls weiterhin durch einen (C₁₋₆)-Alkyl-, Hydroxy-(C₁₋₆)-alkyl-, Aminocarbonyl-(C₁₋₆)-alkyl- oder (C₂₋₆)-Alkenylrest substituiert ist; einem Oxo-; (C₁₋₆)-Alkylsulfonyl-; (C₂₋₆)-Alkenylsulfonyl; oder Aminosulfonylrest, wobei der Aminorest gegebenenfalls durch einen (C₁₋₆)-Alkyl- oder (C₂₋₆)-Alkenylrest substituiert ist; oder
R³ sich in der 3-Position befindet, und R² und R³ zusammen ein zweiwertiger Rest =CR^{5¹} R^{6¹} sind, wobei R^{5¹} und R^{6¹} unabhängig voneinander aus H, einem (C₁₋₆)-Alkyl-, (C₂₋₆)-Alkenyl-, Aryl-(C₁₋₆)-alkyl- und Aryl-(C₂₋₆)-alkenylrest ausgewählt sind, wobei jede Alkyl- oder Alkenyleinheit gegebenenfalls durch 1 bis 3 Reste, ausgewählt aus den vorstehend für Substituenten an R³ genannten, substituiert ist;
R⁴ ein Rest -CH₂-R⁵ ist, wobei R⁵ ausgewählt ist aus:
einem (C₃₋₁₂)-Alkyl-; Hydroxy-(C₃₋₁₂)-alkyl-; (C₁₋₁₂)-Alkoxy-(C₃-₁₂)-alkyl-; (C₁₋₁₂)-Alkanoyloxy-(C₃₋₁₂)-alkyl-; (C₃₋₆)-Cycloalkyl-(C₃₋₁₂)-alkylrest; einem Hydroxy-, (C₁₋₁₂)-Alkoxy- oder (C₁₋₁₂)-Alkanoyloxy-(C₃₋₆)-cycloalkyl-(C₃₋₁₂)-alkylrest; einem Cyano-(C₃-₁₂)-alkyl-; (C₂₋₁₂)-Alkenyl-; (C₂₋₁₂)-Alkinyl-; Tetrahydrofuryl-, Mono- oder Di-(C₁₋₁₂)-alkylamino-(C₃₋₁₂)-alkyl-; Acylamino-(C₃₋₁₂)-alkylrest; einem (C₁₋₁₂)-Alkyl- oder Acyl-aminocarbonyl-(C₃₋₁₂)-alkylrest; einem Mono- oder Di-(C₁₋₂)-alkylamino-(hydroxy)-(C₃₋₁₂)-alkylrest; einem gegebenenfalls substituierten Phenyl-(C₁₋₂)-alkyl-, Phenoxy-(C₁₋₂)-alkyl- oder Phenyl-(hydroxy)-(C₁₋₂)-alkylrest; einem gegebenenfalls substituierten Diphenyl-(C₁₋₂)-alkylrest; einem gegebenenfalls substituierten Phenyl-(C₂₋₃)-alkenylrest; einem gegebenenfalls substituierten Benzoyl- oder Benzoylmethylrest; einem gegebenenfalls substituierten Heteroaryl-(C₁₋₂)-alkylrest und einem gegebenenfalls substituierten Heteroaroyl- oder Heteroaroylmethylrest;
oder R⁴ eine 3-Benzoylpropyl- oder 3-(4-Fluorbenzoyl)propylgruppe ist;
n 0, 1 oder 2 ist;
A ein Rest NR¹¹, O, S(O)ₓ oder CR⁶R⁷ ist und B ein Rest NR¹¹, O, S(O)ₓ oder CR⁸R⁹ ist,
wobei x 0, 1 oder 2 ist und wobei:
R⁶, R⁷, R⁸ und R⁹ jeweils unabhängig voneinander ausgewählt sind aus: H; einem Thiol-; (C₁₋₆)-Alkylthio-; Halogen-; Trifluormethyl-; Azido-; (C₁₋₆)-Alkyl-; (C₂₋₆)-Alkenyl-; (C₁₋₆)-Alkoxycarbonyl-; (C₁₋₆)-Alkylcarbonyl-; (C₂₋₆)-Alkenyloxycarbonyl-; (C₂₋₆)-Alkenylcarbonylrest; einem gegebenenfalls mit entsprechenden Substituenten wie in R³ substituierten Hydroxy-, Amino- oder Aminocarbonylrest; einem (C₁₋₆)-Alkylsulfonyl-; (C₂₋₆)-Alkenylsulfonyl- oder (C₁₋₆)-Aminosulfonylrest, wobei der Aminorest gegebenenfalls durch einen (C₁₋₆)-Alkyl- oder (C₁₋₆)-Alkenylrest substituiert ist;
oder R⁶ und R⁸ zusammen eine Bindung bedeuten und R⁷ und R⁹ wie vorstehend definiert sind;
oder R⁶ und R⁸ zusammen eine Gruppe -O- bedeuten und R⁷ und R⁹ beide ein Wasserstoffatom sind;
oder R⁶ und R⁷ oder R⁸ und R⁹ zusammen einen Oxorest bedeuten;
und R¹¹ jeweils unabhängig voneinander H, ein Trifluoromethyl-, (C₁₋₆)-Alkyl, (C₁₋₆)-Alkenyl-, (C₁₋₆)-Alkoxycarbonyl-, (C₁₋₆)-Alkylcarbonyl-, Aminocarbonylrest ist, wobei der Aminorest gegebenenfalls durch einen (C₁₋₆)-Alkoxycarbonyl-, (C₁₋₆)-Alkylcarbonyl-, (C₁₋₆)-Alkenyloxycarbonyl-, (C₂₋₆)-Alkenylcarbonyl-, (C₁₋₆)-Alkyl- oder (C₁₋₆)-Alkenylrest substituiert ist, und gegebenenfalls weiterhin durch einen (C₁₋₆)-Alkyl- oder (C₁₋₆)-Alkenylrest substituiert ist;
wobei:
"heterocyclisch" wie hierin verwendet ein aromatischer oder nichtaromatischer, einzelner oder kondensierter Ring ist, welcher bis zu vier Heteroatome in jedem Ring, ausgewählt aus Sauerstoff, Stickstoff und Schwefel, enthält, und 4 bis 7 Ringatome aufweist, wobei die Ringe unsubstituiert oder mit bis zu drei Resten, ausgewählt aus einem Amino-, Halogen-, (C₁₋₆)-Alkyl-, (C₁₋₆)-Alkoxy-, Halogen-(C₁₋₆)-alkyl-, Hydroxy-, Carboxy-, Carboxysalz-, (C₁₋₆)-Alkoxycarbonyl-, (C₁₋₆)-Alkoxycarbonyl-(C₁₋₆)-alkyl-, Aryl- und Oxoresten unsubstituiert oder substituiert ist, und wobei jeder Aminorest, welcher einen Teil eines einzelnen oder kondensierten, nichtaromatischen, heterocyclischen Rings, wie vorstehend definiert, . bildet, gegebenenfalls durch einen Hydroxy-, (C₁₋₆)-Alkoxy-, Thiol-, (C₁₋₆)-Alkylthio-, Halogen-, Trifluormethyl-, Acyl- oder.(C₁₋₆)-Alklsulfonylrest substituierten, (C₁₋₆)-Alkylrest substituiert ist;
_{"}Heteroaryl" ein aromatischer, heterocyclischer Rest wie vorstehend definiert ist;
"Aryl" ein Phenyl- oder Naphthylrest ist, jeweils gegebenenfalls durch bis zu fünf Reste substituiert, ausgewählt aus Halogen-, Mercapto-, (C₁₋₆)-Alkyl-, Phenyl-, (C₁₋₆)-Alkoxy-, Hydroxy-(C₁₋₆)-alkyl-, Mercapto-(C₁₋₆)-alkyl-, Halogen-(C₁₋₆)-alkyl-, Hydroxy-, Amino-, Nitro-, Carboxy-, (C₁₋₆)-Alkylcarbonyloxy-, (C₁₋₆)-Alkoxycarbonyl-, Formyl- oder (C₁₋₆)-Alkylcarbonylresten;
"Acyl" ein (C₁₋₆)-Alkoxycarbonyl-, Formyl- oder (C₁₋₆)-Alkylcarbonylrest ist ;
mit der Maßgabe, dass A und B nicht beide aus NR¹¹, O und S(O)'ₓ ausgewählt sein können und, falls ein Rest aus A und B eine Gruppe CO ist, der andere nicht CO, O oder S(O)ₓ ist, zur Herstellung eines Medikaments zur Verwendung in der Behandlung von bakteriellen Infektionen bei Säugetieren.

2. Verbindung der Formel (IA), welche eine Verbindung der Formel (I) wie in Anspruch 1 definiert ist, wobei R³ ein gegebenenfalls an dem/den Hydroxyrest(en) substituierter Hydroxy-(C₁₋₆)-alkyl- oder 1,2-Dihydroxy-(C₂₋₆)-alkylrest ist.

3. Verbindung der Formel (IB), welche eine Verbindung der Formel (I) wie in Anspruch 1 definiert ist, wobei mindestens ein Rest R¹ ein gegebenenfalls durch einen N-substituierten Amino-, Guanidino- oder Amidinorest substituierter (C₂₋₆)-Alkoxyrest oder ein mit einem Piperidylrest substituierter (C₁₋₆)-Alkoxyrest ist, A eine Gruppe CH₂, CHOH, CH(NH₂), C(Me)(OH) oder CH(Me) ist und B eine Gruppe CH₂, CHOH oder CO ist.

4. Verwendung gemäß Anspruch 1, wobei sich R¹ in der 6-Position des Chinolinkerns befindet, und ein Methoxy-, Amino-(C₃₋₅)-alkyloxy-, Nitro- oder Fluorrest ist, und m 1 ist.

5. Verwendung gemäß Anspruch 1 oder 4, wobei R³ ein (C₁₋₆)-Alkyl-, (C₁₋₆)-Alkenyl- oder ein gegebenenfalls substituierter 1-Hydroxy-(C₁₋₆)-alkylrest ist.

6. Verwendung gemäß Anspruch 5, wobei R³ ein Hydroxymethyl-, 1-Hydroxyethyl- oder 1,2-Dihydroxyethylrest ist, wobei der 2-Hydroxyrest gegebenenfalls durch einen Alkylcarbonyl- oder Aminocarbonylrest substituiert ist, wobei der Aminorest gegebenenfalls substituiert ist.

7. Verwendung gemäß einem der Ansprüche 1 und 4 bis 6, wobei sich R³ in der 3-Position befindet.

8. Verwendung gemäß einem der Ansprüche 1 und 4 bis 7, wobei A eine Gruppe NH, NCH₃, O, CH₂, CHOH, CH(NH₂), C(Me)(OH) oder CH(Me) ist und B eine Gruppe CH_{2,} CHOH, CO oder S ist, oder A ein Rest CR⁶R⁷ und B ein Rest CR⁸R⁹ ist und R⁶ und R⁸ zusammen -O- bedeuten und R⁷ und R⁹ beide ein Wasserstoffatom bedeuten, und n 0 oder 1 ist.

9. Verwendung gemäß Anspruch 8, wobei:
A eine Gruppe NH ist, B eine Gruppe CO ist, und n 1 oder 0 ist;
A eine Gruppe O ist, B eine Gruppe CH₂ ist, und n 1 oder 0 ist;
A eine Gruppe CH₂ or CH₂OH ist, B eine Gruppe CH₂ ist, und n 1 oder 0 ist;
A eine Gruppe NCH₃, CH(NH₂), C(Me)(OH) oder CH(Me) ist, B eine Gruppe CH₂ ist, und n 1 ist;
A ein Rest CR⁶R⁷ und B ein Rest CR⁸R⁹ ist, und R⁶ und R⁸ zusammen -O- bedeuten, und R⁷ und R⁹ beide ein Wasserstoffatom sind, und n 1 ist.

10. Verwendung gemäß einem der Ansprüche 1 und 4 bis 9, wobei R⁴ ein (C₅₋₁₀)-Alkyl-, ein
. unsubstituierter Phenyl-(C₂₋₃)-alkyl- oder ein unsubstituierter Phenyl-(C₃₋₄)-alkenylrest ist.

11. Verwendung gemäß einem der Ansprüche 1 und 4 bis 10, wobei R⁵ an den α- und, falls angebracht, β-Positionen unverzweigt ist.

12. Verbindung der Formel (I) wie in Anspruch 1 definiert, ausgewählt aus:
[3R,4R]-3-Ethyl-1-hexyl-4-[3-oxo-3-(6-methoxychinolin-4-yl)propyl]piperidin;
[3R,4R]-3-Ethyl-1-hexyl-4-[3-(R,S)-hydroxy-3-(6-methoxychinolin-4-yl)propyl]piperidin;
[3R,4R]-3-Ethyl-1-heptyl-4-[3-oxo-3-(6-methoxychinolin-4-yl)propyl]piperidin;
[3R,4R]-3-Ethyl-1-heptyl-4-[3-(R,S)-hydroxy-3-(6-methoxychinolin-4-yl)propyl]piperidin;
[3R,4R]-3-Ethyl-1-octyl-4-[3-oxo-3-(6-methoxychinolin-4-yl)propyl]piperidin;
[3R,4R]-3-Ethyl-1-octyl-4-[3-(R,S)-hydroxy-3-(6-methoxychinolin-4-yl)propyl]piperidin;
[3R,4R]-3-Ethyl-1-decyl-4-[3-oxo-3-(6-methoxychinolin-4-yl)propyl]piperidin;
[3R,4R]-3-Ethyl-1-decyl-4-[3-(R,S)-hydroxy-3-(6-methoxychinolin-4-yl)propyl]piperidin;
[3R,4R]-3-Ethyl-1-dodecyl-4-[3-oxo-3-(6-methoxychinolin-4-yl)propyl]piperidin;
[3R,4R]-3-Ethyl-1-dodecyl-4-[3-(R,S)-hydroxy-3-(6-methoxychinolin-4-yl)propyl]piperidin;
[3R,4R]-3-Ethyl-1-cinnamyl-4-[3-(R,S)-hydroxy-3-(6-methoxychinolin-4-yl)propyl]piperidin;
[3R,4R]-3-Ethyl-1-heptyl-4-[3-(6-methoxychinolin-4-yl)propyl]piperidin;
[3R,4R]-3-Ethenyl-1-heptyl-4-[3-(R,S)-hydroxy-3-(6-methoxychinolin-4-yl)propyl]piperidin;
[3R,4R]-3-Ethyl-1-heptyl-4-[3-(R,S)-hydroxy-3-(6-hydroxychinolin-4-yl)propyl]piperidin;
[3R,4R]-1-Heptyl-3-(2-hydroxyethyl)-4-[3-(R,S)-hydroxy-3-(6-methoxychmolin-4-yl)propyl]piperidin;
[3R,4R]-3-Ethyl-1-heptyl-4-[3-(R,S)-hydroxy-3-(6-[5-aminopentyloxy]-chinolin-4-yl)propyl]piperidin;
[3R,4R]-3-Ethyl-1-heptyl-4-[3-(R,S)-hydroxy-3-(6-[2-amino-2-oxo-1,1-dimethyl]ethoxychinolin-4-yl)propyl]piperidin;
[3R,4R]-3-Ethyl-1-heptyl-4-[3-(R,S)-hydroxy-3-(6-aminochinolin-4-yl)propyl]piperidin;
[3R,4R]-3-Ethyl-1-heptyl-4-[3-(R,S)-hydroxy-3-(6-azidochinolin-4-yl)propyl]piperidin;
[3R,4R]-3-Ethyl-1-heptyl-4-[3-(6-hydroxychinolin-4-yl)propyl]piperidin;
[3R,4R]-3-Ethyl-1-heptyl-4-[3-(6-propyloxychinolin-4-yl)propyl]piperidin;
[3R,4R]-3-Ethyl-1-heptyl-4-[3-(6-(5-aminopentyloxy)-chinolin-4-yl)propyl]piperidin;
[3R,4R]-3-Ethenyl-1-(2-phenoxyethyl)-4-[3-(6-methoxychinolin-4-yl)propyl]piperidin;
[3S,4R]-3-Ethenyl-1-heptyl-4-[3-(6-methoxychinolin-4-yl)propyl]piperidin;
[3R,4R]-3-Ethenyl-1-heptyl-4-[3-(6-methoxychinolin-4-yl)propyl]piperidin;
[3R,4R]-1-Heptyl-3-(2-hydroxyethyl)-4-[3-(6-methoxychinolin-4-yl)propyl]piperidin;
[3R,4R]-1-Heptyl-3-(2-acetoxyethyl)-4-[3-(6-methoxychinolin-4-yl)propyl]piperidin;
[3R,4R]-1-Heptyl-3-(3-hydroxypropyl)-4-[3-(6-methoxychinolin-4-yl)propyl]piperidin;
[3R,4R]-1-Heptyl-3-(1-hydroxyethyl)-4-[3-(6-methoxychinolin-4-yl)propyl]piperidin;
[3R,4R]-3-Ethyl-1-(2-phenylethyl)-4-[3-(R,S)-hydroxy-3-(6-methoxychinolin-4-yl)propyl]piperidin;
[3R,4R]-3-Ethyl-1-(3-phenylpropyl)-4-[3-(R,S)-hydroxy-3-(6-methoxychinolm-4-yl)propyl]piperidin;
Heptyl-4-[2-(R,S)-hydroxy-3-(6-methoxychinolin-4-yl)propyl]piperidin;
1-Heptyl-4-[3-(6-methoxychinolin-4-yl)prop-2-enyl]piperidin;
1-Heptyl-4-[3-(6-methoxychinolin-4-yl)propyl]piperidin;
[3R,4R]-3-Ethyl-1-heptyl-4-[3-(R,S)-hydroxy-3-(6-methoxychinolin-4-yl)butyl]piperidin;
[3R,4R]-3-Ethenyl-1-heptyl-4-[3-(R,S)-azido-3-(6-methoxychinolin-4-yl)propyl]piperidin;
[3R,4R]-3-Ethenyl-1-heptyl-4-[3-(R,S)-amino-3-(6-methoxychinolin-4-yl)propyl]piperidin;
[3R,4R]-3-Ethyl-1-heptyl-4-[3-(R,S)-amino-3-(6-methoxychinolin-4-yl)propyl]piperidin;
[3R,4R]-3-Ethyl-1-heptyl-4-[3-(6-methoxychinolin-4-yl)butyl]piperidin;
[3R,4R]-3-Ethenyl-1-heptyl-4-[3-(R,S)-acetamido-3-(6-methoxychinolin4-yl)propyl]piperidin;
[3R,4R]-1-Heptyl-3-(2-(R,S)-hydroxypropyl)-4-[3-(6-methoxychinolin-4-yl)propyl]piperidin;
[3R,4R]-1-Heptyl-3-(1-(R,S),2-dihydroxyethyl)-4-[3-(6-methoxychinolin-4-yl)propyl]piperidin;
[3R,4R]-1-Heptyl-3-aminocarbonyloxyethyl-4-[3-(6-methoxychinolin-4-yl)propyl]piperidin;
[3R,4R]-3-(1-(R,S)-2-dihydroxyethyl)-1-heptyl-4-[3-(R,S)-hydroxy-3-(6-methoxychinolin-4-yl)propyl]piperidin;
[3R,4R]-3-Ethyl-1-heptyl-4-[(6-methoxychinolinyl-4-oxy)methyl)piperidin;
[3R,4S]-3-Ethenyl-1-heptyl-4-[2-(6-methoxychinolin-4-yl)-oxyethyl]piperidin;
1-Heptyl-4-[(6-methoxychinolin-4-yl)oxymethyl]piperidin;
[3R,4R]-3-Ethyl-1-heptyl-4-[(6-methoxychinolin-4-yl)methylthiomethyl]piperidin;
[3R,4R]-1-Heptyl-3-ethenyl-4-[{(6-methoxychinolin-4-yl)carbonylamino}methyl]piperidin;
[3R,4R]-3-Ethenyl-1-heptyl-piperidin-4-[N-(6-methoxychinolin-4-yl)]propionamid;
[3R,4R]-3-Ethenyl-1-heptyl-piperidin-4-[N-(6-methoxychinolin-4-yl)]propylamin;
[3R,4S]-3-Ethenyl-1-heptyl-piperidin-4-[N-(6-methoxychinolin-4-yl)]acetamid;
[3R,4R]-3-Ethenyl-1-heptyl-piperidin-4-[N-(6-methoxychinolin-4-yl)]ethylamin;
[3R,4S]-3-Ethenyl-1-heptyl-4-[2-(R,S)-hydroxy-2-(6-methoxychinolin-4-yl)ethyl]piperidin;
[3R,4R]-3-Ethenyl-1-heptyl-4-[2-(6-methoxychinolin-4-yl)ethyl]piperidin;
[3R,4R]-3-Ethyl-1-heptyl-4-[2-(6-methoxychinolin-4-yl)ethyl]piperidin;
1-Heptyl-4-[2-(R,S)-hydroxy-2-(6-methoxy-4-chinolinyl)ethyl]piperidin;
[3 S,4R]-3-Ethenyl-1-heptyl-4-[2-(6-methoxychinolin-4-yl)ethyl]piperidin;
N-(6-Methoxy-4-chinolinyl)-1-heptyl-4-piperidincarboxamid;
(3Z)-(4R)-3-Ethyliden-1-heptyl-4-[3-(6-methoxychinolin-4-yl)propyl]piperidin;
[3R,4S]-1-Cinnamyl-4-[2-(6-methoxychinolin-4-yl)-oxyethyl]piperidin;
[3R,4R]-3-(2-Acetoxyethyl)-1-heptyl-4-[3-(6-methoxy-chinolin-4-yl)propyl]piperidin;
[3R,4R]-3-Ethyl-1-heptyl-4-[3-(6-{2-hydroxyethyloxy}chinolin-4-yl)propyl]piperidin;
[3R,4R]-3-(Ethylaminocarbonyloxyethyl)-1-heptyl-4-[3-(6-methoxychinolin-4-yl)propyl]piperidin;
[3R,4R]-3-Ethenyl-1-heptyl-4-[3-(R,S)-aminocarbonylamino-3-(6-methoxychinolin-4-yl)propyl]piperidin;
[3R,4R]-3-Ethyl-1-heptyl-4-[3-(6-(4-aminobutyloxy)-chinolin-4-yl)propyl]piperidin;
[3R,4R]-1-Heptyl-3-(1-(R)- und 1-(S)-hydroxy-2-methoxyethyl)-4-[3-(6-methoxychinolin-4-yl)propyl]piperidin;
[3R,4R]-1-Heptyl-3-(1-(R)- und 1-(S)-hydroxy-2-methylthioethyl)-4-[3-(6-methoxychinolin-4-yl)propyl]piperidin;
[3R,4R]-1-(5-Methylhexyl)-3-(1-(R)- und 1-(S),2-dihydroxyethyl)-4-[3-(6-methoxychinolin-4-yl)propyl]piperidin;
[3R,4R]-3-Ethyl-1-heptyl-4-[3-(6-(3-aminopropyl)oxychinolin-4-yl)propyl]piperidin;
[3R,4R]-3-Ethyl-1-heptyl-4-[3-(6-(2-aminoethyl)oxychinolin-4-yl)propyl]piperidin;
[3R,4R]-3-Ethyl-1-heptyl-4-[3-(6-(3-guanidinopropyl)oxychinolin-4-yl)propyl]piperidin;
[3R,4R]-3-Ethyl-1-heptyl-4-[3-(6-(piperidin-4-yl)methoxychinolin-4-yl)propyl]piperidin;
[3R,4S)-1-Heptyl-3-vinyl-4-[3-(6-methoxychinolin-4-yl)-(R,R)-oxiran-2-ylmethyl]piperidin;
[3R,45]-1-Heptyl-4-[(2S)-hydroxy-3-(6-methoxychinolin-4-yl)propyl]-3-vinylpiperidin;
[3R,4S]-1-Heptyl-3-vinyl-4-[3-(6-methoxychinolin-4-yl)-(S,S)-oxiran-2-ylmethyl]piperidin;
[3R,4S]-3-Ethyl-1-heptyl-4-[2-(S)-hydroxy-3-(6-methoxychinolin-4-yl)propyl]piperidin;
[3R,4S]-1-Heptyl-4-[N-methyl-N-(6-methoxychinolin-4-yl)-aminoethyl]-3-vinylpiperidin;
[3R,4R]-1-Heptyl-3-(1-(R,S)-hydroxyethyl)-4-[3-(6-methoxychinolin-4-yl)propyl]piperidin;
[3R,4R]-1-Heptyl-3-(1-(R,S)-hydroxy-1-methylethyl)-4-[3-(6-methoxychinolin-4-yl)propyl]piperidin;
[3R,4R]-1-Heptyl-3-hydroxymethyl-4-[3-(6-methoxychinolin-4-yl)propyl]piperidin;
[3R,4R]-1-(6-Methylheptyl)-3-(1-(R)- und 1-(S)-2-dihydroxyethyl)-4-[3-(6-methoxychinolin-4-yl)propyl)piperidin;
[3R,4S]-1-Heptyl-4-[(2S)-hydroxy-3-(6-methoxychinolin-4-yl)propyl]-3-(2-hydroxyethyl)piperidin;
[3R,45]-1-Heptyl-3-aminocarbonyloxymethyl-4-[3-(6-methoxychinolin-4-yl)propyl]piperidin und
[3R,4R]-1-Heptyl-4-[3-(6-methoxychinolin-4-yl)propyl]-3-(2-carbamoylethyl)piperidin;
oder ein pharmazeutisch verträgliches Derivat einer der vorstehend genannten Verbindungen.

13. Verfahren zur Herstellung einer Verbindung der Formel (I) oder eines pharmazeutisch verträglichen Derivats davon nach Anspruch 2, 3 oder 12, wobei das Verfahren umfasst:
(a) Umsetzen einer Verbindung der Formel (IV) mit einer Verbindung der Formel (V): 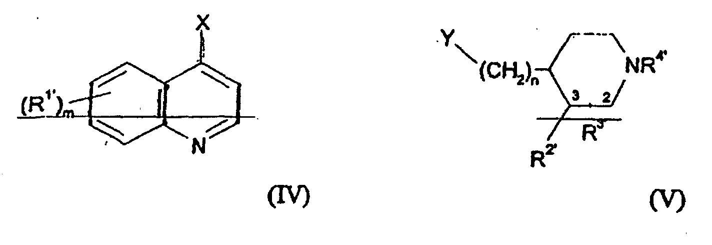 wobei m, n, R¹, R², R³ und R⁴ wie in Formel (I) definiert sind, und X und Y nachstehende Kombinationen sein können:
(i) X ist M und Y ist CH₂CO₂R^{x}
(ii) X ist CO₂R^{y} und Y ist CH₂CO₂R^{x}
(iii) ein Rest aus X und Y ist CH=SPh₂ und der andere ist CHO
(iv) X ist CH₃ und Y ist CHO
(v) X ist CH₃ und Y ist CO₂R^{x}
(vi) X ist CH₂CO₂R^{y} und Y ist CO₂R^{x}
(vii) X ist CH=PR^{z}₃ und Y ist CHO
(viii) X ist CHO und Y ist CH=PR^{z}₃
(ix) X ist ein Halogenatom und Y ist CH=CH₂
(x) ein Rest aus X und Y ist COW und der andere ist NHR^{11'}
(xi) ein Rest aus X und Y ist (CH₂)ₚ-V und der andere ist (CH₂)_{q}NHR^{11'},
(CH₂)_{q}OH, (CH₂)_{q}SH oder (CH₂)_{q}SCOR^{x}, wobei p + q =1
(xii) ein Rest aus X und Y ist CHO und der andere ist NHR^{11'}
(xiii) ein Rest aus X und Y ist OH und der andere ist -CH=N₂
wobei V und W Abgangsgruppen sind, R^{x} und R^{y} (C₁₋₆)-Alkylreste sind und R^{z} ein Aryl- oder (C₁₋₆)-Alkylrest ist;
(b) Umlagern einer Verbindung der Formel (II): 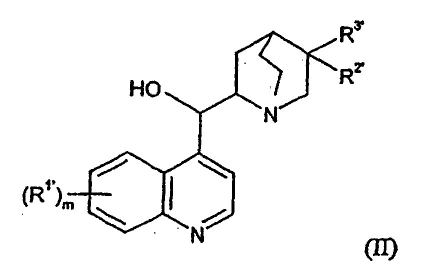 um eine Verbindung der Formel (III) zu ergeben, welche eine Verbindung der Formel (1) ist, wobei sich R³ in 3-Position befindet, n 1 ist, A-B COCH₂ oder ein disubstituiertes Epoxid ist und R² H ist, und nachfolgend gegebenenfalls Reduzieren zu einer Verbindung der Formel (VII), welche eine Verbindung der Formel (I) ist, wobei n 1 ist, A-B CHOHCH₂ oder CH₂CHOH ist und R² H ist;
(c) Photooxidieren einer Verbindung der Formel (VI): 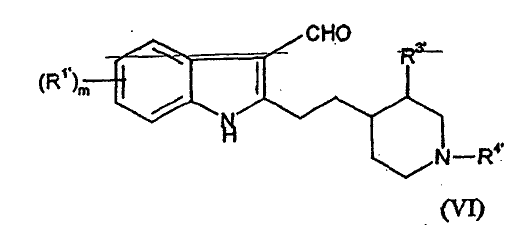 oder
(d) Umsetzen einer Verbindung der Formel (IV) mit einer Verbindung der Formel (Vb): 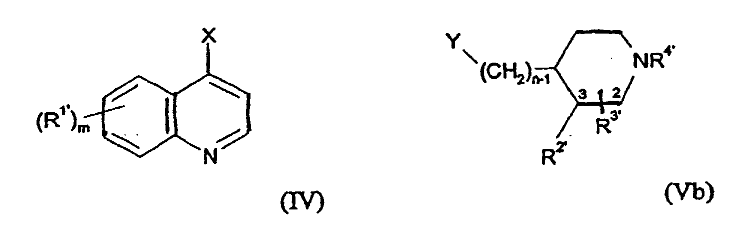 wobei m, n, R¹, R², R³ und R⁴ wie in Formel (I) definiert sind, X CH₂NHR^{11'} ist und Y CHO oder COW ist, oder X CH₂OH ist und Y -CH=N₂ ist;
wobei R^{11'}, R^{1'}, R²', R^{3'} und R^{4'} R¹¹, R¹, R², R³ und R⁴ oder dazu umwandelbare Reste sind, und nachfolgend gegebenenfalls oder falls notwendig Umwandeln von R^{11'}, R^{1'}, R²', R³' und R⁴' in R¹¹, R¹, R², R³ und R⁴, Umwandeln von A-B in einen anderen Rest A-B, gegenseitiges Umwandeln von R¹¹, R¹, R², R³ und/oder R⁴ und Bilden eines pharmazeutisch verträglichen Derivates davon.

14. Arzneimittel, umfassend eine Verbindung nach Anspruch 2, 3 oder 12 und einen pharmazeutisch verträglichen Träger.

## Revendications

1. Utilisation d'un composé de formule (I) sous forme de base libre ou d'un de ses sels d'addition d'acides ou d'ammonium quaternaire pharmaceutiquement acceptables ou de son N-oxyde 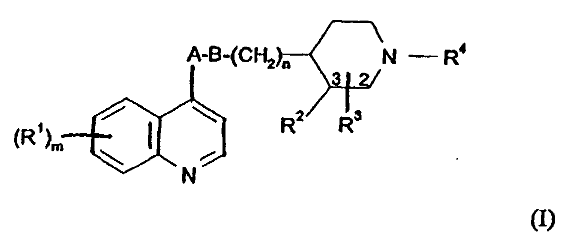 formule dans laquelle ;
m est égal à 1 ou 2 ;
chaque groupe R¹ représente, indépendamment, un groupe hydroxy ; alkoxy en C₁ à C₆ facultativement substitué avec un substituant alkoxy en C₁ à C₆, amino, pipéridyle, guanidino ou amidino facultativement N-substitué avec un ou deux groupes alkyle en C₁ à C₆, acyle ou alkylsulfonyle en C₁ à C₆, NH₂CO, hydroxy, thiol, alkylthio en C₁ à C₆, hétérocyclylthio, hétérocyclyloxy, arylthio, aryloxy, acylthio, acyloxy ou (alkyle en C₁ à C₆) sulfonyloxy ; alkyle en C₁ à C₆ à substituant alkoxy en C₁ à C₆ ; halogéno ; alkyle en C₁ à C₆ ; alkylthio en C₁ à C₆ ; nitro ; azido ; acyle ; acyloxy ; acylthio ; alkylsulfonyle en C₁ à C₆ ; alkylsulfoxyde en C₁ à C₆ ; arylsulfonyle ; arylsulfoxyde, ou un groupé amino, pipéridyle, guanidino ou amidino facultativement N-substitué avec un ou deux groupes alkyle en C₁ à C₆, acyle ou alkylsulfonyle en C₁ à C₆ ;
soit R² représente un atome d'hydrogène ; et
R³ est en position 2 ou 3 et représente un atome d'hydrogène ou un groupe alkyle en C₁ à C₆ ou alcényle en C₂ à C₆ facultativement substitué avec un ou trois groupes choisis entre des groupes :
thiol ; halogéno ; alkylthio en C₁ à C₆ ; trifluorométhyle ; azido ; (alkoxy en C₁ à C₆)carbonyle ; (alkyle en C₁ à C₆) carbonyle ; (alcényle en C₂ à C₆) oxycarbonyle ; (alcényle en C₂ à C₆) carbonyle ; hydroxy facultativement substitué avec un groupe alkyle en C₁ à C₆, alcényle en C₂ à C₆, (alkoxy en C₁ à C₆) carbonyle, (alkyle en C₁ à C₆)-carbonyle, (alcényle en C₂ à C₆) oxycarbonyle, (alcényle en C₂ à C₆) carbonyle ou aminocarbonyle dans lequel le groupe amino est facultativement substitué avec un substituant alkyle en C₁ à C₆, alcényle en C₂ à C₆, (alkyle en C₁ à C₆)-carbonyle ou (alcényle en C₂ à C₆) carbonyle ; amino facultativement mono- ou disubstitué avec un substituant (alkoxy en C₁ à C₆) carbonyle, (alkyle en C₁ à C₆) carbonyle, (alcényle en C₂ à C₆) oxycarbonyle, (alcényle en C₂ à C₆)-carbonyle, alkyle en C₁ à C₆, alcényle en C₂ à C₆, alkylsulfonyle en C₁ à C₆, alcénylsulfonyle en C₂ à C₆ ou aminocarbonyle dans lequel le groupé amino est facultativement substitué avec un substituant alkyle en C₁ à C₆ ou alcényle en C₂ à C₆ ; aminocarbonyle dans lequel le groupe amino est facultativement substitué avec un substituant alkyle en C₁ à C₆, hydroxyalkyle en C₁ à C₆, aminocarbonyl (alkyle en. C₁ à C₆), alcényle en C₂ à C₆, (alkoxy en C₁ à C₆) carbonyle, (alkyle en C₁ à C₆) carbonyle, (alcényle en C₂ à C₆) oxycarbonyle ou (alcényle en C₂ à C₆) carbonyle et facultativement substitué en outre avec un substituant alkyle en C₁ à C₆, hydroxyalkyle en C₁ à C₆, aminocarbonyl-(alkyle en C₁ à C₆) ou alcényle en C₂ à C₆ ; oxo ; alkylsulfonyle en C₁ à C₆ ; alcénylsulfonyle en C₂ à C₆ ; ou aminosulfonyle dans lequel le groupe amino est facultativement substitué avec un substituant alkyle en C₁ à C₆ ou alcényle en C₂ à C₆ ; soit
R³ est en position 3 et R² et R³_{,} conjointement, représentent un résidu divalent =CR^{5¹}R^{6¹} dans lequel R^{5¹} et R^{6¹} (sont choisis, indépendamment, entre H, des groupes alkyleen C₁ à C₆, alcényle en C₂ à C₆, aryl (alkyle en C₁ à C₆) et aryl (alcényle en C₂ à C₆), n'importe quel groupement alkyle ou alcényle étant facultativement substitué avec un à trois groupes choisis parmi ceux énumérés ci-dessus pour les substituants sur R³ ;
R⁴ représente un groupe -CH₂-R⁵ dans lequel R⁵ est choisi entre des groupes :
alkyle en C₃ à C₁₂ ; hydroxyalkyle en C₃ à C₁₂ ; (alkoxy en C₁ à C₁₂) (alkyle en C₃ à C₁₂) ; (alcanoyle en C₁ à C₁₂)oxy-(alkyle en C₃ à C₁₂) ; (cycloalkyle en C₃ à C₆) (alkyle en C₃ à C₁₂) ; hydroxy-, (alkoxy en C₁ à C₁₂)- ou (alcanoyle en C₁ à C₁₂)oxy- (cycloalkyle en C₃ à C₆) (alkyle en C₃ à C₁₂) ; cyano (alkyle en C₃ à C₁₂) ; alcényle en C₂ à C₁₂ ; alcynyle en C₂ à C₁₂ ; tétrahydrofuryle ; mono- ou di-(alkyle en C₁ à C₁₂) amino (alkyle en C₃ à C₁₂) ; acylamino (alkyle en C₃ à C₁₂) ; (alkyle en C₁ à C₁₂)- ou acyl-aminocarbonyl(alkyle en C₃ à C₁₂) ; mono- ou di-(alkyle en C₁ à C₁₂) amino (hydroxy)-(alkyle en C₃ à C₁₂) ; (phényle facultativement substitué)(alkyle en C₁ ou C₂), phénoxy(alkyle en C₁ ou C₂) ou phényl (hydroxy) (alkyle en C₁ ou C₂) ; (diphényle facultativement substitué) (alkyle en C₁ ou C₂) ; (phényle facultativement substitué) (alcényle en C₂ ou C₃) benzoyle ou benzoylméthyle facultativement substitué ; (hétéroaryle facultativement substitué) (alkyle en C₁ ou C₂) ; et hétéro-aroyle ou hétéroaroylméthyle facultativement substitué ;
ou bien R⁴ représente un groupe 3-benzoylpropyle ou 3-(4-fluorobenzoyl)propyle ;
n est égal à 0, 1 ou 2 ;
A représente un groupe NR¹¹, O, S(O)ₓ ou CR⁶R⁷ et B représente un groupe NR¹¹, O, S(O)ₓ ou CR⁸R⁹, dans lesquels x est égal à 0, 1 ou 2, et dans lesquels :
chacun de R⁶, R⁷, R⁸ et R⁹ est choisi, indépendamment, entre des groupes : H ; thiol ; alkylthio en C₁ à C₆ ; halogéno ; trifluorométhyle ; azido ; alkyle en C₁ à C₆ ; alcényle en C₂ à C₆ ; (alkoxy en C₁ à C₆)-carbonyle ; (alkyle en C₁ à C₆) carbonyle ; (alcényle en C₂ à C₆)oxycarbonyle; (alcényle en C₂ à C₆)carbonyle ; hydroxy, amino ou aminocarbonyle facultativemenet substitué comme pour les substituants correspondants dans R³ ; alkylsulfonyle en C₁ à C₆ ; alcénylsulfonyle en C₂ à C₆ ; ou aminosulfonyle en C₁ à C₆ dans lequel le groupe amino est facultativement substitué avec un substituant alkyle en C₁ à C₆ ou alcényle en C₁ à C₆ ;
ou bien R⁶ et R⁸, conjointement, représentent une liaison et R⁷ et R⁹ répondent aux définitions précitées ;
ou bien R⁶ et R⁸, conjointement, représentent un groupe -O- et R⁷ et R⁹ représentent l'un et l'autre un atome d'hydrogène ;
ou bien R⁶, R⁷ ou R⁸ et R⁹, conjointement, représentent un groupe oxo ;
et chaque groupe R¹¹ représente, indépendamment, H, un groupe trifluorométhyle, alkyle en C₁ à C₆, alcényle en C₁ à C₆, (alkoxy en C₁ à C₆) carbonyle, (alkyle en C₁ à C₆)carbonyle, aminocarbonyle dans lequel le groupe amino est facultativement substitué avec un substituant (alkoxy en C₁ à C₆) carbonyle, (alkyle en C₁ à C₆) carbonyle, (alcényle en C₁ à C₆) oxycarbonyle, (alcényle en C₂ à C₆)carbonyle, alkyle en C₁ à C₆ ou alcényle en C₁ à C₆ et facultativement substitué en outre avec un substituant alkyle en C₁ à C₆ ou alcényle en C₁ à C₆ ;
où :
le terme "hétérocyclique", de la manière utilisée dans le présent mémoire, désigne un noyau aromatique ou non aromatique, simple ou condensé, contenant dans chaque noyau jusqu'à 4 hétéroatomes choisis entre des atomes d'oxygène, d'azote et de soufre, et comprenant 4 à 7 atomes de noyau, le noyau pouvant être non substitué ou substitué avec jusqu'à trois groupes choisis entre des groupes amino, halogéno, alkyle en C₁ à C₆, alkoxy en C₁ à C₆, halogénalkyle en C₁ à C₆, hydroxy, carboxy, sels carboxyliques, (alkoxy en C₁ à C₆) carbonyle, (alkoxy en C₁ à C₆) carbonyl (alkyle en C₁ à C₆), aryle et oxo, et n'importe quel groupe amino faisant partie d'un noyau hétérocyclique non aromatique simple ou condensé tel que défini étant facultativement substitué avec un substituant alkyle en C₁ à C₆ facultativement substitué avec un substituant hydroxy, alkoxy en C₁ à C₆, thiol, alkylthio en C₁ à C₆, halogéno, trifluorométhyle, acyle ou alkylsulfonyle en C₁ à C₆ ;
le terme "hétéroaryle" désigne un groupe hétérocyclique aromatique précité ;
le terme "aryle" désigne un groupe phényle ou naphtyle, chacun facultativement substitué avec jusqu'à cinq groupes choisis entre des groupes halogéno, mercapto, alkyle en C₁ à C₆, phényle, alkoxy en C₁ à C₆, hydroxyalkyle en C₁ à C₆, mercapto(alkyle en C₁ à C₆), halogénalkyle en C₁ à C₆, hydroxy, amino, nitro, carboxy, (alkyle en C₁ à C₆) carbonyloxy, (alkoxy en C₁ à C₆) carbonyle, formyle et (alkyle en C₁ à C₆)carbonyle
le terme "acyle" désigne un groupe (alkoxy en C₁ à C₆) carbonyle, formyle ou (alkyle en C₁ à C₆)carbonyle ;
sous réserve que A et B ne puissent ni l'un ni l'autre être choisis entre NR¹¹, O et S(O)ₓ et, lorsqu'un des groupes A et B représente un groupe CO, l'autre ne représente pas un groupe CO, O ou S (O)_{x,} dans la production d'un médicament destiné à être utilisé dans le traitement d'infections bactériennes chez des mammifères.

2. Composé de formule (IA) qui est un composé de formule (I), répondant à la définition suivant la revendication 1, dans lequel R³ représente un groupe hydroxyalkyle en C₁ à C₆ ou 1,2-dihydroxy(alkyle en C₂ à C₆) facultativement substitué sur le ou les groupes hydroxy.

3. Composé de formule (IB) qui et un composé de formule (I), répondant à la définition suivant la revendication 1, dans lequel au moins un groupe R¹ est un groupe alkoxy en C₂ à C₆ substitué avec un groupe amino, guanidino ou amidino facultativement N-substitué, ou alkoxy en C₁ à C₆ substitué avec un substituant pipéridyle, A représente un groupe CH₂, CHOH, CH(NH₂), C(Me)(OH) ou CH(Me) et B représente un groupe CH₂, CHOH ou CO.

4. Utilisation suivant la revendication 1, dans laquelle R¹ est en position 6 sur le noyau quinoléinee et représente un groupe méthoxy, amino(alkyle en C₃ à C₅)oxy, nitro ou fluoro et m est égal à 1.

5. Utilisation suivant la revendication 1 ou 4, dans laquelle R³ représente un groupe alkyle en C₁ à C₆, alcényle en C₁ à C₆, ou 1-hydroxy(alkyle en C₁ à C₆) facultativement substitué.

6. Utilisation suivant la revendication 5, dans laquelle R³ représente un groupe hydroxyméthyle, 1-hydroxy-éthyle ou 1,2-dihydroxyéthyle dans lequel le groupe 2-hydroxy est facultativement substitué avec un substituant alkylcarbonyle ou aminocarbonyle dans lequel le groupe amino est facultativement substitué.

7. Utilisation suivant l'une quelconque des revendications 1 et 4 à 6, dans laquelle R³ est en position 3.

8. Utilisation suivant l'une quelconque des revendications 1 et 4 à 7, dans laquelle A représente un groupe NH, NCH₃, O, CH₂, CHOH, CH(NH₂), C(Me)(OH) ou CH(Me) et B représente un groupe CH₂, CHOH, CO ou S, ou bien A représente un groupe CR⁶R⁷ et B représente un groupe CR⁸R⁹ et R⁶ et R⁸, conjointement, représentent un groupe -O- et R⁷ et R⁹ représentent l'un et l'autre un atome d'hydrogène et n est égal à 0 ou 1.

9. Utilisation suivant la revendication 8, dans laquelle :
A représente un groupe NH, B représente un groupe CO et n est égal à 1 ou 0 ;
A représente O, B représente un groupe CH₂ et n est égal à 1 ou 0 ;
A représente un groupe CH₂ ou CH_{z}OH, B représente un groupe CH₂ et n est égal à 1 ou 0 ;
A représente un groupe NCH₃, CH (NH₂), C(Me)(OH) ou CH(Me), B représente un groupe CH₂ et n est égal à 1 ;
A représente un groupe CR⁶ R⁷ et B représente un groupe CR⁸R⁹ et R⁶ et R⁸, conjointement, représentent un groupe -O- et R⁷ et R⁹ représentent l'un et l'autre un atome d'hydrogène et n est égal à 1.

10. Utilisation suivant l'une quelconque des revendications 1 et 4 à 9, dans laquelle R⁴ représente un groupe alkyle en C₅ à C₁₀, (phényle non substitué) (alkyle en C₂ ou C₃) ou (phényle non substitué) (alcényle en C₃ ou C₄).

11. Utilisation suivant l'une quelconque des revendications 1 et 4 à 10, dans laquelle R⁵ est non ramifié" en positions α et, lorsque cela est approprié, en positions β.

12. Composé de formule (I) répondant à la définition suivant la revendication 1, choisi entre les suivants : .
[3R,4R]-3-éthyl-1-hexyl-4-[3-oxo-3-(6-méthoxy-quinoléine-4-yl)propyl]pipéridine ;
[3R,4R]-3-éthyl-1-hexyl-4-[3-(R,S) -hydroxy-3-6-méthoxyquinoléine-4-yl)propyl]pipéridine ;
[3R,4R]-3-éthyl-1-heptyl-4-[3-oxo-3-(6-méthoxy-quinoléine-4-yl)propyl]pipéridine ;
[3R,4R]-3-éthyl-1-heptyl-4- [3-(R,S)-hydroxy-3-(6-méthoxyquinoléine-4-yl)propyl]pipéridine ;
[3R,4R]-3-éthyl-1-octyl-4-[3-oxo-3-(6-méthoxy-quinoléine-4-yl)propyl]pipéridine ;
[3R,4R]-3-éthyl-1-octyl-4-[3-(R, S)-hydroxy-3-(6-méthoxyquinoléine-4-yl)propyl)pipéridine ;
[3R,4R]-3-éthyl-1-décyl-4-[3-oxo-3-(6-méthoxyquinoléine-4-yl)propyl]pipéridine ;
[3R,4R]-3-éthyl-1-décyl-4-[3-(R,S)-hydroxy-3-(6-méthoxyquinoléine-4-yl)propyl]pipéridine ;
[3R,4R]-3-éthyl-1-dodécyl-4-[3-oxo-3-(6-méthoxy-quinoléine-4-yl)propyl]pipéridine ;
[3R,4R]-3-éthyl-1-dodécyl-4-[3-(R,S)-hydroxy-3-(6-méthoxyquinoléine-4-yl)propyl]pipéridine ;
[3R,4R]-3-éthyl-1-cinnamyl-4-[3-(R,S)-hydroxy-3-(6-méthoxyquinoléine-4-yl)propyl]pipéridine ;
[3R,4R]-3-éthyl-1-heptyl-4-[3-(6-méthoxyquinoléine-4-yl)propyl]pipéridine ;
[3R,4R]-3-éthényl-1-heptyl-4-[3-(R,S)-hydroxy-3-(6-méthoxyquinoléine-4-yl)propyl]pipéridine ;
[3R.4R]-3-éthyl-1-heptyl-4-[3-(R,S)-hydroxy-3-(6-hydroxyquinoléine-4-yl)propyl]pipéridine;
[3R,4R]-1-heptyl-3-[2-hydroxyéthyl)-4-[3-(R,S)-hydroxy-3-(6-hydroxyquinoléine-4-yl)propyl]pipéridine;
[3R,4R]-3-éthyl-1-heptyl-4-[3-(R,S)-hydroxy-3-(6-[5-aminopentyloxy]-quinoléine-4-yl)propyl] pipéridine ;
[3R,4R]-3-éthyl-1-heptyl-4-[3-(R,S)-hydroxy-3-(6-[2-amino-2-oxo-1,1-diméthyl]éthoxyquinoléine-4-yl)propyl]-pipéridine ;
[3R,4R]-3-éthyl-1-heptyl-4-[3-(R, S)-hydroxy-3-(6-aminoquinoléine-4-yl)propyl]pipéridine;
[3R,4R]-3-éthyl-1-heptyl-4-[3-(R,S)-hydroxy-3-(6-azidoquinoléine-4-yl)propyl]pipéridine;
[3R,4R]-3-éthyl-1-heptyl-4-[3-(6-hydroxyquinoléine-4-yl)propyl]pipéridine ;
[3R,4R]-3-éthyl-1-heptyl-4-[3-(6-propyloxyquinoléine-4-yl)propyl]pipéridine ;
[3R,4R]-3-éthyl-1-heptyl-4-[3-(6-(5-aminopentyloxy)-quinoléine-4-yl)propyl]pipéridine ;
[3R,4R]-3-éthényl-1-(2-phénoxyéthyl)-4-[3-(6-méthoxy-quinoléine-4-yl)propyl]pipéridine ;
[3S,4R]-3-éthényl-1-heptyl-4-[3-(6-méthoxy-quinoléine-4-yl)propyl]pipéridine ;
[3R,4R]-3-éthényl-1-heptyl-4-[3-(6-méthoxyquinoléine-4-yl)propyl]pipéridine ;
[3R, 4R]-1-heptyl-3-(2-hydroxyéthyl)-4-[3-(6-méthoxy-quinoléine-4-yl)propyl]pipéridine ;
[3R,4R]-1-heptyl-3-(2-acétoxyéthyl)-4-[3-(6-méthoxy-quinoléine-4-yl)propyl]pipéridine ;
[3R,4R]-1-heptyl-3-(3-hydroxypropyl)-4-[3-(6-méthoxy-quinoléine-4-yl)propyl]pipéridine ;
[3R,4R]-1-heptyl-3-(1-hydroxyéthyl)-4-[3-(6-méthoxy-quinoléine-4-yl)propyl]pipéridine ;
[3R,4R]-3-éthyl-1-(2-phényléthyl)-4-[3-(R,S)-hydroxy-3-(6-méthoxyquinoléine-4-yl)propyl]pipéridine ;
[3R,4R]-3-éthyl-1-(-phénylpropyl)-4-[3-(R,S)-hydroxy-3-(6-méthoxyquinoléine-4-yl)propyl]pipéridine ;
heptyl-4-[2-(R,S)-hydroxy-3-(6-méthoxyquinoléine-4-yl)-propyl] pipéridine ;
1-heptyl-4-[3-(6-méthoxyquinoléine-4-yl)prop-2-ényl]-pipéridine ;
1-heptyl-4-[3-(6-méthoxyquinoléine-4-yl)propyl]-pipéridine ;
[3R,4R]-3-éthyl-1-heptyl-4-[3-(R,S)-hydroxy-3-(6-méthoxyquinoléine-4-yl)butyl]pipéridine;
[3R,4R]-3-éthényl-1-heptyl-4-[3-(R,S)-azido-3-(6-méthoxyquinoléine-4-yl)propyl]pipéridine ;
[3R,4R]-3-éthényl-1-heptyl-4-[3-(R,S)-amino-3-(6-méthoxyquinoléine-4-yl)propyl]pipéridine;
[3R,4R]-3-éthyl-1-heptyl-4-[3-(R,S)-amino-3-(6-méthoxyquinoléine-4-yl) propyl] pipéridine ;
[3R,4R]-3-éthyl-1-heptyl-4-[3-(6-méthoxyquinoléine-4-yl)butyl]pipéridine ;
[3R,4R]-3-éthényl-1-heptyl-4-(3-(R,S)-acétamido-3-(6-méthoxyquinoléine-4-yl)propyl]pipéridine ;
[3R,4R]-1-heptyl-3-(2-(R,S)-hydroxypropyl)-4-[3-(6-méthoxyquinoléine-4-yl)propyl]pipéridine;
[3R,4R]-1-heptyl-3-(1-(R,S)-2-dihydroxyéthyl)-4-[3-(6-méthoxyquinoléine-4-yl)propyl]pipéridine ;
[3R,4R]-1-heptyl-3-aminocarbonyloxyéthyl-4-[3-(6-méthoxyquinoléine-4-yl)propyl]pipéridine ;
[3R,4R]-3-(1-(R,S),2-dihydroxyéthyl)-1-heptyl-4-[3-(R,S)-hydroxy-3-(6-méthoxyquinoléine-4-yl)propyl]pipéridine ;
[3R,4R]-3-éthyl-1-heptyl-4-[(6-méthoxyquinoléine-4-oxy)méthyl]pipéridine ;
[3R,4S]-3-éthényl-1-heptyl-4-[2-(6-méthoxyquinoléine-4-yl)oxyéthyl]pipéridine ;
1-heptyl-4-[(6-méthoxyquinoléine-4-yl)oxyméthyl]-pipéridine ;
[3R,4R]-3-éthyl-1-heptyl-4-[(6-méthoxyquinoléine-4-yl)-méthylthiométhyl]pipéridine ;
[3R,4R]-1-heptyl-3-éthényl-4-[{(6-méthoxyquinoléinee-4-yl)carbonylamino}méthyl]pipéridine ;
[3R,4R]-3-éthényl-1-heptyl-pipéridine-4-[N-(6-méthoxy-quinoléine-4-yl)]propionamide ;
[3R,4R]-3-éthényl-1-heptyl-pipéridine-4- [N- (6-méthoxy-quinoléine-4-yl)]propylamine ;
[3R,4S]-3-éthényl-1-heptyl-pipéridine-4-[N-(6-méthoxy-quinoléine-4-yl)]acétamide ;
[3R,4R]-3-éthényl-1-heptyl-pipéridine-4-[N-(6-méthoxy-quinoléine-4-yl)]éthylamine ;
[3R,4R]-3-éthényl-1-heptyl-4-[2-(R,S)-hydroxy-2-(6-méthoxyquinoléine-4-yl)éthyl]pipéridine ;
[3R,4R]-3-éthényl-1-heptyl-4-[2 (6-méthoxyquinoléine-4-yl)éthyl]pipéridine ;
[3R,4R]-3-éthyl-1-heptyl-4-[2-(6-méthoxyquinoléine-4-yl) éthyl]pipéridine ;
1-heptyl-4-[2(R,S)-hydroxy-2-(6-méthoxy-4-quinoléi-nyl)-éthyl]pipéridine ;
[3S,4R]-3-éthényl-1-heptyl-4-[2-(6-méthoxyquinoléine-4-yl)éthyl]pipéridine ;
N-(6-méthoxy-4-quinoléineyl)-1-heptyl-4-pipéridine-carboxamide ;
(3z)-(4R)-3-éthylidène-1-héptyl-4-[3-(6-méthoxy-quinoléine-4-yl)propyl]pipéridine ;
[3R,4S]-1-cinnamyl-4-[2-(6-méthoxyquinoléine-4-yl)oxy-éthyl]pipéridine ;
[3R,4R]-3-(2-acétoxyéthyl)-1-heptyl-4-[3-(6-méthoxy-quinoléine-4-yl)propyl]pipéridine ;
[3R,4R]-3-éthyl-1-heptyl-4-[3- (6-{2-hydroxyéthyloxy}-quinoléine-4-yl)proyl]pipéridine ;
[3R,4R]-3-(éthylaminocarbonyloxyéthyl)-1-heptyl-4-[3-(6-méthoxyquinoléine-4-yl)propyl]pipéridine ;
[3R,4R]-3-éthényl-1-heptyl-4-[3-(R,S)-aminocarbonyl-amino-3-(6-méthoxyquinoléine-4-yl)propyl]pipéridine ;
[3R,4R]-3-éthyl-1-heptyl-4-[3-(6-(4-aminobutyloxy)-quinoléine-4-yl)propyl]pipéridine ;
[3R,4R]-1-heptyl-3-(1-(R)- et 1-(S)-hydroxy-2-méthoxy-éthyl)-4-[3-(6-méthoxyquinoléine-4-yl)propyl]pipéridine ;
[3R,4R]-1-heptyl-3-(1-(R)- et 1-(S)-hydroxy-2-méthyl-thio-éthyl)-4-[3-(6-méthoxyquinoléine-4-yl)propyl]-pipéri-dine ;
[3R,4R]-1-(5-méthylhexyl)-3-(1-(R)- et 1-(S),2-dihydroxyéthyl)-4-[3-(6-méthoxyquinoléine-4-yl)propyl]-pipéridine ;
[3R,4R]-3-éthyl-1-heptyl-4-[3-(6-(3-aminopropyl)oxyquinoléine-4-yl)propyl]pipéridine;
[3R,4R]-3-éthyl-1-heptyl-4-[3-(6-(2-aminoéthyl)oxyquinoléine-4-yl)propyl]pipéridine ;
[3R,4R]-3-éthyl-1-heptyl-4-[3-(6-(3-guanidinopropyl)oxyquinoléine-4-yl)propyl]pipéridine;
[3R,4R]-3-éthyl-1-heptyl-4-[3-(6-(pipéridine-4-yl)-méthoxyquinoléine-4-yl)propyl]pipéridine ;
[3R, 4S]-1-heptyl-3-vinyl-4-[3-(6-méthoxyquinoléine-4-yl)-(R,R)-oxiranne-2-ylméthyl]pipéridine ;
[3R,4S]-1-heptyl-4-[(2S)-hydroxy-3-(6-méthoxyquinoléine-4-yl)propyl]-3-vinylpipéridine ;
[3R,4S]-1-heptyl-3-vinyl-4-[3-(6-méthoxyquinoléine-4-yl)-(S,S)-oxiranne-2-ylméthyl]pipéridine ;
[3R,4S]-3-éthyl-1-heptyl-4-[2-(S)-hydroxy-3-(6-méthoxyquinoléine-4-yl)propyl]pipéridine ;
[3R,4S]-1-heptyl-4-[N-méthyl-N-(6-méthoxyquinoléine-4-yl)amino-éthyl]-3-vinylpipéridine ;
(3R,4R]-1-heptyl-3-(1-(R,S)-hydroxyéthyl)-4-[3-(6-méthoxyquinoléine-4-yl)propyl]pipéridine ;
[3R,4R]-1-heptyl-3-(1-(R,S)-hydroxy-1-méthyléthyl)-4-[3-(6-méthoxyquinoléine-4-yl)propyl]pipéridine ;
[3R,4R]-1-heptyl-3-hydroxyméthyl-4-[3-(6-méthoxyquinoléine-4-yl)propyl]pipéridine ;
[3R,4R]-1-(6-méthylheptyl)-3-(1-(R) et 1-(S)-2-dihydroxyéthyl)-4-[3-(6-méthoxyquinoléine-4-yl)propyl]-pipéridine ;
[3R,4S]-1-heptyl-4-[(2S)-hydroxy-3-(6-méthoxyquinoléin-4-yl)propyl]-3-(2-hydroxyéthyl)pipéridine ;
[3R,4S]-1-heptyl-3-aminocarbonyloxyméthyl-4-[3-(6-méthoxyquinoléine-4-yl)propyl]pipéridine et
[3R,4R]-1-heptyl-4-[3-(6-méthoxyquinoléine-4-yl)propyl]-3-(2-caramoyltéhyl)pipéridine ;
ou un dérivé pharmaceutiquement acceptable de n'importe lequel des composés précités.

13. Procédé pour la préparation d'un composé de formule (I), ou d'un de ses dérivés pharmaceutiquement acceptables, suivant la revendication 2, 3 ou 12, procédé qui comprend :
(a) la réaction d'un composé de formule (IV) avec un composé de formule (V) : 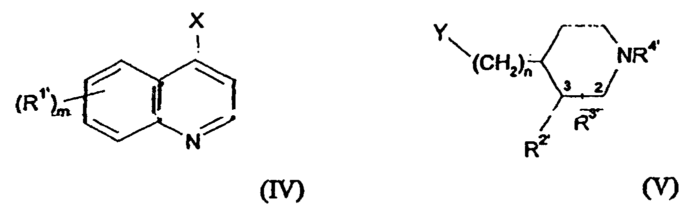 formules dans lesquelles m, n, R¹_{,} R², R³ et R⁴ répondent aux définitions mentionnées pour la formule (I), et X et Y peuvent représenter les associations suivantes :
(i) X représente M et Y représente un groupe CH₂CO₂R^{x}
(ii) X représente un groupe CO₂R^{y} et Y représente un groupe CH₂CO₂R^{x}
(iii) un de X et Y représente un groupe CH=SPh₂ et l'autre représente un groupe CHO
(iv) X représente un groupe CH₃ et Y représente un groupe CHO
(v) X représente un groupe CH₃ et Y représente un groupe CO₂R^{x}
(vi) X représente un groupe CH₂CO₂R^{y} et Y représente un groupe CO₂R^{x}
(vii) X représente un groupe CH=PR^{z}₃ et Y représente un groupe CHO
(viii) X représente un groupe CHO et Y représente un groupe CH= PR^{z}₃
(ix) X représente un atome d'halogène et Y représente un groupe CH=CH₂
(x) un de X et Y représente un groupe COW et l'autre représente un groupe NHR^{11'}
(xi) un de X et Y représente un groupe (CH₂)ₚ-V et l'autre représente un groupe (CH₂)_{q}NHR^{11'}, (CH₂)_{q}OH, (CH₂)_{q}SH
ou (CH₂)_{q}SCOR^{x} dans lequel la somme p+q est égale à 1
(xii) un de X et Y représente un groupe CHO et l'autre représente un groupe NHR^{11'}
(xiii) un de X et Y représente un groupe OH et l'autre représente un groupe CH=N₂
dans lesquelles V et W représentent des groupes partants, R^{x} et R^{y} représentent un groupe alkyle en C₁ à C₆ et R^{z} représente un groupe aryle ou alkyle en C₁ à C₆ ;
(b) le réarrangement d'un composé de formule (II) : 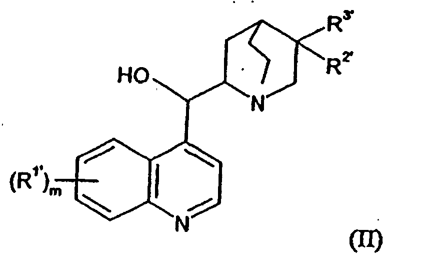 pour obtenir un composé de formule (III) qui est un composé de formule (I) dans laquelle R³ est en position 3, n est égal à 1, A-B représente un groupe COCH₂ ou un époxyde disubstitué et R² représente H, et ensuite, facultativement, la réduction en un composé de formule (VII) qui est un composé de formule (I) dans laquelle n est égal à 1, A-B représente un groupe CHOHCH₂ ou CH₂CHOH et R² représente H ;
(c) la photo-oxygénation d'un composé de formule (VI) : 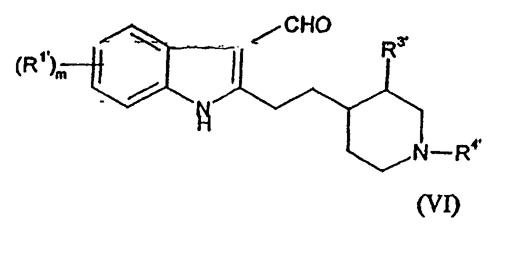 ou
(d) la réaction d'un composé de formule (IV) avec un composé de formule (Vb) : 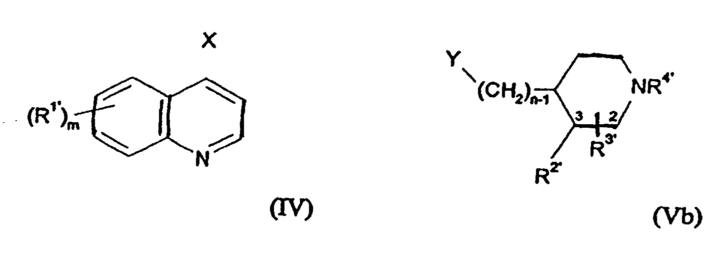 formules dans lesquelles m, n, R¹, R², R³ et R⁴ répondent aux définitions mentionnées pour la formule (I), X représente un groupe CH₂NHR^{11'} et Y représente un groupe CHO ou COW ou bien X représente un groupe CH₂OH et Y représente un groupe -CH=N₂ ;
formules dans lesquelles R^{11'}, R^{1'}, R^{2'}, R^{3'} et R^{4'} représentent des groupes R¹¹, R¹, R², R³ et R⁴ ou des groupes pouvant être convertis en ceux-ci, et ensuite, facultativement ou de manière nécessaire, la conversion des groupes R^{11'}, R^{1'}, R^{2'}, R^{3'} et R^{4'} en des groupes R^{11'}, R¹, R², R³ et R⁴, la conversion de A-B en un autre groupement A-B, l'interconversion de R¹¹, R¹, R², R³ et/ou R⁴ et la formation d'un de ses dérivés pharmaceutiquement acceptables.

14. Composition pharmaceutique comprenant un composé suivant la revendication 2, 3 ou 12 et un support pharmaceutiquement acceptable.
